## Europäisches Patentamt

## European Patent Office

## Office européen des brevets

(11) Veröffentlichungsnummer: **0 374 753**
**A2**

(12) **EUROPÄISCHE PATENTANMELDUNG**

(21) Anmeldenummer: **89123226.6**

(22) Anmeldetag: **15.12.89**

(51) Int. Cl.5: **C12N 15/12, C12P 21/02, A01H 5/00, A01N 63/02, C12P 21/08, C07K 7/10, C12N 5/10, C12N 1/21**

(30) Priorität: **19.12.88 US 285924**
**19.12.88 US 286002**
**19.12.88 US 286087**

(43) Veröffentlichungstag der Anmeldung:
**27.06.90 Patentblatt 90/26**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel(CH)**

(72) Erfinder: **Zlotkin, Eliahu**
**Mevobosmat 5**
**Mevaseret Zion-90805(IL)**
Erfinder: **Eitan, Michal**
**Kibbutz Ayeleth Hashahar**

**Upper Galilee(IL)**
Erfinder: **Ben-Yehuda, Oz**
**2, Ben Zion St.**
**Jerusalem(IL)**
Erfinder: **Fowler, Elizabeth**
**111 Briarcliff Road**
**Durham, N.C. 27707(US)**
Erfinder: **Belagaje, Rama M.**
**7821 Mohawk Lane**
**Indianapolis Indiana 46260(US)**
Erfinder: **Roberts, Jean L.**
**203 North Hugo Street**
**Indianapolis Indiana 46229(US)**

(74) Vertreter: **Zumstein, Fritz, Dr. et al**
**Bräuhausstrasse 4**
**D-8000 München 2(DE)**

(54) **Insektizide Toxine, Gene, die diese Toxine kodieren, Antikörper, die sie binden, sowie transgene Pflanzenzellen und transgene Pflanzen, die diese Toxine exprimieren.**

(57) Diese Erfindung betrifft transgene Pflanzen, Pflanzenzellen und Mikroorganismen, die unter Einsatz der rekombinanten DNA-Techniken so transformiert sind, dass sie selektiv auf Insekten wirkende Toxine aus giftigen Tieren, vorteilhafterweise aus Vertretern des Stammes *Arthropoda*, insbesondere aus Vertretern der Klasse *Arachnida* (Spinnentiere), vor allem aus Vertretern der Ordnung *Scorpiones*, sowie insbesondere aus Vertretern der Klasse *Chilopoda* (Hundertfüssler) synthetisieren können. Die Erfindung betrifft ferner Gene, die besagte Toxine kodieren, und den Einsatz dieser Gene, um Pflanzen eine genetisch vermittelte Resistenz gegenüber Insekten zu verleihen und um phytopathogene Insekten zu bekämpfen. Die Erfindung betrifft auch ein Toxin aus dem Gift des Skorpions *Leiurus quinquestriatus hebraeus* mit der Aminosäuresequenz VRDAYIAKNY NCVYECFRDA YCNELCTKNG ASSGYCQWAG KYGNACWCYA LPDNVPIRVP GKCR. Die Erfindung betrifft weiterhin Antikörper gegen besagte Toxine und insektizide Mittel, die besagte Toxine enthalten.

Sequenzen von Skorpionentoxinen

Toxine der *Buthinae* Skorpione

| | | | | |
|---|---|---|---|---|
| LqhIT2 | DGYIKRRDGC | KVACLIGNEG | CDKECKAYGG | SYGYCWTWGL | ACWCEGLPDD KTWKSETNTC G |
| LqqIT2 | DGYIRRRDGC | KLSCLFGNEG | CNKECKSYGG | SYGYCWTWGL | ACWCEGLPDE KTWKSETNTC G |
| BjIT2 | DGYIRKRDGC | KVSCIIGNEG | CRKECVAHGG | SFGYCWTWGL | ACWCENLPDA VTWKSSTNTC G |
| LqhP35 | VRDAYIAKNY | NCVYECFRDA | YCNELCTKNG | ASSGYCQWAG | KYGNACWCYA LPDNVPIRVP GKCR |

Toxine der *Chactidae* Skorpione

| | | | |
|---|---|---|---|
| SmpIT2 | ALPLSGEYEP | CVRPRNCKPG | LVCNKQGICV | DPK |
| SmpCT2 | VSCTGSRDCY | APCRKQTGCT | SAKCINKSCK | CYGC |
| SmpCT3 | VSCTGSKDCY | APCRKQTGCP | NAKCINKSCK | CYGC |
| SmpM4T | VSCTGSKECY | APCRKQTGCP | NAKCMNRNCK | CYGC |

*Fig. 1*

## Insektizide Toxine, Gene, die diese Toxine kodieren, Antikörper, die sie binden, sowie transgene Pflanzenzellen und transgene Pflanzen, die diese Toxine exprimieren

Diese Erfindung betrifft transgene Pflanzen, Pflanzenzellen und Mikroorganismen, die unter Einsatz der rekombinanten DNA-Techniken so transformiert sind, dass sie selektiv auf Insekten wirkende Toxine aus giftigen Tieren, vorteilhafterweise aus Vertretern des Stammes *Arthropoda*, insbesondere aus Vertretern der Klasse *Arachnida* (Spinnentiere), vor allem aus Vertretern der Ordnung *Scorpiones*, sowie insbesondere aus Vertretern der Klasse *Chilopoda* (Hundertfüssler) synthetisieren können. Die Erfindung betrifft ferner Gene, die besagte Toxine kodieren, und den Einsatz dieser Gene, um Pflanzen eine genetisch vermittelte Resistenz gegenüber Insekten zu verleihen und um phytopathogene Insekten zu bekämpfen. Die Erfindung betrifft auch ein Toxin aus dem Gift des Skorpions *Leiurus quinquestriatus hebraeus*. Die Erfindung betrifft weiterhin Antikörper gegen besagte Toxine und insektizide Mittel, die besagte Toxine enthalten.

Tierische Gifte sind Mischungen von Stoffen, die in speziellen Drüsen im Körper des giftigen Tieres gebildet werden. Das Gift wird in das Beutetier oder in den Widersacher mit Hilfe eines stechend-bohrenden Apparates eingebracht, um es/ihn zu lähmen und/oder zu töten. Skorpione enthalten in ihrem Gift eine Reihe von Proteinen bzw. Neurotoxinen, die giftig sind und auf das Nervensystem einwirken. Die einzelnen Neurotoxine unterscheiden sich in ihrer Wirksamkeit gegenüber verschiedenartigen Tierspezies.

Die Gifte von Skorpionen, die zur Unterfamilie *Buthinae* gehoren, enthalten drei Hauptgruppen von Polypeptid-Neurotoxinen, die die axonale Membranleitfähigkeit für Natrium verändern. Die erste Gruppe von Neurotoxinen wird von den $\alpha$-Toxinen gebildet, die spezifisch Säugetiere durch eine extreme Verlängerung des Aktionspotentials beeinträchtigen, was auf eine Verlangsamung oder Blockierung des Natrium-Kanals bei der Aktivierung zurückzuführen ist (Catterall, 1984, Rochat et al., 1979). Die $\alpha$-Toxine AaH1 und AaH2 sind im Gift des Skorpions *Androctonus australis* Hector [aus dem das erste erregende, auf Insekten wirkende Toxin AaIT isoliert wurde (Zlotkin et al., 1971a)] enthalten. Diese $\alpha$-Toxine zeigen auf Schmeissfliegen-Larven keine Wirkung (Zlotkin et al., 1971c). Die zweite Gruppe von Neurotoxinen wird von den hemmenden, selektiv auf Insekten wirkenden Toxinen gebildet, die eine sich progressiv entwickelnde schlaffe Lähmung von Insekten bewirken, indern sie durch die Unterdrückung des Natrium-Stroms die Aktionspotentiale wesentlich blockieren (Lester et al., 1982, Zlotkin et al., 1985). Die dritte Gruppe von Neurotoxinen wird von den erregenden, selektiv auf Insekten wirkenden Toxinen gebildet, die eine sofortige (knock down) spastische Lähmung von Insekten bewirken, indern sie durch eine Zunahme des Natrium-Spitzenstroms und die Spannungsabhängige Verlangsamung seiner Inaktivierung wiederholtes Feuern in den motorischen Nerven der Insekten induzieren (Walther et al., 1976, Pelhate und Zlotkin, 1981).

Der Nachweis und die Überwachung der aus Skorpiongift stammenden, auf Insekten wirkenden Toxine während ihrer Isolierung wird vorteilhafterweise mit Hilfe typischer Reaktionen von *Sarcophaga*-Larven durchgeführt, die sich in der sofortigen und vorübergehenden Kontraktionslähmung durch die erregenden Toxine und in der sich progressiv entwickelnden Erschlaffung durch die hemmenden Toxine zeigt (Zlotkin et al., 1971b, Lester et al., 1982). Trotz der gegensätzlichen Symptomatologie, die durch die obigen hemmenden und erregenden, auf Insekten wirkenden Toxine induziert wird, betreffen beide Gruppen ausschliesslich die Membranleitfähigkeit für Natrium und teilen sich beide dieselbe Bindungsstelle in den Membranen der Insektenneurone (Zlotkin et al., 1985, Gordon et al., 1984).

Selektiv auf Insekten wirkende Toxine sind auch in den Giften einer Reihe anderer Arthropoden vorhanden (Zlotkin, 1985). Die Gifte von Schlupfwespen sind hochtoxisch für Schmetterlingslarven. Das Gift der Schlupfwespe *Bracon hebetor* verursacht eine erschlaffende Lähmung in Schmetterlingslarven, indem an der neuromuskulären Endplatte der Insekten ein präsynaptischer Bruch der erregenden glutaminergen Übertragung induziert wird (Piek et al., 1982). Die Gifte von Solitärwespen wirken auf eine grosse Zahl von Insekten und Spinnen verschiedener Ordnungen (Rathmeyer, 1962). Ein Beispiel für diese Gifte ist das Gift von *Philanthus triangulum*, das in Insekten eine erschlaffende Lähmung verursacht, die im wesentlichen auf die präsynaptische Blockade der neuromuskulären Übertragung zurückzuführen ist; dieses Gift beeinträchtigt sowohl die erregende als auch die hemmende Übertragung (May und Piek, 1979). Das Gift der Spinne Schwarze Witwe, *Latrodectus mactans*, enthält Bestandteile, die für Insekten, nicht jedoch für Säugetiere neurotoxisch sind, und andere, die spezifisch Crustaceen beeinträchtigen (Fritz et al., 1980, Ornberg et al., 1976).

Gifte von Hundertfüsslern der Gattung *Scolopendra* werden von Jangi (1984) beschrieben. Da die Hundertfüssler der Gattung *Scolopendra* eine geringe Bedeutung für die Volksgesundheit haben, sind ihre Gifte bisher jedoch nicht ausführlich untersucht und charakterisiert worden.

Es ist ein Anliegen der vorliegenden Erfindung, transgene Pflanzen, die eine genetisch vermittelte Resistenz gegenüber Insekten haben, dadurch herzustellen, dass Gene eingeführt werden, die die Produktion von selektiv auf Insekten wirkenden Toxinen durch die pflanzlichen Gewebe induzieren. Es ist weiterhin ein Anliegen der vorliegenden Erfindung, tierische Gifte und insbesondere die darin enthaltenen Toxine in einer Form zu erhalten, die im wesentlichen frei von natürlichen Verunreinigungen ist. Derartig gereinigte Toxine sind wertvoll als Insektizide.

Die vorliegende Erfindung betrifft selektiv gegen Insekten wirkende Toxine aus giftigen Tieren, vorteilhafterweise aus Vertretern des Stammes *Arthropoda*, insbesondere aus Vertretern der Klasse *Arachnida* (Spinnentiere), vor allem aus Vertretern der Ordnung *Scorpiones*, sowie insbesondere aus Vertretern der Klasse *Chilopoda* (Hundertfüssler), insbesondere das aus dem Gift eines gelben Skorpions *L. quinquestriatus hebraeus, Buthinae, Buthidae*, isolierte Toxin LqhP35, sowie Gene, die diese Toxine kodieren.

Die vorliegende Erfindung betrifft ferner die Herstellung von transgenen Pflanzen, deren Resistenz gegenüber Insekten darauf beruht, dass Gene in das Pflanzengenom eingeführt werden, die die Produktion von selektiv auf Insekten wirkenden Toxinen durch die pflanzlichen Gewebe induzieren. Die Erfindung betrifft ferner rekombinante DNA-Moleküle, die eine Gen-Sequenz umfassen, die ein selektiv gegen Insekten wirkendes Toxin kodiert, und gegen Insekten tolerante bzw. für Insekten toxische, transformierte Pflanzenzellen und daraus resultierende transformierte Pflanzen. Erfindungsgemäss werden Pflanzenzellen mit besagten Genen transformiert, was den Zellen als Folge der Expression oder Überexpression die Toleranz bzw. Toxizität gegenüber Insekten verleiht.

Die vorliegende Erfindung betrifft auch Pflanzen, die aus den transformierten Pflanzenzellen regeneriert werden, und deren Saatgut. Die vorliegende Erfindung betrifft ferner die Nachkommen von Pflanzen, die aus den transformierten Pflanzenzellen regeneriert werden, einschliesslich Mutanten und Variantennachkommenschaft, sofern diese insektizide bzw. Insekten-tolerierende Eigenschaften aufweisen.

Die vorliegende Erfindung umfasst demzufolge auch ein Verfahren zum Schutz von Pflanzen oder Pflanzenteilen vor Insektenschädlingen, das darauf beruht, dass der Pflanze oder dem Pflanzenteil, die/das geschützt werden soll, eine insektizid wirksame Menge eines oder mehrerer Toxine verliehen wird, von denen wenigstens eines aus einem Arthropodengift, beispielsweise dem Skorpiongift stammt.

Dieses Verfahren ist auf den Schutz von Pflanzen oder Pflanzenteilen anwendbar, einschliesslich Saatgut von denjenigen Pflanzen, die von Insekten bedroht sind.

Die vorliegende Erfindung betrifft auch chimäre genetische Konstrukte, die das besagte Gen enthalten, Klonierungsvektoren und -wirte sowie Verfahren, die Pflanzen eine Toleranz gegenüber Insekten verleihen.

Diese Erfindung betrifft auch die Verwendung dieser Toxine als Insektizide. Andere, beispielsweise aus dem Gift des Skorpions *Scorpio maurus palmatus* (Familie *Chactidae*) isolierte Toxine können ebenfalls erfindungsgemäss verwendet werden.

Die Erfindung betrifft auch Antikörper, die diese Toxine binden können.


Abbildungen

Fig. 1: Konstruktion von pRK252/Tn903/Bg1II

Fig. 2: Konstruktion von pCIB5

Fig. 3 und 4: Konstruktion von pCIB4

Fig. 5: Konstruktion von pCIB2

Fig. 6: Konstruktion von pCIB10, einem Plasmid mit breitem Wirtsbereich, das T-DNA-Grenzsequenzen und ein Gen zur Pflanzenselektion enthält

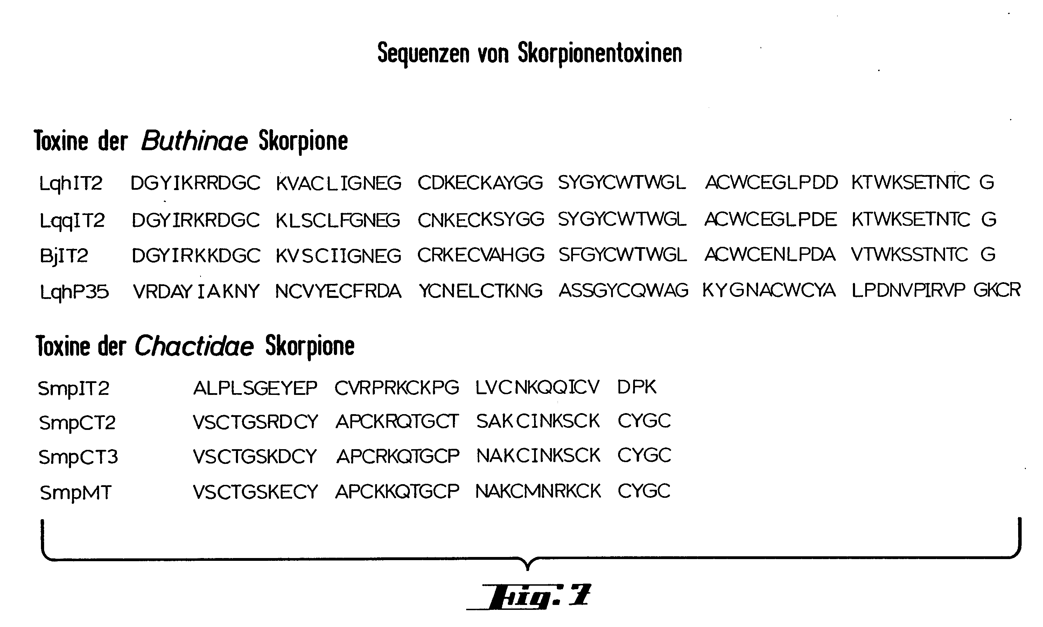

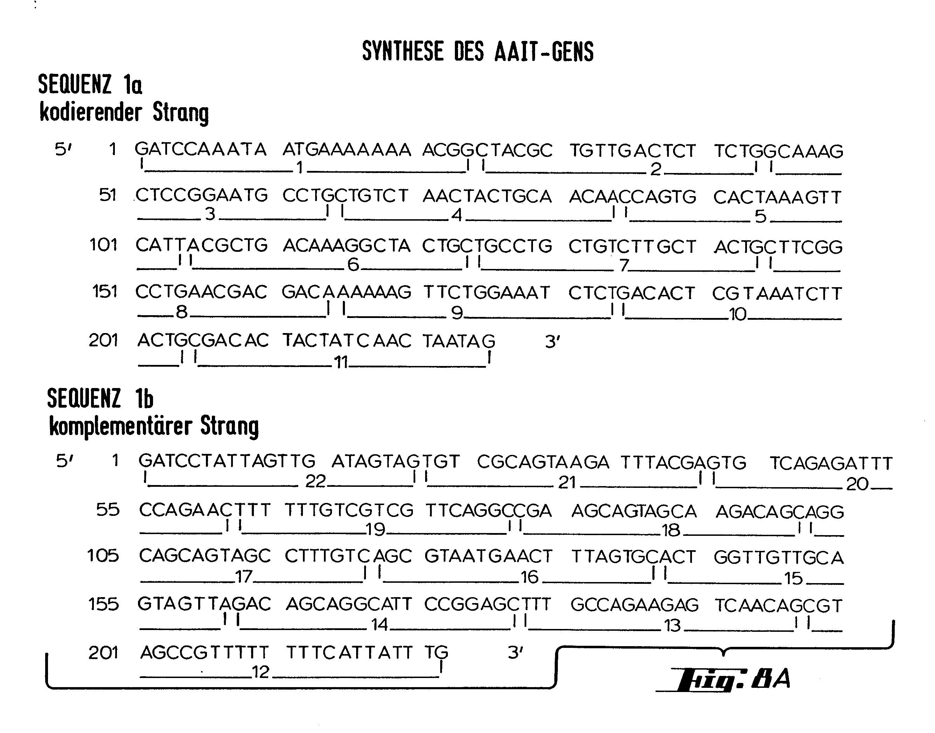

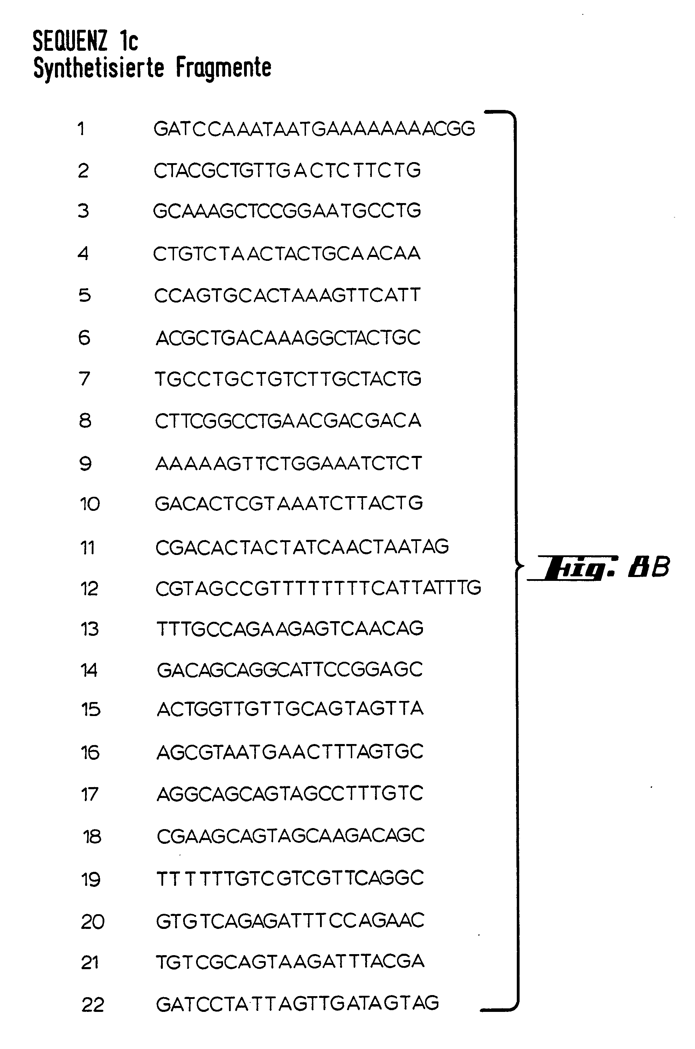

Fig. 7: Aminosäurensequenzen verschiedener Skorpiontoxine, die wie in Beispiel 10 beschrieben erhalten werden. LqhIT2 ist das repräsentative Toxin in Beispiel 10. LqqIT2 ist ein hemmendes Toxin für Insekten aus *L. quinquestriatus quinquestriatus*, dessen Reinigung in Zlotkin et al. (1985) beschrieben ist. BjIT2 ist ein hemmendes Toxin für Insekten aus *Buthotus judaicus*, dessen Reinigung in Lester et al. (1982) beschrieben ist. LqhP35 wird hierin beschrieben und ist ein Zwischentoxin, das die Natrium-Kanäle von Insekten in einer Weise beeinträchtigt, die sehr derjenigen ähnelt, die die α-Toxine auf Natrium-Kanäle der Säugetiere haben. Die smp-Toxine werden aus dem Gift des Skorpions *Scorpio maurus palmatus* (Familie *Chactidae*) gewonnen. SmpIT2 ist ein auf Insekten wirkendes Toxin, dessen Reinigung in Lazarovici et al. (1982) beschrieben ist. SmpCT2 und SmpCT3 sind gegen Crustaceen wirkende Toxine, deren Reinigung in Lazarovici et al. (1984) beschrieben ist. Die Reinigung von SmpMT, eines gegen Säugetiere wirkenden Toxins, wird in Lazarovici und Zlotkin (1982) beschrieben.

Fig. 8: Synthese und Sequenz des Gens für AaIT. Sequenz 1a zeigt die Sequenz des kodierenden Strangs. Sequenz 1b zeigt die Sequenz des komplementären Strangs. Sequenz 1c zeigt die Sequenz der synthetisierten Fragmente. Sequenz 1d zeigt die Sequenz des endgültigen Gens.

Fig. 9: Sequenz des Gens, das das gegen Insekten wirkende Toxin LqhIT2 kodiert (in den Beispielen als "Sequenz 2" bezeichnet).

Abkürzungen

AaIT Gegen Insekten wirkendes Toxin von *Androctonus australis*
LqhP35 Lqh bezeichnet den Skorpion, P steht für Paralyse und 35 entspricht der Elutionsdauer auf der HPLC Säule
HPLC High Performance Liquid Chromatography
MG Molekulargewicht
SDS-PAGE Natriumdodecylsulfat-Polyacrylamid Gelelektrophorese
PU Lähmende Einheit (Paralytic Unit)
3,4-DAP 3,4-Diaminopyridin
LD Lethale Dosis
pI Isoelektrischer Punkt
Bp Basenpaare
CaMV Cauliflower Mosaic Virus
Asp-N *Pseudomonas fragi* Endoproteinase Asp-N
Lys-C Lysin-Endopeptidase
Glu-C *Staphylococcus aureus* Protease V8
RSA Rinderserumalbumin
TTX Tetrodotoxin
PEG Polyethylenglykol
Tris-HCl Tris(hydroxymethyl)methylaminhydrochlorid
EDTA Ethylendiamin-N,N,N',N'-tetraessigsäure
TFA Trifluoressigsäure
4-AP 4-Aminopyridin
NPT Neomycin-phosphotransferase
STX Saxitoxin
w/v Gewicht/Volumen
TEA Tetraethylammoniumchlorid
ATCC American Type Culture Collection, Rockville, Maryland
D Dalton

## I. Aus tierischen Giften stammende, selektiv gegen Insekten wirkende Toxine

Die Gene verschiedener Toxine können zur erfindungsgemässen Transformation von Pflanzen benutzt werden, um sie tolerant gegenüber Insekten zu machen.

Die Aminosäurensequenz eines gegen Insekten wirkenden Toxins, eines erregenden Toxins aus *Androctonus australis* (AaIT) wurde zum ersten Mal von Darbon et al. (1982) bestimmt und veröffentlicht. Die Aminosäurensequenz dieses Neurotoxins ist wie folgt:
KKNGYAVDSS GKAPECLLSN YCNNQCTKVH YADKGYCCLL SCYCFGLNDD KKVLEISDTR KSYCDTTIIN.

LqqIT2 ist ein Insekten hemmendes Toxin aus *L. quinquestriatus quinquestriatus* (Zlotkin et al., 1985) mit nachfolgender Aminosäurensequenz:
DGYIRKRDGC KLSCLFGNEG CNKECKSYGG SYGYCWTWGL ACWCEGLPDE KTWKSETNTC G.

BjIT2 ist ein Insekten hemmendes Toxin aus *Buthotus judaicus* (Lester et al., 1982). BjIT2 existiert in zwei Isoformen, die sich in der Aminosäurensequenz in Position 15 unterscheiden. Form 1 enthält in dieser Position Isoleucin, während Form 2 Valin enthält. Die Aminosäurensequenz dieses Neurotoxins ist wie folgt:
DGYIRKKDGC KVSC(V/I)IGNEG CRKECVAHGG SFGYCWTWGL ACWCENLPDA VTWKSSTNTC G

LqhIT2 ist ein Insekten hemmendes Toxin aus *L. quinquestriatus hebraeus*, das über Reverse Phase HPLC gereinigt wird. Die Aminosäurensequenz dieses Neurotoxins ist wie folgt:
DGYIKRRDGC KVACLIGNEG CDKECKAYGG SYGYCWTWGL ACWCEGLPDD KTWKSETNTC G

SmpIT2, aus dem Skorpion *Scorpio maurus palmatus* (Familie *Chactidae*), ist ein hemmendes gegen Insekten wirkendes Toxin (Lazarovici et al., 1982) mit folgender Aminosäurensequenz:
ALPLSGEYEP CVRPRKCKPG LVCNKQQICV DPK

Ein neues, erfindungsgemäss einsetzbares Toxin ist LqhP35, das die verzögerte und anhaltende Kontraktionslähmung von Schmeissfliegen-Larven induziert. Es beeinträchtigt die Natrium-Kanäle von Insekten in einer Weise, die sehr derjenigen ähnelt, die die α-Toxine auf Natrium-Kanäle der Säugetiere haben. Dieses Neurotoxin stammt aus einem gelben Skorpion, *L. quinquestriatus hebraeus, Buthinae, Buthidae*,

4

und weist folgende Aminosäurensequenz auf:

VRDAYIAKNY NCVYECFRDA YCNELCTKNG ASSGYCQWAG KYGNACWCYA LPDNVPIRVP GKCR

Verglichen mit den erregenden und hemmenden, auf Insekten wirkenden Toxinen, die aus den Giften der *Buthinae*-Skorpione stammen, (a) induziert dieses Toxin eine unterschiedliche Symptomatologie in der Schmeissfliegen-Larve (PU: 14 ng/100 mg KG), (b) ist dieses Toxin unfähig, das markierte erregende auf Insekten wirkende $^{125}$I AaiT von seinen Bindungsstellen an der neuronalen Membran von Insekten zu verdrängen, (c) ist dieses Toxin ebenfalls sehr wirksam gegen Crustaceen (20 ng/100 mg KG), jedoch sehr schwach toxisch für Mäuse (100 µg/20 g KG) und (d) induziert dieses Toxin einen völlig anderen Effekt auf die Natrium-Leitfähigkeit in einem Axon-Präparat eines Insekts.

*S. falculata* Schmeissfliegen-Larven können auf Grund der Anordnung ihrer Skelettmuskeln in Segmenten, der weichen und flexiblen Kutikula und der konstanten Beweglichkeit einfache Verhaltensreaktionen auf verschiedene Neurotoxine zeigen. Mit Hilfe derartiger Verhaltensreaktionen ist eine klare Unterscheidung zwischen den erregenden und hemmenden, auf Insekten wirkenden Toxinen, die aus den Giften der *Buthinae*-Skorpionen stammen, möglich (Zlotkin, 1986).

Trotz seiner extrem niedrigen Toxizität gegenüber Säugetieren zeigt das LqhP35-Toxin eine starke funktionelle und strukturelle Ähnlichkeit mit den α-Toxinen, die Säugetiere betreffen und aus den Giften von *Buthinae*-Skorpionen stammen und die sich an die Spannungs-abhängigen oder Tor-Strukturen der Natrium-Kanäle in Wirbeltieren binden und sie sondieren (Catterall, 1984):

(a) es bewirkt eine extreme Verlängerung der Aktionspotentiale in einem Präparat eines Insektenaxons (etwa zwei Grössenordnungen niedrigere Konzentration als beim wirkungsvollen, gegen Säugetiere wirkenden α-Toxin AaH2) und einer isolierten Sketettmuskelfaser der Ratte (wenigstens eine Grössenordnung höhere Konzentration als beim AaH2-Toxin), zurückzuführen auf die Verlangsamung des Inaktivierungsprozesses des Natrium-Kanals.

(b) es weist eine Übereinstimmung mit den oben genannten, Säugetiere betreffenden α-Toxinen in der Aminosäurensequenz von etwa 75 % auf.

Das LqhP35-Toxin zeigt in seinem Molekulargewicht, seiner Basizität und seiner Aminosäurenzusammensetzung die typischen physikochemischen Eigenschaften von Polypeptidneurotoxinen aus Skorpionengift (Possani, 1984). Seine Pharmakologie zeigt jedoch bestimmte einzigartige Eigenschaften. Die Untersuchung der pharmakologischen Bedeutung des LqhP35-Toxins erfordert eine kurze Betrachtung der Skorpiontoxine, die Wirbeltiere betreffen, die sogenannten gegen Säugetiere wirksamen Toxine. Diese Toxine spielen eine wesentliche Rolle in der pharmakologischen und chemischen Charakterisierung der Na$^+$-Kanäle in erregbaren Geweben von Säugetieren (Catterall, 1984) und werden gewöhnlicherweise in zwei Kategorien eingeteilt: Die α-Toxine wie AaH2 oder LqqV betreffen die Natrium-Inaktivierung, besitzen eine Spannungs-abhängige Bindungsfähigkeit und positive Kooperativität mit lipidlöslichen Alkaloiden wie Veratridin (Catterall, 1984, Zlotkin et al., 1985).

Die aus den Giften von *Centruroides* und *Tityus* (Skorpione) abstammenden β-Toxine betreffen die Natrium-Aktiviervng, besitzen mögliche unabhängige Bindungsstellen, die von denen der α-Toxine verschieden sind und nicht synergistisch mit Veratridin reagieren (Couraud et al., 1982, Couraud und Jover, 1984).

Die Wechselwirkungen der gegen Insekten wirksamen erregenden Toxine (repräsentiert durch AaiT) mit den neuronalen Membranen der Insekten ähneln stark dem Effekt der β-Toxine in den neuronalen Systemen der Säugetiere, der sich in der Induktion wiederholten Feuerns (Pelhate und Zlotkin, 1981) und der Spannungs-unabhängigen Bindung (Gordon et al., 1984) zeigt. Vor diesem Hintergrund stellt sich heraus, dass das LqhP35-Toxin eine offensichtlich α-Toxin-ähnliche Wirkung auf die neuronale Insekten-Membran hat.

Die Ähnlichkeit zwischen LqhP35 und den α-Toxinen der Skorpiongifte hat zwei hauptsächliche Auswirkungen - eine elektrophysiologische und eine strukturelle. LqhP35 indu ziert in zwei verschiedenen erregbaren Gewebepräparaten den "klassischen" Effekt auf die Natrium-Inaktivierung, die vorher für die *Buthinae*-Skorpiongifte und deren abgeleitete, gegen Wirbeltiere aktiven Toxine gezeigt wurde (Catterall, 1980). Die zweite Ähnlichkeit mit den α-Toxinen zeigt sich in der Primärstruktur von LqhP35. Die Aminosäurensequenz von LqhP35 stimmt in etwa 75 % mit den α-Toxinen, aber nur in 17 % mit dern erregenden, gegen Insekten wirksamen Toxin überein. Mit anderen Worten ist die Ähnlichkeit zwischen dern LqhP35-Toxin und den α-Toxinen so gross wie die zwischen den α-Toxinen untereinander.

Obwohl das LqhP35-Toxin eine grosse strukturelle und pharmakologische Ähnlichkeit mit den α-Toxinen hat, zeigt sich für das LqhP35-Toxin eine sehr niedrige Toxizitat gegenüber Säugetieren, im Gegensatz zu seiner relativ hohen Toxizität gegenüber Insekten. Die typischen α-Toxine AaH1 und 2 und das β-Toxin Css2 sind inaktiv gegen *Sarcophaga*-Larven und zeigen keine spezifische Bindung an Neuronenpräparate von Insekten (Zlotkin et al., 1971c, Gordon et al., 1984).

Die starke Toxizität des vorliegenden LqhP35 gegenüber Arthropoden verdient Beachtung, weil derarti-

5

ge Substanzen als Modelle für der Aufklärung der Tiergruppenspezifität von Toxinen aus Skorpiongiften dienen können. Das Anliegen der vorliegenden Erfindung ist jedoch auch der pharmakologische Wert der α-Toxine in den Untersuchungen der Eigenschaften und der Funktion der Natrium-Kanäle von Wirbeltieren (Catterall, 1980 und 1984). Diese Toxine können als Marker und Sonden der Spannungs-stimulierten Konformationsänderungen dienen, die mit dem Mechanismus des Kanalöffnens verbunden sind (Catterall, 1984). Die starke Toxizität von LqhP35 gegenüber Insekten, gekoppelt mit dessen starker Wirkung auf die Natrium-Inaktivierung in einem Insektenaxon ist ein bedeutendes pharmakologisches Werkzeug für das Studium der Natrium-Leitfähigkeit im Zusammenhang mit der neuronalen Erregbarkeit von Insekten.

Das LqhP35-Neurotoxin kann wie oben angegeben als eine Sonde oder als ein Marker der Spannungs-stimulierten Konformationsänderung, die mit dem Mechanismus des Kanalöffnens in Zusammenhang steht, in den Untersuchungen der Natrium-Leitfähigkeit benutzt werden, die mit der Erregbarkeit von Insektenneuronen verbunden ist. Zusätzlich kann das LqhP35-Toxin als Insektizid erfindungsgemäss in der Bekämpfung von Insektenschädlingen eingesetzt werden.

Das Gift der Hundertfüssler *Scolopendra canidens* und *S. cinqulata* hat eine geringe Toxizität gegenüber Mäusen, aber einen raschen und wirksamen Effekt gegenüber Insekten.

Insbesonders hat das Gift von *S. canidens* aus der Umgebung des Toten Meeres in einer Dosis von 1 mg/10 g KG keine Toxizität für Mäuse, ist aber sehr wirksam gegenüber Insekten. Die Toxine von Hundertfüsslern können im Rahmen der vorliegenden Erfindung benutzt werden.

Hundertfüssler der Gattung *Scolopendra* greifen ihre Insektenbeutetiere (beispielsweise Heuschrecken, Fliegen usw.) an, indem sie sowohl mechanische als auch chemische Mittel verwenden. Zuerst wird das Insektenbeutetier durch die feste Umklammerung des Insekts durch die kraftvollen Beine des Hundertfüsslers eingefangen. Nachdem das Insektenbeutetier immobilisiert worden ist, injiziert der Hundertfüssler sein Gift in das Insekt. Die Injektion des Gifts induziert eine rasche Lähmung der Beute. Der Hundertfüssler ist resistent gegen sein eigenes Gift und gegen das Gift anderer Hundertfüssler derselben Art. Er kann einer Giftdosis widerstehen, die ausreichend ist, wenigstens 150 Heuschrecken desselben Gewichtes zu lähmen.

Im Feld gesammelte Hundertfüssler können für lange Zeit (etwa 1 Jahr) im Labor gehalten werden. Die Hundertfüssler werden vorzugsweise in Behältern gehalten, die ein Feuchtigkeit absorbierendes Substrat und eine Wasserversorgung enthalten. Einmal alle zwei Wochen werden die Hundertfüssler mit lebenden Insekten gefüttert.

Das Gift der Hundertfüssler der Gattung *Scolopendra* kann vorzugsweise für Forschungs-oder andere Zwecke gewonnen werden, indern die Giftdrüsen des Tieres "gemolken" werden. Das Giftmelken wird vorzugsweise durchgeführt, indem die Basis der Gift"zähne" elektrisch stimuliert wird. Das ausgepresste Gift wird vorzugsweise in Kapillarröhrchen aus Plastik gesammelt, die fest an den Spitzen der Gift"zähne" befestigt wurden.

Das durch derartiges Melken erhaltene Giftvolumen hängt von der Grosse des Hundertfüsslers ab, reicht aber allgemein von etwa 0,25 bis etwa 5 μl. Das Trockengewicht des Giftes entspricht etwa 25 %. Etwa 70 % des Trockengewichtes des Giftes ist Protein. Die Toxizität des Giftes in Lösung hält wenigstens fünf Tage bei Raumtemperatur an und sie ist resistent gegenüber Lyophilisierung.

Gift wird vorzugsweise von jeder geeigneten Hundertfüssler-Art gewonnen. Vorzugsweise wird Gift von *S. canidens* oder *S. cingulata* gewonnen. *S. canidens* und besonders *S. canidens* aus der Umgebung des Toten Meeres sind besonders bevorzugt. Die Erfindung kann jedoch auch mit anderen *S. canidens* oder mit anderen Hundertfüsslern der Gattung *Scolopendra* durchgeführt werden. Äquivalente Hundertfüssler-Gattungen und -Arten können ebenfalls erfindungsgemäss verwendet werden. Heuschrecken können durch Giftkonzentrationen von 0,25 bis 6,5 μg Gift/g KG gelähmt werden. Das Gift von *S. canidens* (Totes Meer) ist das wirkungsvollste gegen Insekten und hat keinen Effekt gegenüber Mäusen.

Die Toxizität das Hundertfüssler-Giftes wird durch proteolytische Enzyme zerstört, was nahelegt, dass die aktive Komponente des Giftes der Proteinbestandteil ist. Auftrennungen von Hundertfüssler-Gift über eine Molekularsiebsäule in einem HPLC-System zeigen das Vorkommen aktiver Komponenten im MG-Bereich von 10 bis 130 kD an.

Es ist möglich, das erfindungsgemässe, von Hundertfüsslern gewinnbare selektiv auf Insekten wirkende Toxin zu reinigen, indem Mittel wir HPLC, Molekularsieb-Chromatographie, Elektrophorese usw. angewendet werden. Beispielsweise kann das Toxin über HPLC aufgetrennt werden und die isolierten Fraktionen können auf ihre Kapazität getestet werden, Insekten zu hemmen oder zu töten. Fraktionen mit einer derartigen insektiziden Aktivität können gegebenenfalls einer weiteren Reinigung unterworfen werden, bis eine Toxin-Probe im wesentlichen von ihren natürlichen Verunreinigungen befreit ist. Chromatographie-Techniken sind in der Fachwelt wohlbekannt und können vom Fachmann für erfindungsgemässen Zwecke leicht adaptiert werden.

Alternativ dazu können die Toxin-Moleküle mit immunologischen Mitteln, insbesondere der Immunaffinitäts-Chromatographie gereinigt werden.

Hier und im folgenden ist unter dem Begriff "von Hundertfüsslern gewinnbares, selektiv auf Insekten wirkendes Toxin" ein chemisches Toxin, das identisch oder im wesentlichen identisch ist mit der insektiziden Komponente des Hundertfüssler-Giftes zu verstehen. Das erfindungsgemässe "von Hundertfüsslern gewinnbare selektiv auf Insekten wirkende Toxin" kann entweder von einem Hundertfüssler, durch Peptid- oder andere synthetische Chemie oder durch die Anwendung molekularbiologischer Techniken gewonnen werden. Das Toxin wird als selektiv bezeichnet, wenn es fähig ist, ein Insekt zu beeinträchtigen, auf ein Nicht-Insekt jedoch keinen oder nur einen unwesentlichen Effekt hat.

## II. Antikörper gegen Neurotoxine

Ein weiterer Gegenstand der vorliegenden Erfindung sind Antikörper gegen die erfindungsgemässen Neurotoxine. Im folgenden werden verschiedene Verfahren referiert, die dem Fachmann der Immunologie wohlvertraut sind. Standardarbeiten, die die allgemeinen Prinzipien der Immunologie darlegen, sind Klein (1982), Kennett et al. (1980), Campbell (1984) und Eisen (1980).

Ein Antikörper ist dann funktionsfähig, wenn er in der Lage ist, spezifisch mit einem Antigen zu reagieren und dadurch das Antigen an den Antikörper zu binden. Der Ausdruck "Epitop" bezieht sich auf den Teil eines Haptens, der von einem Antikörper erkannt und gebunden werden kann. Ein Antigen kann ein Epitop oder mehr als ein Epitop haben. Ein "Antigen" ist fähig, in einem Tier die Bildung eines Antikörpers zu induzieren, der ein Epitop dieses Antigens binden kann. Die spezifische Reaktion, auf die oben Bezug genommen wird, ist so zu verstehen, dass das Antigen in einer höchst selektiven Art und Weise mit dem entsprechenden Antikörper reagiert und nicht mit der Vielzahl von anderen Antikörpern, die von anderen Antigen hervorgerufen worden sein können.

Die Begriffe "Antikörper" (Ab) und "monoklonaler Antikörper" (MAb) sind hier so zu verstehen, dass sowohl intakte Moleküle als auch Fragmente davon (beispielsweise Fab-und F(ab')₂-Fragmente), die fähig sind, eine Antigen zu binden, inbegriffen sind. Fab- und F(ab')₂-Fragmenten fehlt das Fc-Fragment intakter Antikörper, sie verschwinden rascher aus dem Blutkreislauf und sie könnten eine geringere unspezifische Gewebebindung aufweisen als ein intakter Antikörper (Wahl et al., 1983).

Um mit der Antikörper-Affinitäts-Chromatographie ein selektiv auf Insekten wirkendes Toxin zu reinigen, ist es notwendig, einen Antikörper zu verwenden, der das Toxin binden kann. Vorzugsweise ist ein derartiger Antikörper ein monoklonaler Antikörper.

Die erfindungsgemässen Antikörper können durch irgendeines einer Vielzahl von Verfahren hergestellt werden. Beispielsweise können Zellen, die das Neurotoxin oder ein Fragment davon exprimieren, einem Tier verabreicht werden, um die Produktion von Seren zu induzieren, die polyklonale Antikörper, die zur Bindung des Neurotoxins fähig sind, enthalten. Vorteilhafterweise wird ein Neurotoxin hergestellt und gereinigt, um es im wesentlichen frei von Kontaminationen zu erhalten. Ebenfalls vorteilhaft ist es, ein Neurotoxin-Fragment gemäss den dem Fachmann bekannten Verfahren zu synthetisieren.

Sowohl das gereinigte Fragment als auch das synthetisierte Fragment oder auch eine Kombination von gereinigtem natürlichen und synthetischem Fragment können in ein Tier eingebracht werden, um polyklonale Antiseren mit grösserer Spezifität zu produzieren.

Im bevorzugtesten erfindungsgemässen Verfahren sind die Antikörper monoklonale Antikörper. Derartige monoklonale Antikörper können unter Anwendung der Hybridoma-Technik hergestellt werden (Köhler und Milstein, 1975 und 1976, Köhler et al., 1976, Hämmerling et al., 1981). Ganz allgemein gesehen beinhalten solche Verfahren, ein Tier mit einem Neurotoxin-Antigen zu immunisieren.Die Splenozyten solcher Tiere werden extrahiert und mit einer geeigneten Myeloma-Zellinie fusioniert. Jede geeignete Myeloma-Zellinie kann erfindungsgemäss verwendet werden; es ist jedoch vorteilhaft, die Eltern-Myeloma- Zellinie (SP₂O) zu verwenden, die von der ATCC bezogen werden kann. Nach der Fusion werden die resultierenden Hybridoma-Zellen in HAT-Medium kultiviert und anschliessend durch limitierende Verdünnung kloniert, wie von Wands und Zurawski (1981) beschrieben. Die durch eine derartige Selektion erhaltenen Hybridoma-Zellen werden anschliessend geprüft, um solche Klone zu identifizieren, die Antikörper sezernieren, die das Neurotoxin-Antigen binden können.

Wenn die Toxinquelle unsauber ist, werden nur einige der Hybridoma-Zellen Antikörper bilden, die fähig sind, das Toxin zu binden (andere Hybridoma-Zellen werden Antikörper bilden, die fähig sind, die Verunreinigungen des Toxins zu binden). So kann es nötig sein, unter den Hybridoma-Zellen nach denjenigen zu suchen, die fähig sind, einen Antikörper zu sezernieren, der fähig ist, das Toxin zu binden. Eine derartige Suche wird vorzugsweise durchgeführt, indem eine Probe des Toxins (oder Giftes) mit einem monoklonalen Antikörper inkubiert wird, der von jeder einer Gruppe bestimmter Hybridoma-Zellen sezerniert wird, und indem Hybridoma-Zellen identifiziert werden, die einen Antikörper sezernieren können, der fähig ist, die Fähigkeit des Giftes, ein Insekt zu lähmen, zu neutralisieren oder abzuschwächen. Wenn eine derartige Hybridoma-Zelle identifiziert worden ist, kann sie mit Hilfe von in der Fachwelt bekannten Mitteln vermehrt werden, um den Toxin-spezifischen monoklonalen Antikörper zu produzieren.

Nachdem ein Toxin-spezifischer monoklonaler Antikörper erhalten worden ist, kann er durch die Bindung an einen festen Träger immobilisiert werden und verwendet werden, das Toxin aus natürlichem Gift oder aus anderen Quellen zu reinigung, indem Immunaffinitäts-Chromatographie gemäss dem Fachmann bekannter Verfahren angewendet wird. Durch derartige Verfahren kann ein hoher Reinheitsgrad erhalten werden und dadurch ein Toxin, das im wesentlichen frei von natürlichen Verunreinigungen ist. Hier und im folgenden bedeutet der Ausdruck "im wesentlichen frei von natürlichen Verunreinigungen", dass das Toxin in einer Form vorliegt, die keine von den Stoffen enthält, mit denen es natürlicher- und normalerweise assoziiert ist (beispielsweise andere Proteine, Lipide, Kohlenhydrate usw.).

Wenn das Toxin gereinigt worden ist, dann es benutzt werden, um ein Tier (beispielsweise eine Maus oder ein Kaninchen) zu immunisieren, um die Produktion eines Toxin-spezifischen polyklonalen Antikörpers anzuregen.

Somit betrifft die Erfindung auch derartige Toxin-spezifische mono- und polyklonale Antikörper. Ein weiterer erfindungsgemässer Aspekt betrifft Hybridoma-Zellen, die einen Toxin-spezifischen monoklonalen Antikörper produzieren können.

Durch die Anwendung der oben beschriebenen Verfahren können zusätzliche Zellinien erhalten werden, die fähig sind, Antikörper zu produzieren, die Epitope des gewünschten selektiv auf Insekten wirkenden Toxins erkennen. Die Anwendung der oben beschriebenen Verfahren reicht aus, um die Gewinnung eines hochgereinigten Toxinpräparates zu gewährleisten.

Durch die Anwendung der oben beschriebenen Verfahren können zusätzliche Zellinien erhalten werden, die Antikörper produzieren, welche die Epitope des gewünschten, selektiv auf Insekten wirkenden Toxins erkennen.


III. Gentechnik mit den selektiv auf Insekten wirkenden Toxinen

Die vorliegende Erfindung betrifft auch die Gen-Sequenzen, die die selektiv auf Insekten wirkenden Toxine kodieren, Expressionsvehikel, die diese Gen-Sequenzen enthalten, damit transformierte Wirtszellen, das Toxin, das durch die transformierte Wirtsexpression produziert wird, und die Verwendungen der rekombinanten Toxine.

Es gibt zahlreiche Verfahren, die man benutzen kann, um die das Toxin kodierende Gensequenz zu klonieren. Eines von diesen Verfahren basiert darauf, dass eine shuttle vector-Bibliothek von cDNA-Einschüben (abgeleitet von einer ein Toxin exprimierenden Zelle) auf das Vorhandensein eines Einschubs analysiert wird, der die Toxin-Gensequenz enthält.

Bei dieser Vorgehensweise werden Zellen mit dem Vektor transfiziert und anschliessend auf die Toxin-Expression hin untersucht.

Zur Klonierung der Toxin-Gensequenz lässt sich auch die Aminosäurensequenz des Toxin-Moleküls bestimmen. Dazu wird das Toxin-Protein gereinigt (wie oben beschrieben) und analysiert, um die Aminosäurensequenz des Toxin-Proteins zu bestimmen, wobei jede Sequenzanalyse verwendbar ist, insbesondere der Edman-Abbau, ganz besonders bevorzugt ist jedoch der Einsatz automatischer Sequenzierapparate.

Die Sequenz der einzelnen Aminosäurebestandteile wird hier durch den Gebrauch ihrer gemeinhin verwendeten Ein-Buchstaben-Bezeichnungen beschrieben. Eine Auflistung dieser Ein-Buchstaben- und der Drei-Buchstaben-Bezeichnungen ist in Lehrbüchern wie von Lehninger (1975) nachzulesen. Bei horizontaler Schreibweise der Aminosäurensequenz steht das Aminoende links und das Carboxylende rechts.

Ist die Aminosäurensequenz bekannt, so lassen sich daraus DNA-Sequenzen ableiten, die fähig sind, das gewünschte Protein zu kodieren. Ein solches Gen kann dann kloniert werden. Da der genetische Kode degeneriert ist, gibt es bekanntlich mehr als ein Codon, das eine bestimmte Aminosäure kodiert (Watson, 1977).

Obwohl es möglich ist, die gesamte Aminosäurensequenz des Toxins zu bestimmen, genügt es im allgemeinen, die Sequenz eines Fragments des Moleküls zu bestimmen und die resultierenden Sequenzdaten zur Herstellung einer Oligonukleotidsonde zu benutzen, mit der die gesamte Toxin-Gensequenz herausgefunden werden kann. Toxin-Peptidfragmente können durch Inkubation des intakten Moleküls mit Cyanbromid oder mit Proteasen wie Papain, Chymotrypsin oder Trypsin erhalten werden (Oike et al., 1982, Liu et al., 1983).

Unter Verwendung des genetischen Kodes (Watson, 1977) können ein Oligonukleotid oder mehrere verschiedene Oligonukleotide identifiziert werden, von denen jedes fähig ist, die Toxin-Peptide zu kodieren. Die Wahrscheinlichkeit, dass ein bestimmtes Oligonukleotid tatsächlich die eigentliche, das Toxin kodierende Sequenz sein wird, kann durch die Beachtung abnormer Basenpaarungsverhältnisse und die Häufigkeit, mit der ein bestimmtes Codon wirklich in eukaryotischen Zellen (um eine bestimmte Aminosäure zu kodieren) benutzt wird, abgeschätzt werden. Derartige Regeln zur Codon-Benutzung ("Codon usage rules") sind in Lathe (1985) veröffentlicht. Unter Verwendung dieser Regeln zur Codon-Benutzung von Lathe wird das Oligonukleotid oder ein Set von Oligonukleotiden identifiziert, das mit grösster Wahrscheinlichkeit die die Toxin-Peptidsequenzen kodierende Nukleotidsequenz enthält.

Dieses resultierende Oligonukleotid oder Set von Oligonukleotiden wird benutzt, um die Sequenz eines komplementären Oligonukleotids oder eines Sets von Oligonukleotiden zu identifizieren, das/die fähig ist/sind, mit der "höchst wahrscheinlichen" Sequenz oder dem "höchst wahrscheinlichen" Set von Sequenzen zu hybridisieren. Ein Oligonukleotid, welches eine derartige komplementäre Sequenz enthält, kann als Sonde verwendet werden, um das Toxin-Gen zu identifizieren und zu isolieren (Maniatis et al., 1982).

Zusammengefasst erlaubt somit die tatsächliche Identifizierung von Toxin-Peptidsequenzen die Identifizierung einer theoretischen "höchst wahrscheinlichen" DNA-Sequenz oder eines Sets von DNA-Sequenzen, die fähig ist/sind, ein derartiges Peptid zu kodieren. Wenn die Fragmente mehr als 10 Aminosäuren aufweisen, reicht die Sequenzinformation im allgemeinen bereits aus, um die Klonierung einer das Toxin kodierenden Gensequenz zu gewährleisten. Durch die Konstruktion eines Oligonukleotids, das der theoretischen Sequenz komplementär ist (oder durch die Konstruktion eines Sets von Oligonukleotiden, die dem Set der "höchst wahrscheinlichen" Oligonukleotide komplementär sind), wird ein DNA-Molekül (oder ein Set von DNA-Molekülen) erhalten, das fähig ist, als Sonde zur Identifizierung und Isolierung des Toxin-Gens benutzt zu werden.

Die Klonierung und die Verwendung der verschiedenen oben beschriebenen Toxine wird hier und im folgenden allgemein als die Klonierung und die Verwendung "eines Toxins" bezeichnet. Es ist so zu verstehen, dass jedes der genannten Toxine in jedem der beschriebenen erfindungsgemässen Verfahren zu verwenden ist. Die Verwendung der erfindungsgemässen Gentechniken in Bezug auf das Toxin wird durch die Klonierung von Gen-Sequenzen, die das Toxin kodieren, und durch die Expression derartiger Sequenzen erleichtert. Hier und im folgenden bezieht sich der Begriff "Gen-Sequenzen" auf ein Nukleinsäuremolekül (vorzugsweise DNA). Gen-Sequenzen, die das Toxin kodieren, können aus einer Vielzahl von Quellen stammen. Beispiele solcher Quellen sind genomische DNA, cDNA, synthetische DNA und Kombinationen davon.

Genomische DNA kann gegebenenfalls natürlich vorkommende Introns enthalten. Darüberhinaus kann eine derartige genomische DNA in Verbindung mit der 5'-Promotor-Region der Toxin-Gensequenzen erhalten werden. So weit wie eine Wirtszelle die Regulationssignale der Transkription und die Initiationssignale der Translation erkennen kann, die mit der Expression des Proteins verbunden sind, kann die 5'-Region erhalten bleiben und für die Initiationsregulation der Transkription und Translation verwendet werden.

Im Fall der cDNA kann diese kloniert und der resultierende Klon mit einer geeigneten Sonde für cDNA, die die gewünschten Sequenzen kodiert, herausgesucht werden. Wenn der gewünschte Klon erst einmal isoliert worden ist, kann die cDNA in im wesentlichen derselben Weise gehandhabt werden wie die genomische DNA. Die cDNA enthält jedoch keine Introns oder dazwischenliegenden Sequenzen. Aus diesem Grund ist ein cDNA-Molekül, welches das Toxin kodiert, die bevorzugte Gen-Sequenz der vorliegenden Erfindung.

Genomische DNA bzw. cDNA kann auf mehreren Wegen erhalten werden. Genomische DNA kann aus geeigneten Zellen mit dem Fachmann gut bekannten Verfahren extrahiert und gereinigt werden. Alternativ dazu kann mRNA aus einer Toxin-produzierenden Zelle isoliert und dazu benutzt werden, um mit dem Fachmann gut bekannten Verfahren cDNA zu produzieren. Derartige geeignete DNA-Präparate werden enzymatisch gespalten oder zufallsmässiggeschert und mit rekombinanten Vektoren verknüpft, um eine Gen-Bibliothek aufzubauen. Derartige Vektoren können anschliessend mit den oben beschriebenen Oligonukleotid-Sonden gescreent werden, um eine ein Toxin kodierende Sequenz zu identifizieren.

Ein geeignetes Oligonukleotid oder ein Set von Oligonukleotiden, das/die fähig ist/sind, ein Fragment des Toxins zu kodieren (oder das einem derartigen Oligonukleotid oder einem Set von Oligonukleotiden komplementär ist), welches nach dem oben beschriebenen Verfahren identifiziert worden ist, wird synthetisiert und mit Mitteln, die dem Fachmann bekannt sind, mit einer DNA oder vorzugsweise mit einem cDNA-Präparat hybridisiert, die/das aus Zellen abgeleitet ist, die fähig sind, das Toxin-Gen zu exprimieren. Die Quelle der verwendeten DNA oder cDNA wird vorteilhafterweise vorher mit Toxin-Sequenzen angereichert. Eine derartige Anreicherung kann am leichtesten mit cDNA erreicht werden, die erhalten wurde, indem RNA aus Zellen extrahiert wurde, die grosse Mengen des Toxins produzieren. Techniken der Nukleinsäurenhybridisierung sind in Maniatis et al. (1982) und Hames und Higgins (1985) veröffentlicht.

Um das Herausfinden der das Toxin kodierenden Sequenz zu erleichtern, kann die DNA-Sonde mit einer nachweisbaren Gruppe markiert sein. Eine derartige nachweisbare Gruppe kann jeder Stoff mit einer auffindbaren physikalischen oder chemischen Eigenschaft sein. Derartige Stoffe sind auf dem Gebiet der Immunassays gut entwickelt. Ganz allgemein kann nahezu jede Markierung, die in derartigen Verfahren geeignet ist, erfindungsgemäss verwendet werden. Besonders gut geeignet sind enzymatisch aktive Gruppen, wie Enzyme (Wisdom, 1976), Enzymsubstrate (GB 1,584,741), Coenzyme (US 4,230,797 und US 4,238,565) und Enzym-Inhibitoren (US 4,134,792); Fluoreszenzfarbstoffe (Soini und Hemmilä, 1979); chromophore Stoffe; Luminiszenzfarbstoffe wie Chemi- und Bioluminiszenzfarbstoffe (Gorus und Schram, 1979); spezifisch bindungsfähige Liganden; proximale, zusammenwirkende Paare; und Radioisotope wie $^3$H, $^{35}$S, $^{32}$P, $^{125}$I und $^{14}$C. Derartige Markierung und Markierungspaare werden auf der Basis ihrer eigenen physikalischen Eigenschaften (beispielsweise Fluoreszenzfarbstoffe, chromophore Stoffe und Radioisotope) oder ihrer reaktiven oder Bindungseigenschaften (beispielsweise Enzyme, Substrate, Coenzyme und Inhibitoren) nachgewiesen. Beispielsweise kann eine mit einem Cofaktor markierte Sonde nachgewiesen werden, indem das Enzym, für das der Marker Cofaktor ist, und ein Substrat des Enzyms hinzugefügt werden. Beispeilsweise kann ein Enzym benutzt werden, das auf ein Substrat derartig einwirkt, dass ein Produkt mit einer messbaren physikalischen Eigenschaft entsteht. Beispiele für solche Enzyme schliessen $\beta$-Galaktosidase, alkalische Phosphatase und Peroxidase ein, ohne jedoch darauf beschränkt zu sein.

Allgemeine Verfahren zur Hybridisierung sind beispielsweise in Maniatis et al. (1982) und in Hames und Higgins (1985) publiziert. Diejenigen Mitglieder der oben beschriebenen Gen-Bibliothek, die zu einer derartigen Hybridisierung fähig sind, werden anschliessend analysiert, um das Ausmass und die Natur der in ihnen enthaltenen, das Toxin-kodierenden Sequenzen zu bestimmen.

In einem alternativen Verfahren, das Toxin-Gen zu klonieren, wird eine Bibliothek von Expressionsvektoren angelegt, indem DNA oder bevorzugt cDNA, die aus einer Zelle stammt, die fähig ist, das Toxin zu exprimieren, in einem Expressionsvektor kloniert wird. Die Bibliothek wird anschliessend auf Mitglieder hin untersucht, die zur Expression eines proteins fähig sind, das einen Anti-Toxin-Antikörper bindet, und die eine Nukleotidsequenz aufweisen, die fähig ist, Polypeptide zu exprimieren, die dieselbe Aminosäurensequenz haben wie das Toxin oder Fragmente des Toxins.

Die klonierten, das Toxin kodierenden Sequenzen, die durch die oben beschriebenen Verfahren erhalten werden, können funktionstüchtig mit einem Expressionsvektor verknüpft sein und in bakterielle oder eukaryotische Zellen eingeführt werden, um das Toxin oder ein funktionelles Derivat davon zu produzieren.Techniken für derartige Handhabungen sind in Maniatis et al. (1982) veröffentlicht und in der Fachwelt gut bekannt.

Mit den oben diskutierten Verfahren können deshalb Gen-Sequenzen identifiziert werden, die fähig sind, das Toxin oder Derivate davon zu kodieren. Um derartige Gen-Sequenzen weiter zu charakterisieren, ist es wünschenswert, die Toxine, die von diesen Sequenzen kodiert werden, zu exprimieren und zu bestätigen, dass sie die Eigenschaften der Toxin-Peptide besitzen. Solche Eigenschaften sind die Fähigkeit, spezifisch Anti-Toxin-Antikörper zu binden, die Fähigkeit, die Produktion eines zur Bindung an das Toxin fähigen Antikörpers auszulösen, die Fähigkeit, einer Empfänger-Zelle eine Toxin-Funktion zu verleihen, etc..

An Stelle der oben beschriebenen Verfahren zur Rekombination kann eine Gensequenz, die das Toxin kodiert, auch synthetisch hergestellt werden (beispielsweise durch organische Synthese).

Ein alternatives Verfahren, um eine Gen-Sequenz zu erhalten, die fähig ist, das Toxin zu kodieren, ist, sie durch Oligonukleotid-Synthese herzustellen. Dieses Verfahren ist speziell bei Proteinen mit weniger als 100 Aminosäuren, wie den erfindungsgemässen Toxinen anwendbar. Der genetische Kode wird benutzt, um die Oligonukleotid-Sequenz zu bestimmen, die fähig ist, die Aminosäurensequenz zu kodieren.

Bei der Vorhersage der Oligonukleotid-Sequenz wird vorteilhafterweise die passende Codon-Häufigkeit, des Organismus, in dem das Gen exprimiert werden soll, berücksichtigt. Derartige Codon-Häufigkeiten für einige Organismen sind als Teil eines Sequenzanalyse-Computerprogramms der Genetics Computer Group der Universität von Wisconsin erhältlich. Codon-Häufigkeiten für weitere Organismen können mit Hilfe desselben Computer-Programm-Paketes berechnet werden, indem Daten aus den verfügbaren Sequenzdatenbanken verwendet werden. In einigen Fällen können alternative Codons ausgewählt werden, um die Synthese zu erleichtern und/oder für passende Restriktionsschnittstellen zu sorgen. Stop- und Startsignale der Translation werden an den geeigneten Punkten eingeführt und Sequenzen an die Enden angefügt, um passende Klonierungsstellen zu schaffen. Die jeweils obere Nukleotidsequenz stellt den "kodierenden Strang" dar. Die Sequenz des "komplementären Strangs" wird mit den oben erwähnten Computerprogrammen vorhergesagt.

Eine Serie von Oligonukleotiden, deren Länge von 20 bis 50 Basen reicht, wird synthetisiert, um eine Serie von überlappenden Fragmenten zu schaffen, die beide Stränge des Gens bilden, wenn sie später gepaart und verknüpft sind. Diese Fragmente werden anschliessend gepaart und miteinander verknüpft, indem Verfahren angewendet werden, die dem Fachmann gut bekannt sind (Maniatis et al., 1982). Das resultierende DNA-Fragment mit der vorhergesagten Grösse wird durch eine Elektrophorese isoliert und mit einem geeigneten Klonierungsvektor zur Amplifikation und weiteren Handhabung verknüpft. Dieses synthetische Gen kann unter Verwendung der oben für die aus genomischer und/oder cDNA isolierten Gene beschriebenen Techniken gehandhabt werden.

IV. Expression des selektiv auf Insekten wirkenden Toxins und seiner funktionellen Derivate.

Die das Toxin kodierenden Sequenzen, die mit den oben beschriebenen Verfahren erhältlich sind, können gemäss an sich bekannter Verfahren funktionstüchtig mit einem Expressionsvektor verknüpft werden und in pro- oder eukaryotische Zellen eingeführt werden, um das Toxin oder dessen funktionelle Derivate zu produzieren. Die vorliegende Erfindung betrifft sowohl das intakte Toxin als auch funktionelle Derivate bzw. Bruchstücke dieses Toxins.

Hier und im folgenden bedeutet der Begriff "im wesentlichen rein" oder "im wesentlichen gereinigt", dass das Neurotoxin keine verunreinigenden Substanzen enthält, die normalerweise im natürlichen Isolat vorkommen, und somit namentlich frei von Protein-und Kohlenhydrat-Bestandteilen ist. Mit den Begriff ist ferner ein Neurotoxin gemeint, das auf Grund anerkannter Reinheits- oder Homogenitätskriterien als homogen bezeichnet werden kann. Beispielsweise zeigt ein im wesentlichen reines Neurotoxin konstante und reproduzierbare Eigenschaften innerhalb gewisser standardmässiger Abweichungen bei Parametern wie den folgenden: MG, chromatographisches Verhalten und andere derartige Parameter. Der Begriff ist jedoch nicht so zu verstehen, dass er künstliche oder synthetische Mischungen des Neurotoxins mit anderen Verbindungen ausschliesst. Der Begriff ist auch nicht so zu verstehen, dass er die Gegenwart von geringfügigen Unreinheiten, die die biologische Aktivität des Neurotoxins nicht stören und die beispielsweise auf Grund unvollständiger Reinigung vorhanden sein können, ausschliesst.

Ebenso werden die Begriffe "LqhP35", "das LqhP35-Neurotoxin" und "das LqhP35-Toxin" austauschbar und beispielhaft benutzt, um ein vom Skorpiongift abgeleitete Toxin zu benennen. Die vorliegende Erfindung betrifft sowohl das intakte Neurotoxin als auch die funktionellen Derivate des LqHP35-Toxins.

Ein "funktionelles Derivat" des Toxins ist ein Stoff, der eine biologische Aktivität (entweder funktionell oder strukturell) besitzt, die im wesentlichen einer biologischen Aktivität des Toxins ähnlich ist. Der Ausdruck "funktionelles Derivat" ist so zu verstehen, das er die "Fragmente", "Varianten", "Analogen" oder "chemischen Derivate" eines Moleküls einschliesst. Mit einem "Fragment" eines Moleküls wie das des Toxins ist jedes Polypeptidteilstück des Moleküls gemeint. Der Begriff eine "Variante" eines Moleküls wie das des Toxins ist so zu verstehen, das sie sich auf ein Molekül bezieht, das im wesentlichen in der Struktur und in der Funktion entweder dem gesamten Molekül oder einem Fragment davon ähnlich ist. Ein Molekül wird als "im wesentlichen ähnlich" zu einem anderen Molekül angesehen, wenn beide Moleküle im wesentlichen ähnliche Strukturen haben und/oder wenn beide Moleküle eine ähnliche biologische Aktivität aufweisen. Hier und im folgenden werden deshalb zwei Moleküle als Varianten bezeichnet, wenn sie eine ähnliche Aktivität zeigen, selbst dann, wenn die Struktur des einen Moleküls nicht in der des anderen wiedergefunden wird, oder wenn die Aminosäurensequenzen nicht identisch sind. Mit dem "Analogen" eines Moleküls wie das des Toxins soll ein Molekül gemeint sein, dass in der Funktion entweder dem gesamten Molekül oder einem Fragment davon im wesentlichen ähnlich ist. Hier und im folgenden wird ein Molekül dann als ein "chemisches Derivat" eines anderen Moleküls angesehen, wenn es zusätzliche chemische Anteile enthält, die normalerweise keinen Teil des Moleküls bilden. Derartige Anteile können die Löslichkeit, die Absorption, die biologische Halbwertzeit etc. verbessern. Anteile, sie solche Effekte hervorrufen können, sind in Osol, A. (Hrsg.) Remington's Pharmaceutical Sciences (Mack Publishing Co., Easton, PA, 1980) veröffentlicht. Verfahren, mit denen solche Anteile an ein Molekül gekoppelt werden können, sind dem Fachmann wohlbekannt.

Eine DNA-Sequenz, die ein erfindungsgemässes Toxin oder dessen funktionelle Derivate kodiert, kann mit Vektor-DNA gemäss herkömmlicher Techniken rekombiniert werden, beispielsweise Verwendung von glatten oder kohäsiven Enden bei der Verknüpfung, Schneiden mit Restriktionsenzymen zur Erzeugung geeigneter Enden, geeignetes Auffüllen kohäsiver Enden, Behandlung mit alkalischer Phosphatase, um unerwünschte Verbindungen zu vermeiden, und das Verknüpfen mit geeigneten Ligasen. Techniken für derartige Handhabungen sind in Maniatis et al. (1982) veröffentlicht und der Fachwelt bekannt.

Ein Nukleinsäuremolekül wird als "fähig zur Expression" eines Polypeptids angesehen, wenn es Nukleotidsequenzen enthält, die wiederum Informationen zur Regulation von Transkription und Translation enthalten, und wenn derartige Sequenzen "funktionstüchtig verknüpft" mit Nukleotidsequenzen sind, die das Polypeptid kodieren. Eine funktionstüchtige Verknüpfung ist eine Verknüpfung, in welcher die regulatorischen DNA-Sequenzen und die zu exprimierende DNA-Sequenz derartig verknüpft sind, dass damit Genexpression ermöglicht wird. Die genaue Natur der für die Genexpression benötigten regulatorischen Bereiche können von Organismus zu Organismus verschieden sein, im allgemeinen enthalten sie jedoch eine Promotor-Region, die in Prokaryoten sowohl den Promotor (der die Initiation der RNA-Transkription lenkt) als auch die DNA-Sequenzen enthält, die, wenn sie in RNA transkribiert werden, die Initiation der Toxin-Synthese signalisieren. Derartige Regionen enthalten normalerweise diejenigen 5′-nicht-kodierenden Sequenzen, die an der Initiation der Transkription und der Translation beteiligt sind, wie die TATA-Box, die capping-Sequenz, die CAAT-Sequenz und dergleichen.

Wenn es gewünscht wird, kann die nicht-kodierende Region 3′ zur Gensequenz, die das Toxin kodiert, durch die oben beschriebenen Verfahren erhalten werden. Diese Region kann wegen ihrer regulatorischen Sequenzen für die Beendigung der Transkription, wie Termination und Polyadenylierung, beibehalten werden. Somit können durch die Beibehaltung der sich natürlicherweise an die DNA-Sequenz, die das Toxin kodiert, anschliessenden 3′-Region die Terminationssignale für die Transkription beschafft werden. In den Fällen, in denen die Terminationssignale für die Transkription in der Expression der Wirtszelle nicht befriedigend funktionieren, können sie durch eine 3′-Region, die in der Wirtszelle funktioniert, ersetzt werden.

Zwei DNA-Sequenzen (beispielsweise eine Sequenz aus der Promotor-Region und die das Toxin-kodierende Sequenz) werden als funktionstüchtig verknüpft angesehen, wenn die Natur der Verknüpfung zwischen den zwei DNA-Sequenzen nicht (1) in der Einführung einer Leseraster-Verschiebungs-Mutation resultiert, (2) die Fähigkeit der Promotorsequenz-Region, die Transkription der Toxin-Gensequenz zu lenken, stört oder (3) die Fähigkeit der Toxin-Gensequenz, durch die Promotorsequenz-Region transkribiert zu werden, stört. Eine Promotor-Region ist funktionstüchtig verknüpft mit einer DNA-Sequenz, wenn der Promotor fähig ist, die Transkription dieser DNA-Sequenz zu beeinflussen.

Um das Toxin zu exprimieren sind somit Transkriptions- und Translationssignale erforderlich, die von einem geeigneten Wirt erkannt werden können.

Die vorliegende Erfindung betrifft auch die Expression des Toxin-Proteins (oder eines funktionellen Derivats davon) in sowohl prokaryotischen als auch eukaryotischen Zellen. Bevorzugte prokaryotische Wirte sind beispielsweise Bakterien wie *E. coli, Bacillus, Streptomyces, Pseudomonas, Salmonella, Serratia,* usw.. Der bevorzugteste prokaryotische Wirt ist *E. coli.* Bakterielle Wirte von besonderem Interesse sind beispielsweise *E. coli* K12 Stamm 294 (ATCC 31446), *E. coli* X1776 (ATCC 31537), *E. coli* W3110 (F⁻, λ⁻, prototrophisch (ATCC 27325) und andere Enterobakterien wie *Salmonella typhimurium* oder *Serratia marcescens* und verschiedene *Pseudomonas*-Arten. Unter derartigen Bedingungen wird das Toxin nicht glykosyliert. Der prokaryotische Wirt muss mit den Replikon- und Kontrollsequenzen im Expressionsplasmid kompatibel sein.

Um ein erfindungsgemässes Toxin (oder ein funktionelles Derivat davon) in einer prokaryotischen Zelle (wie *E. coli, B. subtilis, Pseudomonas, Streptomyces* usw.) zu exprimieren, ist es erforderlich, die das Toxin kodierende Sequenz funktionstüchtig mit einem funktionellen prokaryotischen Promotor zu verknüpften. Derartige Promotoren können entweder konstitutiv oder vorteilhafterweise regulierbar (d. h. induzierbar oder dereprimierbar) sein. Beispiele konstitutiver Promotoren sind der *int*-Promotor des Bakteriophagen λ, der *bla*-Promotor des β-Lactamase-Gens von pBR322, der CAT-Promotor des Chloramphenicol-Acetyl-Transferase-Gens von pBR325 usw.. Beispiele induzierbarer Promotoren sind die rechten und linken Promotoren des Bakteriophagen λ ($P_L$ und $P_R$), die *trp-, recA-, lacZ-, lacI-* und *gal*-Promotoren von *E. coli,* der α-Amylase-Promotor (Ulmanen et al., 1985) und die σ²⁸-spezifischen Promotoren von *B. subtilis* - (Gilman et al., 1984), die Promotoren der Bakteriophagen von *Bacillus* (Gryczan, 1982) und *Streptomyces*-Promotoren (Ward et al., 1986). Eine Übersicht über prokaryotische Promotoren vermitteln Glick und Whitney (1987), Cenatiempo (1986) und Gottesman (1984).

Eine effektive Expression in einer prokaryotischen Zelle erfordert auch die Anwesenheit einer Ribosomen-Bindungsstelle stromaufwärts (upstream) der das Gen-kodierenden Sequenz. Derartige Ribosomen-Bindungsstellen sind beispielsweise in Gold et al. (1981) veröffentlicht.

Nicht-limitierende Beispiele bevorzugter eukaryotischer Wirte sind Hefen, Pilze, Insekten-Zellen und Säugerzellen sowohl in vivo als auch in Gewebekultur. Als Wirte geeignete Säugerzellen sind beispielsweise Zellen, die von Fibroblasten, wie VERO oder CHO-K1, oder von Lymphzellen abstammen, wie das Hybridoma SP2/0-AG14 oder das Myeloma P3x63Sg8, und ihre Derivate. Bevorzugte Säuger-Wirtszellen sind beispielsweise SP2/0 und J558L ebenso wie Neuroblastoma-Zellinien wie IMR 332, die eventuell über bessere Kapazitäten für das post-translationale Processing verfügen.

Für Säugerwirtszellen stehen verschiedene mögliche Vektorsysteme für die Expression des Toxins zur Verfügung. Eine grosse Vielfalt von Regulationssequenzen der Transkription und Translation können je nach der Art des Wirts verwendet werden. Die Regulationssequenzen der Transkription und Translation können von viralen Quellen stammen, beispielsweise vom Adenovirus, Rinder-Papillomavirus, Simianvirus oder ähnlichen, in denen die Regulationssignale mit einem bestimmten Gen, das eine hohe Expressionsrate hat, gekoppelt sind. Alternativ dazu können Promotoren von Säuger-Expressionsprodukten, wie Aktin, Kollagen, Myosin etc. verwendet werden. Regulationssignale der Transkriptionsinitiation können ausgewählt werden, die eine Repression oder Aktivierung zulassen, so dass die Expression der Gene moduliert werden kann. Von Interesse sind Temperatur-sensitive Regulationssignale, so dass durch Veränderung der Temperatur die Expression reprimiert oder initiiert werden kann, oder auch Regulationssignale, die einer chemischen Regulation, beispielsweise durch einen Metaboliten, zugänglich sind.

Hefen weisen wesentliche Vorteile dadurch auf, dass sie auch post-translationale Peptidmodifikationen ausführen können. Es gibt eine Anzahl von Strategien zur Rekombination von DNA, die starke Promotor-Sequenzen und eine hohe Zahl von Plasmidkopien verwenden, die zur Produkten der gewünschten Proteine in Hefen genutzt werden können. Hefen erkennen sogenannte leader-Sequenzen klonierter Säuger-Genprodukte und sezernieren Peptide, die diese leader-Sequenzen tragen (d. h. Präpeptide)

Jedes einer Reihe von Gen-Expressionssystemen auf Hefe kann eingesetzt werden, das Promotor- und Terminationselemente aus aktiv exprimierten Genen enthält, die glykolytische Enzyme kodieren. Diese Enzyme werden in grossen Mengen produziert, wenn die Hefe in Medien gezogen wird, die reich an Glucose sind. Bekannte glykolytische Gene können auch sehr wirksame Kontrollsignale der Transkription beitragen. Beispielsweise können die Promotor- und Terminatorsignale des Phosphoglycerat-Kinase-Gens verwendet werden.

Andere bevorzugte Wirtszellen sind Insektenzellen, beispielsweise die der *Drosophila*-Larven. Wenn Insektenzellen als Wirte benutzt werden, kann der *Drosophila*-Alkohol-Dehydrogenase-Promotor verwendet werden (Rubin, 1988). Alternativ dazu können Baculovirus-Vektoren konstruiert werden, die grosse Mengen des Toxins in Insektenzellen exprimieren (Jasny, 1987, Miller et al., 1986).

Wie bereits oben diskutiert, erfordert die Expression des Toxins in eukaryotischen Wirtszellen den Einsatz eukaryotischer Regulationsregionen. Derartige Regionen umfassen im allgemeinen eine Promotor-Region, die zur Initiation der RNA-Synthese ausreicht. Bevorzugte eukaryotische Promotoren sind beispielsweise der Promotor des Metallothionein I-Gens der Maus (Hamer und Walling, 1982), der TK-Promotor des Herpesvirus (McKnight, 1982), der frühe Promotor von SV40 (Benoist und Chambon, 1981) und der *gal4*-Gen-Promotor aus Hefe (Johnston und Hopper, 1982, Silver et al., 1984).

Wie weithin bekannt ist, wird die Translation eukaryotischer mRNA an dem Codon initiiert, das das erste Methionin kodiert. Deshalb ist es vorteilhaft, sicherzustellen, dass die Verbindung zwischen einem eukaryotischen Promotor und einer DNA-Sequenz, die das Toxin (oder ein funktionelles Derivat davon) kodiert, keine dazwischenliegenden Codons enthält, die ein Methionin kodieren (d. h. AUG). Die Anwesenheit derartiger Codons resultiert entweder in der Bildung eines Fusionsproteins (falls das AUG-Codon im selben Leserahmen wie die das Toxin kodierende DNA-Sequenz ist) oder einer Leserahmenverschiebungs-Mutation (falls das AUG-Codon nicht im selben Leserahmen wie die das Toxin kodierende DNA-Sequenz ist).

Die ein erfindungsgemässes Toxin kodierende Sequenz und ein funktionstüchtig damit verknüpfter Promotor können als ein nicht-replizierendes DNA- (oder RNA-)Molekül, das entweder ein lineares Molekül oder, bevorzugter, ein ringgeschlossenes Molekül sein kann, in eine prokaryotische oder eukaryotische Empfängerzelle eingeführt werden. Da derartige Moleküle nicht zur autonomen Replikation fähig sind, kann die Expression des Toxins durch die vorübergehende Expression der eingeführten Sequenz erfolgen. Alternativ dazu kann eine dauernde Expression durch die Integration der Eingeführten Sequenz in das Wirtschromosom erfolgen.

Ein weiterer erfindungsgemässer Gegenstand betrifft Vektoren, die fähig sind, die gewünschten Gensequenzen in das Chromosom der Wirtszelle einzubauen. Zellen, bei denen die eingeführte DNA stabil in das Chromosom eingebaut ist, können dadurch selektioniert werden, dass ein Marker oder mehrere Marker ebenfalls eingeführt wird/werden, der/die die Selektion von Wirtszellen, die den Expressionsvektor enthalten, erlaubt/erlauben. Der Marker kann einen auxotrophen Wirt prototroph machen oder kann eine Resistenz gegenüber Bioziden, beispielsweise Antibiotika, oder Schwermetallen, wie Kupfer usw., verleihen. Der selektierbare Marker kann entweder direkt mit den zu exprimierenden DNA-Sequenzen verknüpft sein oder er wird durch Ko-Transfektion in dieselbe Zelle eingeführt. Zusätzliche Elemente werden eventuell für eine optimale Synthese einer Einzelstrang-mRNA benötigt. Diese Elemente sind beispielsweise Splice-Signale sowie Promotoren, Verstärker und Terminationssignale der Transkription. cDNA-Expressionsvektoren, die solche Elemente enthalten, sind beispielsweise die von Okayama und Berg (1983) beschriebenen.

Vorteilhafterweise ist die eingeführte Sequenz in einem Plasmid- oder Virus-Vektor eingebaut, der zur autonomen Replikation in der Empfängerwirtszelle fähig ist. Eine Vielfalt von Vektoren ist für diesen Zweck

13

geeignet. Wichtige Faktoren bei der Auswahl eines bestimmten Plasmid- oder Virus-Vektors sind beispielsweise: die Einfachheit, mit der Empfängerzellen, die den Vektor enthalten, erkannt und von den Empfängerzellen, die den Vektor nicht enthalten, unterschieden werden können; die Anzahl von Vektorkopien, die in einer bestimmten Wirtszelle gewünscht werden; und ob es wünschenswert ist, den Vektor zwischen Wirtszellen verschiedener Spezies hin und her benutzen (shuttle) zu können. Bevorzugte prokaryotische Vektoren sind beispielsweise Plasmide, wie diejenigen, die zur Replikation in *E. coli* fähig sind (beispielsweise pBR322, ColE1, pSC101, pACYC 184, πVX). Derartige Plasmide sind beispielsweise in Maniatis et al. (1982) veröffentlicht. *Bacillus*-Plasmide sind beispielsweise pC194, pC221, pT127 usw.. Derartige Plasmide sind in Gryczan (1982) veröffentlicht. Geeignete *Streptomyces*-Plasmide sind beispielsweise pIJ101 (Kendall und Cohen, 1987) und *Streptomyces*-Bakteriophagen wie φ2C31 (Chater et al., 1986). Übersichtsartikel über *Pseudomonas*-Plasmide erschienen von John und Twitty (1986) und Izaki (1978).

Bevorzugte eukaryotische Plasmide sind beispielsweise BPV, Vaccinia, SV40, 2-μ-Zirkel und andere sowie deren Derivate. Derartige Plasmide sind dem Fachmann wohlbekannt (Botstein et al., 1982; Broach, 1981 und 1982; Bollon und Stauver, 1980; Maniatis, 1980).

Wenn der Vektor oder die DNA-Sequenz, der/die das Konstrukt/die Konstrukte enthält, für die Expression vorbereitet ist, kann das DNA-Konstrukt/können die DNA-Konstrukte auf eine Vielzahl von Wegen in die geeigneten Wirtszellen eingeführt werden: Transformation, Transfektion, Konjugation, Protoplasten-Fusion, Elektroporation, Calciumphosphat-Präzipitation, direkte Mikroinjektion, etc.. Nach der Einführung des Vektors werden die Empfängerzellen in einem selektiven Medium, das das Wachstum der Vektorenthaltenden Zellen begünstigt, kultiviert. Die Expression der klonierten Gensequenz/-en zeigt sich in der Produktion des Toxins oder in der Produktion eines Fragmentes dieses Toxins. Dieses kann in den transformierten Zellen selbst stattfinden oder der Induktion dieser Zellen zur Differenzierung (beispielsweise durch die Verabreichung von Bromdesoxyuracil an Neuroblastoma-Zellen oder dergleichen) folgen.

Das exprimierte Protein wird gemäss herkömmlicher Verfahren, wie Extraktion, Präzipitation, Chromatographie, Affinitätschromatographie, Elektrophorese und dergleichen, isoliert und gereinigt.

V. Verwendung der selektiv auf Insekten wirkenden Toxine, um Pflanzen genetisch zu verändern

Die erfindungsgemässen Gene der selektiv auf Insekten wirkenden Toxine können in eine Pflanze eingeführt und dort zur Expression gebracht werden. Die Folge sind Toxine in der Pflanzenzelle, was zur Resistenz bzw. Toleranz der Pflanze gegenüber Insekten führt und als Mittel zur Bekämpfung von phytopathogenen Insekten genutzt werden kann. Auf diese Weise ist es möglich, Pflanzen herzustellen, die gegenüber Insekten resistenter bzw. toleranter sind. So ist ein weiterer Gegenstand der vorliegenden Erfindung die Benutzung des Toxin-Gens zur Transformation von Pflanzen, um die Resistenz bzw. Toleranz der Pflanzen gegenüber Insekten zu erhöhen.

Die kodierende Region des erfindungsgemässen Toxin-Gens kann sowohl die gesamte Länge als auch eine teilweise aktive Länge des Gens sein. Es ist jedoch erforderlich, dass die Gen-Sequenz, die das Toxin kodiert, das Toxin als funktionelles Toxin in der resultierenden Pflanzenzelle exprimiert und produziert.

Sowohl von DNA als auch von cDNA abstammende DNA sowie synthetische DNA können erfindungsgemäss verwendet werden, wenn sie ein erfindungsgemässes Toxin-Gen kodieren. Desweiteren kann ein solches Gen teilweise aus einem cDNA-Klon, teilweise aus einem genomischen Klon und teilweise aus einem synthetischen Gen sowie verschiedenen Kombinationen davon konstruiert sein. Ausserdem kann die DNA, die das Toxin-Gen kodiert, Gensequenzen aus verschiedenen Spezies enthalten.

Es gibt eine Vielzahl von Gegenständen, die das vorliegende Erfindungskonzept einschliesst. So betrifft ein weiterer Gegenstand dieser Erfindung, das selektiv auf Insekten wirkende Neurotoxin mit einem anderen Stoff oder anderen Stoffen zu kombinieren, um unerwartete, beispielsweise synergistische Eigenschaften zu Tage treten zu lassen. Diese anderen Stoffe sind beispielsweise Proteinase-Inhibitoren, die für Insekten toxisch sind, wenn sie oral aufgenommen werden. Andere Stoffe, die in Verbindung mit dem selektiv auf Insekten wirkenden Toxin verwendet werden können, um eine Pflanze genetisch zu verändern, um ihr eine Toleranz gegenüber Insekten zu verleihen, sind beispielsweise Polypeptide von *Bacillus thuringiensis*. Das *B. thuringiensis*-Protein verursacht Veränderungen in der Kalium-Permeabilität der Zellmembranen im Darm von Insekten (Sacchi et al., 1986) und es wird postuliert, dass es kleine Poren in der Membran verursacht (Knowles und Ellar, 1987). Andere Poren-erzeugenden Proteine können ebenfalls in Kombination mit den Toxinen verwendet werden. Beispiele derartiger Poren-erzeugender Proteine sind die Magainine (Zasloff, 1987), die Cecropine (Hultmark et al., 1982), die Attacine (Hultmark et al., 1983), Melittin, Gramicidin S (Katsu et al., 1988), Proteine des Natrium-Kanals und synthetische Fragmente (Oiki et al.,

14

1988), das α-Toxin von *Staphylococcus aureus* (Tobkes et al., 1985), Apolipoproteine und deren Fragmente (Knott et al., 1985; Nakagawa et al., 1985), Alamethicin und eine Vielzahl synthetischer amphipathischer Peptide (siehe den Übersichtsartikel von Kaiser und Kezdy, 1987). Lektine (Lis und Sharon, 1986), die sich an Zellmembranen binden und die Endocytose erhöhen, bilden eine weitere Klasse von Proteinen, die in Kombination mit selektiv auf Insekten wirkenden Toxinen verwendet werden können, um Pflanzen zum Zwecke der Insekten-Resistenz genetisch zu verändern.

Ein weiterer Gegenstand der Erfindung umfasst daher ferner chimäre Gen-Sequenzen bestehend aus:

(a) einer ersten Gen-Sequenz, die ein Toxin kodiert, welches durch die Expression des Gens in einer vorgegebenen Pflanzenzelle funktionstüchtig im Hinblick auf das Toxin ist, und gegebenenfalls einer zweiten Gen-Sequenz, die ein Polypeptid kodiert, welches nach oraler Aufnahme toxisch für Insekten ist; und

(b) einer oder mehreren zusätzlichen Gen-Sequenz/en, die funktionell mit der einen oder anderen Seite der das Toxin kodierenden Region verknüpft ist/sind. Diese zusätzlichen Gen-Sequenzen sind beispielsweise Promotor- und Terminatorsequenzen. Die Regulationssequenzen können heterolog oder homolog zur Wirtszelle sein.

Bei einem bevorzugten Gegenstand wird ein Promotor des Toxin-Gens benutzt, um die chimäre Gen-Sequenz zu exprimieren. Andere Promotoren, die erfindungsgemäss verwendet werden können, sind beispielsweise der *nos*- und der *ocs*-Promotor und CaMV-Promotoren. Wirkungsvolle pflanzliche Promotoren sind überproduzierende Promotoren. Die Promotoren sind in funktionstüchtiger Verknüpfung mit der kodierenden Sequenz fähig, die Expression des besagten Toxins zu fördern, so dass die transformierte Pflanze eine erhöhte Toleranz gegenüber Insektenschädlingen aufweist. Beispiele verwendbarer überproduzierender pflanzlicher Promotoren sind der Promotor des Gens, das die kleine Untereinheit (ss) der Ribulose-1,5-bisphosphat-Carboxylase der Sojabohne (Berry-Lowe et al., 1982) kodiert, und der Promotor des Gens, das das Chlorophyll a/b-Bindungsprotein kodiert. Von beiden ist bekannt, dass sie in Pflanzenzellen Lichtinduzierbar sind (siehe beispielsweise Cashmore, 1983; Corruzi et al., 1983; und Dunsmuir et al., 1983).

Ein weiterer bevorzugter Gegenstand vorliegender Erfindung ist die Expression der chimären Gensequenz in Pflanzen, bei der das Toxin-Gen funktionsfähig im korrekten Leseraster mit einem pflanzlichen Promotor und mit einer Sekretionssignalsequenz verknüpft ist.

Die chimäre Gen-Sequenz, die das funktionstüchtig mit einem pflanzlichen Promotor verknüpfte Toxin-Gen enthält, und vorteilhafterweise mit Sekretionssignalsequenzen versehen ist, kann mit einem geeigneten Klonierungsvektor verknüpft sein. Es lassen sich Plasmid- oder Virus- (Bakteriophagen)Vektoren verwenden, die Replikations- und Kontrollsequenzen enthalten, die aus Spezies stammen, die mit der Wirtszelle verträglich sind. Der Klonierungsvektor trägt typischerweise einen Startpunkt für die Replikation und spezifische Gene, die fähig sind, in transformierten Wirtszellen für phänotypische Selektionsmarker zu sorgen, üblicherweise Resistenz gegenüber Antibiotika. Transformierende Vektoren können nach der Transformation einer Wirtszelle an Hand dieser phänotypischen Marker herausgefunden werden.

Wirtszellen, die erfindungsgemäss verwendet werden können, sind beispielsweise Prokaryoten, einschliesslich bakterielle Wirte wie *E. coli, S. typhimurium* und *Serratia marcescens*. Eukaryotische Wirtszellen wie Hefen oder Fadenpilze können ebenfalls in dieser Erfindung verwendet werden.

Der Klonierungsvektor und die mit dem Vektor transformierte Wirtszelle werden erfindungsgemäss üblicherweise dazu verwendet, um die Kopienzahl des Vektors zu erhöhen. Als Folge einer erhöhten Kopienzahl können besagte Vektoren isoliert werden und beispielsweise dazu benutzt werden, die chimären Gen-Sequenzen in Pflanzen- oder andere Wirtszellen einzuführen.

Pflanzengewebe wird mit einem der oben beschriebenen Vektoren durch ein etabliertes Verfahren transformiert. Derartige, zum Transfer von DNA in Pflanzenzellen verwendete Verfahren sind der Fachwelt bekannt. Beispiele hierfür sind die direkte Infektion oder die Kokultivierung von Pflanzen, Pflanzengewebe oder -zellen mit *A. tumefaciens* (Horsch et al., 1985; Marton, 1984), der direkte Gentransfer exogener DNA in Protoplasten (Paszkowski et al., 1984, EP 129,668, EP 164,575, Shillito et al., 1985, Potrykus et al., 1985, Lörz et al., 1985, Fromm et al., 1985 und 1986, GB 2,140,822 und Negrutiu et al., 1987), die Inkubation in Gegenwart von PEG (Negrutiu et al., 1987), die Mikroinjektion (Reich et al., 1986a und 1986b) sowie der Beschuss mit Mikroprojektilen (Klein et al., 1987).

Ein erfindungsgemässes Verfahren, das Toxin-Gen in Pflanzenzellen einzuführen, ist, eine Pflanzenzelle mit *Agrobacterium tumefaciens*, das mit dem Toxin-Gen transformiert wurde, zu infizieren. Unter geeigneten, dem Fachmann bekannten Bedingungen werden die transformierten Pflanzenzellen kultiviert, damit sie Sprosse und Wurzeln bilden und sich letztlich zu transformierten, vermehrungsfähigen Pflanzen entwickeln. Die Gen-Sequenzen für das Toxin können in geeignete Pflanzenzellen beispielsweise mittels des Ti-Plasmids von *A. tumefaciens* eingeführt werden. Das Ti-Plasmid wird bei der Infektion mit *A. tumefaciens* in Pflanzenzellen übertragen und stabil in das pflanzliche Genom integriert (Horsch et al., 1984; Fraley et al., 1983).

Ti-Plasmide enthalten zwei für die Produktion transformierter Zellen wichtige Regionen. Die eine von ihnen, Transfer-DNA (T-DNA) genannt, induziert die Tumor-Bildung. Die andere, virulente Region genannt, ist wichtig für die Bildung, aber nicht die Aufrechterhaltung von Tumoren. Die T-DNA-Region, die in das pflanzliche Genom überträgt, kann durch die Einfügung der Gen-Sequenz eines Enzyms vergrössert werden, ohne ihre Übertragungsfähigkeit zu beeinflussen. Durch die Entfernung der Tumor-verursachenden Gene, so dass sie nicht länger stören können, kann das veränderte Ti-Plasmid anschliessend als Vektor für den Transfer der erfindungsgemässen Gen-Konstrukte in eine geeignete Pflanzenzelle benutzt werden.

Alle Pflanzenzellen, die mit *Agrobacterium* transformiert, und alle ausgewachsenen Pflanzen, die aus den transformierten Zellen regeneriert werden können, können ebenfalls erfindungsgemäss transformiert werden, um so transformierte ausgewachsene Pflanzen zu produzieren, die das transferierte Toxin-Gen enthalten.

Momentan gibt es insbesondere folgende zwei Wege, um Pflanzenzellen mit *Agrobacterium* zu transformieren:

(1) Kokultivierung von *Agrobacterium* mit kultivierten isolierten Protoplasten; oder

(2) Transformation von Zellen oder Geweben mit *Agrobacterium*.

Verfahren (1) erfordert ein etabliertes Kultivierungssystem, das die Kultivierung von Protoplasten und die Regenerierung von Pflanzen aus kultivierten Protoplasten erlaubt.

Verfahren (2) erfordert, dass (a) die Pflanzenzellen oder Gewebe durch *Agrobacterium* transformiert werden können und (b) in den transformierten Zellen oder Geweben die Regenerierung in ausgewachsene Pflanzen induziert werden kann. Um im binären System eine Infektion zu erzielen, werden zwei Plasmide benötigt: ein T-DNA-enthaltendes Plasmid und ein *vir*-Plasmid.

Ein weiterer erfindungsgemässer Gegenstand ist, das Toxin-Gen durch Elektroporation in die Pflanzenzellen einzuführen (Fromm et al., 1985). Bei dieser Technik werden Pflanzen-Protoplasten in Gegenwart von Plasmiden, die das Toxin-Genkonstrukt enthalten, elektroporiert. Elektrische Impulse hoher Feldstärke permeabilisieren reversibel Biomembranen und erlauben somit die Einführung der Plasmide. Elektroporierte Pflanzen-Protoplasten bilden neue Zellwände, teilen sich und bilden pflanzlichen Kallus. Die Selektion der transformierten Pflanzenzellen, die das Toxin exprimieren, kann mit den phänotypischen Markern erfolgen, wie es oben beschrieben ist.

Die exogene DNA kann in jeder Form zu den Protoplasten hinzugefügt werden, beispielsweise als nackte lineare, zirkuläre oder superspiralisierte DNA, DNA, eingeschlossen in Liposomen, DNA in Sphäroplasten, DNA in anderen Pflanzenprotoplasten, DNA komplexiert mit Salzen, und dergleichen.

Das genetische Material kann auch in die Pflanzenzelle transferiert werden, indem PEG verwendet wird, das einen Präzipitationskomplex mit dem genetischen Material bildet, der von der Zelle aufgenommen wird (Paszkowski et al., 1984).

DNA kann auch in Pflanzenzellen transferiert werden, indem sie direkt in isolierte Protoplasten, kultivierte Zellen und Gewebe (Reich et al., 1986 a und b) sowie in meristematische Gewebe von Keimlingen und Pflanzen (de la Peña et al., 1987, Graves und Goldman, 1986, Hooykaas-Van Slogteren et al., 1984 und Grimsley et al., 1987 und 1988) mikroinjiziert wird. Transgene Pflanzen und deren Nachkommenschaft werden durch der Fachwelt bekannte Verfahren erhalten.

Ein anderes Verfahren, fremde DNA-Sequenzen in Pflanzenzellen einzuführen, umfasst das Anheften besagter DNA an Partikel, die anschliessend den Pflanzenzellen mit Hilfe eines Apparates, wie er von Klein et al. (1988) beschrieben wird, eingeschossen werden. Jedes pflanzliche Gewebe oder pflanzliche Organ kann als Ziel dieser Prozedur verwendet werden, einschliesslich, aber nicht darauf beschränkt, Embryonen, apikale und andere Meristeme, Knospen, somatische und sexuelle Gewebe in vivo und in vitro. Transgene Zellen und Kallus werden gemäss etablierter Verfahren ausgewählt. In getroffenen Geweben wird die Bildung somatischer Embryonen und die Regenerierung von Sprossen induziert, um transgene Pflanzen gemäss etablierter Verfahren zu erhalten. Die geeigneten Verfahren können entsprechend der verwendeten Pflanzenart gewählt werden.

Die regenerierte Pflanze kann hinsichtlich der eingebauten fremden DNA chimär sein. Wenn die Zellen, die die fremde DNA enthalten, sich entweder in Mikro- oder Makrosporen entwickeln, wird die integrierte fremde DNA der sexuellen Nachkommenschaft vererbt werden. Wenn die Zellen, die die fremde DNA enthalten, somatische Zellen der Pflanze sind, werden nicht-chimäre transgene Pflanzen durch konventionelle Verfahren der vegetativen Vermehrung entweder in vivo, d.h. aus Knospen oder Stengelabschnitten, oder in vitro gemäss etablierter der Fachwelt bekannter Verfahren hergestellt. Solche Verfahren können entsprechend der verwendeten Pflanzenart gewählt werden.

Nach der Transformation der Pflanzenzelle oder der Pflanze können diejenigen Pflanzenzellen oder Pflanzen, die so transformiert wurden, dass das Enzym exprimiert wird, durch einen geeigneten phänotypischen Marker selektioniert werden. Diese phänotypischen Marker sind beispielsweise Antibiotika-Resistenz. Es gibt andere phänotypische Marker, die der Fachwelt bekannt sind und ebenfalls erfindungsgemäss benutzt werden können.

Auf Grund der Vielzahl an unterschiedlichen Transformationsverfahren können im Prinzip alle Pflanzentypen so transformiert werden, dass sie ein erfindungsgemässes Toxin exprimieren und dadurch resistent gegen Insekten werden. Bevorzugt sind im vorliegenden Fall jedoch Verfahren, die auf der Transformation mit *Agrobacterium*, insbesondere aber auf direktem Gentransfer beruhen. Beim direkten Gentransfer stehen die Verfahren im Vordergrund, bei denen Protoplasten transformiert werden.

Alle Pflanzen, die regenerierbare Protoplasten liefern, können erfindungsgemäss transformiert werden, so dass ausgewachsene transgene Pflanzen gewonnen werden können, die das transferierte Toxin-Gen enthalten und gegen Insekten tolerant bzw. resistent sind. Geeignete Pflanzen sind beispielsweise Vertreter von Arten der folgenden Gattungen:

*Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solanum, Petunia, Dactylis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Zea, Triticum, Sorghum* und *Datura*.

Es gibt immer mehr Anhaltspunkte dafür, dass praktisch alle Pflanzen aus kultivierten Zellen oder Geweben regeneriert werden können, einschliesslich aller Hauptgetreidearten, Zuckerrohr, Zuckerrübe, Baumwolle, Obst- und anderer Bäume, Hülsenfrüchte und Gemüse. Bisher ist wenig bekannt, ob alle diese Pflanzen mit *Agrobacterium* transformiert werden können. Arten, die natürliche pflanzliche Wirte von *Agrobacterium* sind, könnten in vitro transformierbar sein. Einkeimblättrige Pflanzen, insbesondere Getreide und Gräser, sind keine natürlichen Wirte von *Agrobacterium*. Bis kürzlich sind Versuche, sie mit *Agrobacterium* zu transformierten, erfolglos geblieben (Hooykaas-Van Slogteren et al., 1984), doch vermehren sich die Anzeichen, dass auch gewisse Monokotyledonen durch *Agrobacterium* transformiert werden können. Unter Verwendung neuartiger experimenteller Vorgehensweisen, die nun verfügbar sind, können auch Getreide- und Grasarten transformiert werden.

Beispiele von Pflanzengattungen, die durch *Agrobacterium* transformierbar sind, schliessen *Ipomoea, Passiflora, Cyclamen, Malus, Prunus, Rosa, Rubus, Populus, Santalum, Allium, Lilium, Narcissus, Ananas, Arachis, Phaseolus* und *Pisum* ein.

Die Regenerierung von Pflanzen aus kultivierten Protoplasten wird beispielsweise in Evans und Bravo (1983), Davey (1983), Dale (1983) und Binding (1985) beschrieben.

Die Regenerierungsverfahren variieren von Pflanzenart zu Pflanzenart. Im allgemeinen wird jedoch zuerst eine Suspension transformierter Protoplasten, die mehrfache Kopien des Toxin-Gens enthalten, hergestellt. Anschliessend wird in den Protoplasten-Suspensionen die Bildung von Embryonen bis zum Stadium der Reife und der Keimung als natürliche Embryonen induziert. Die Kulturmedium enthalten gewöhnlicherweise verschiedene Aminosäuren und Hormone, wie Auxine und Cytokinine. Vorteilhafterweise werden Glutaminsäure und Prolin dem Medium zugefügt, speziell bei Arten wie Mais und Alfalfa. Sprosse und Wurzeln entwickeln sich normalerweise gleichzeitig. Die wirkungsvolle Regenerierung hängt im allgemeinen vom Medium, dem Genotyp und der Vorgeschichte der Kultur ab. Wenn diese drei Variablen kontrolliert werden, sind solche Regenerierungen reproduzier- und wiederholbar.

Die reifen transgenen Pflanzen, die aus den transformierten Pflanzenzellen gezogen wurden, werden selbstbefruchtet, um Inzuchtpflanzen zu produzieren. Die Inzuchtpflanzen produzieren Samen, der das Gen für das Toxin enthält. Diese Samen können ausgesät werden, um transgene Pflanzen zu produzieren, die das Toxin enthalten.

Die erfindungsgemässen Inzuchtpflanzen können beispielsweise benutzt werden, um gegen Insekten tolerante Hybride zu produzieren. In diesem Verfahren wird eine gegen Insekten tolerante Inzuchtlinie mit einer anderen Inzuchtlinie gekreuzt und so das Hybrid erzeugt.

Aus den regenerierten Pflanzen erhaltene Teile, wie Blumen, Saatgut, Blätter, Zweige, Früchte und dergleichen sind Bestandteil dieser Erfindung, vorausgesetzt, dass diese Teile die gegen Insekten toleranten Zellen enthalten. Die Nachkommenschaft sowie Varianten und Mutanten der regenerierten Pflanzen mit besagter Eigenschaft sind ebenfalls Bestandteil dieser Erfindung.

Bei diploiden Pflanzen wird typischerweise ein Elter mit der Gen-Sequenz für das Toxin transformiert und der andere Elter ist ein Wildtyp. Nach Kreuzung der Eltern haben die Hybride der ersten Generation ($F_1$) eine Verteilung von 1/2 Toxin/Wildtyp : 1/2 Toxin/-Wildtyp. Diese Hybride der ersten Generation ($F_1$) werden selbstbefruchtet, um Hybride der zweiten Generation ($F_2$) zu produzieren. Die genetische Verteilung der $F_2$-Hybride ist 1/4 Toxin/Toxin : 1/2 Toxin/Wildtyp : 1/4 Wildtyp/Wildtyp. Die $F_2$-Hybride mit der

genetischen Ausstattung Toxin/Toxin werden als die gegen Insekten toleranten Pflanzen ausgewählt.

Der Begriff "Variante" beschreibt hier und im folgenden phänotypische Änderungen, die stabil und vererbbar sind, einschliesslich vererbbarer Variation, die sexuell auf die Nachkommen von Pflanzen übertragen wird, vorausgesetzt, die Variante ist immer noch eine gegen Insekten tolerante Pflanze. Ausserdem beschreibt der Begriff Mutante hier und im folgenden eine Variation als Ergebnis von Umweltbedingungen, wie Strahlung, oder als Ergebnis genetischer Variation, in der eine Eigenschaft meiotisch, den gut bekannten Vererbungsgesetzen folgend, vermittelt wird. Die mutierte Pflanze muss jedoch immer noch die erfindungsgemässe Toleranz gegenüber Insekten aufweisen.

## VI. Verwendung der selektiv auf Insekten wirkenden Toxine, um insektizide Mikroben zu verbessern

Das selektiv auf Insekten wirkende Toxin allein oder in Kombination mit irgendeinem der weiter oben erwähnten Stoffe kann benutzt werden, um die Toxizität insektizider Mikroben zur erhöhen. Verschiedene Baculoviren, einschliesslich derer, die *Heliothis virescens* (Baumwolleule), *Orgyia pseudotsugata* - (Trägspinner der Douglasie), *Lymantia dispar* (Grosser Schwammspinner), *Autographica californica, Neodiprion sertifer* (Europäische Fichtenfliege) und *Laspeyresia pomonella* (Apfelwickler) infizieren, sind bereits als Pestizide registriert und eingesetzt worden. Die Einführung eines selektiv aus auf Insekten wirkenden Toxins in ihr Genom könnte ihre pestizide Wirkung verstärken. Verfahren, fremde Gene in das Genom von Baculoviren einzuführen, sind in US 4,745,051 und EP 175,852 offenbart. EP 225,777 beschreibt die Produktion eines mikrobiellen Insektizids, das gegen zwei Insektenarten wirksam ist und auf der Konstruktion eines rekombinanten Baculovirus, der DNA-Segmente zweier Arten von Nuclear Polyhedrosis (Baculovirus) Virus enthält, basiert. Verschiedene Pilze sind ebenfalls fähig, Insekten zu infizieren. Die Einführung des selektiv gegen Insekten wirkenden erfindungsgemässen Toxins in das Genom derartiger Pilze kann deren insektizide Wirkung verstärken. *Beau varia bassania* und *B. brongniartii* haben einen weiten Wirtsbereich und sind als Kandidaten für mikrobielle Pestizide vorgeschlagen worden (siehe den Übersichtsartikel von Miller et al., 1983). Unabhängig von *B. thuringiensis* sind andere Bakterien als Agentien zur Bekämpfung von Insekten in Betracht gezogen worden, einschliesslich *B. popilliae, B. lentimorbus* und *B. sphaericus*. Ihr Potential als Insektizide kann dadurch verstärkt werden, dass ihre Wirkung durch den Einbau eines erfindungsgemässen Gens für ein selektiv auf Insekten wirkendes Toxin in ihr Genom erhöht wird.

## VII. Applikation der Toxine als Insektizide auf Pflanzen

Die Verleihung einer insektizid wirksamen Menge eines oder mehrerer Toxine kann durch externe Verabreichung erfolgen. Diese Verabreichung des Toxins an die Pflanzen oder Pflanzenteile kann entweder direkt oder in die Umgebung der Pflanzen oder Pflanzenteile erfolgen.

Das natürliche Toxin und/oder sein rekombinantes Äquivalent kann auf vielerlei Arten formuliert und verabreicht werden, beispielsweise in Pulver- oder Kristallform, als Suspension, als emulgierte Suspension, als Staub, Pellet, in Körnerform, in Kapseln, in Mikrokapseln, als Aerosol, als Lösung, Gel oder in anderen Dispersionen. Diese Erfindung betrifft deshalb auch ein Mittel, das ein oder mehrere der besagten Toxine zusammen mit Zusatzstoffen enthält, zur Verabreichung an Pflanzen.

Die erfindungsgemässen Mittel werden allgemein an Pflanzen oder Pflanzenteile in üblichen landwirtschaftlichen Formulierungen verabreicht, die einen oder mehrere landwirtschaftliche Trägermaterialien enthalten. Brauchbare landwirtschaftliche Trägermaterialien sind Stoffe, die dazu verwendet werden, eine Aktivsubstanz in einem Mittel zu lösen, zu verteilen oder zu verdünnen, ohne die biologische Wirkung der Substanz zu beeinträchtigen. Ein derartiger Trägerstoff hat selbst keinen schädlichen Einfluss auf den Boden, die Ausrüstung, die Ernteerzeugnisse oder das agronomische Umfeld. Das erfindungsgemässe Mittel kann entweder eine feste oder flüssige Formulierung oder eine Lösung sein. Der Stoff kann als benetzbares Pulver oder als ein emulgierfähiges Konzentrat formuliert sein.

Zusätzliche Stoffe können als Hilfsmittel eingeschlossen sein, das wären Benetzungs-, Spreitungs-, Verteilungs- oder Bindemittel gemäss gebräuchlicher landwirtschaftlicher Praktiken.

Eine weitere Gruppe von Hilfsmitteln können Verstärker der Toxinaktivität sein. Derartige Verstärker können Lektine, amphipathische Peptide, amphipathische Proteine oder Protease-Inhibitoren sein.

Formulierungshilfstoffe für Insektizide sind dem Fachmann hinlänglich bekannt.

Zusammengefasst betrifft die vorliegende Erfindung somit unter anderen folgende bevorzugte Ausführungsformen:

Ein selektiv gegen Insekten wirkendes Toxin mit nachfolgender Aminosäuresequenz:
VRDAYIAKNY NCVYECFRDA YCNELCTKNG ASSGYCQWAG KYGNACWCYA LPDNVPIRVP GKCR.

Eine rekombinante DNA enthaltend eine DNA-Sequenz erhältlich aus Tieren, wobei besagte DNA-Sequenz ein selektiv gegen Insekten wirkendes Toxin oder ein funktionsfähiges Derivat oder Bruchstück davon kodiert.

Eine rekombinante DNA enthaltend eine DNA-Sequenz, wobei besagte DNA-Sequenz erhältlich ist aus Tieren, vorteilhafterweise aus Arthropoden, vor allem aus Vertretern der Klassen *Arachnida* (Spinnentiere) oder *Chilopoda* (Hundertfüssler), insbesondere aus Vertretern der Ordnung *Scorpiones* sowie der Gattung *Scolopendra*, und ein selektiv gegen Insekten wirkendes Toxin kodiert oder ein funktionsfähiges Derivat oder Bruchstück davon, wobei besagte DNA-Sequenz in einer in Pflanzen exprimierbaren Form vorliegt.

Eine rekombinante DNA enthaltend eine DNA-Sequenz, wobei besagte DNA-Sequenz ein selektiv gegen Insekten wirkendes Toxin mit der Aminosäuresequenz:
KKNGYAVDSS GKAPECLLSN YCNNQCTKVH YADKGYCCLL SCYCFGLNDD KKVLEISDTR KSYCDTTIIN,
DGYIRKRDGC KLSCLFGNEG CNKECKSYGG SYGYCWTWGL ACWCEGLPDE KTWKSETNTC G,
DGYIRKKDGC KVSC(V/I)IIGNEG CRKECVAHGG SFGYCWTWGL ACWCENLPDA VTWKSSTNTC G,
DGYIKRRDGC KVACLIGNEG CDKECKAYGG SYGYCWTWGL ACWCEGLPDD KTWKSETNTC G,
ALPLSGEYEP CVRPRKCKPG LVCNKQQICV DPK oder
VRDAYIAKNY NCVYECFRDA YCNELCTKNG ASSGYCQWAG KYGNACWCYA LPDNVPIRVP GKCR oder
ein funktionsfähiges Derivat oder Bruchstück davon kodiert.

Einen Vektor enthaltend eine DNA-Sequenz erhältlich aus Tieren, wobei besagte DNA-Sequenz ein selektiv gegen Insekten wirkendes Toxin oder ein funktionsfähiges Derivat oder Bruchstück davon kodiert und in einer Pflanzen exprimierbaren Form vorliegt.

Einen Wirtsorganismus enthaltend eine DNA-Sequenz erhältlich aus Tieren, wobei besagte DNA-Sequenz ein selektiv gegen Insekten wirkendes Toxin oder ein funktionsfähiges Derivat oder Bruchstück davon kodiert und in einer in Pflanzen exprimierbaren Form vorliegt.

Eine transgene Pflanzenzelle enthaltend eine DNA-Sequenz erhältlich aus Tieren, vorteilhafterweise aus Arthropoden, vor allem aus Vertretern der Klassen *Arachnida* (Spinnentiere) oder *Chilopoda* - (Hundertfüssler) insbesondere aus Vertretern der Ordnung *Scorpiones* sowie der Gattung *Scolopendra*, wobei besagte DNA-Sequenz ein selektiv gegen Insekten wirkendes Toxin oder ein funktionsfähiges Derivat oder Bruchstück davon kodiert.

Eine transgene Pflanzenzelle enthaltend eine DNA-Sequenz erhältlich aus Tieren, vorteilhafterweise aus Arthropoden, vor allem aus Vertretern der Klassen *Arachnida* (Spinnentiere) oder *Chilopoda* - (Hundertfüssler), insbesondere aus Vertretern der Ordnung *Scorpiones* sowie der Gattung *Scolopendra*, wobei besagte DNA-Sequenz ein selektiv gegen Insekten wirkendes Toxin oder ein funktionsfähiges Derivat oder Bruchstück davon kodiert und stabil in das pflanzliche Genom integriert ist.

Eine transgene Pflanzenzelle enthaltend eine DNA-Sequenz erhältlich aus Tieren, vorteilhafterweise aus Arthropoden, vor allem aus Vertretern der Klassen *Arachnida* (Spinnentiere) oder *Chilopoda* - (Hundertfüssler), insbesondere aus Vertretern der Ordnung *Scorpiones* sowie dr Gattung *Scolopendra*, wobei besagte DNA-Sequenz ein selektiv gegen Insekten wirkendes Toxin oder ein funktionsfähiges Derivat oder Bruchstück davon kodiert und in exprimierbarer Form vorliegt.

Eine transgene Pflanzenzelle, die das von besagter DNA-Sequenz kodierte selektiv gegen Insekten wirkende Toxin oder ein funktionsfähiges Derivat oder Bruchstück davon exprimiert.

Eine transgene Pflanze sowie deren sexuelles und asexuelles Vermehrungsgut enthaltend eine DNA-Sequenz erhältlich aus Tieren, vorteilhafterweise aus Arthropoden, vor allem aus Vertretern der Klassen *Arachnida* (Spinnentiere) oder *Chilopoda* (Hundertfüssler), insbesondere aus Vertretern der Ordnung *Scorpiones* sowie der Gattung *Scolopendra*, wobei besagte DNA-Sequenz ein selektiv gegen Insekten wirkendes Toxin oder ein funktionsfähiges Derivat oder Bruchstück davon kodiert.

Eine transgene Pflanze sowie deren sexuelles und asexuelles Vermehrungsgut enthaltend eine DNA-Sequenz erhältlich aus Tieren, wobei besagte DNA-Sequenz ein selektiv gegen Insekten wirkendes Toxin oder ein funktionsfähiges Derivat oder Bruchstück davon kodiert und stabil in das pflanzliche Genom integriert ist.

Eine transgene Pflanze sowie deren sexuelles und asexuelles Vermehrungsgut enthaltend eine DNA-Sequenz erhältlich aus Tieren, wobei besagte DNA-Sequenz ein selektiv gegen Insekten wirkendes Toxin oder ein funktionsfähiges Derivat oder Bruchstück davon kodiert und in exprimierbarer Form vorliegt.

Eine transgene Pflanze sowie deren sexuelles und asexuelles Vermehrungsgut, die das von besagter DNA-Sequenz kodierte Toxin oder ein funktionsfähiges Derivat oder Bruchstück davon exprimiert.

Einen transgenen Mikroorganismus enthaltend eine DNA-Sequenz erhältlich aus Tieren, vorteilhafterweise aus Arthropoden, vor allem aus Vertretern der Klassen *Arachnida* (Spinnentiere) oder *Chilopoda* -

(Hundertfüssler), insbesondere aus Vertretern der Ordnung *Scorpiones* sowie der Gattung *Scolopendra*, wobei besagte DNA-Sequenz ein selektiv gegen Insekten wirkendes Toxin oder ein funktionsfähiges Derivat oder Bruchstück davon kodiert.

Einen transgenen Mikroorganismus enthaltend eine DNA-Sequenz erhältlich aus Tieren, wobei besagte DNA-Sequenz ein selektiv gegen Insekten wirkendes Toxin oder ein funktionsfähiges Derivat oder Bruchstück davon kodiert und stabil in das Genom integriert ist.

Einen transgenen Mikroorganismus enthaltend eine DNA-Sequenz erhältlich aus Tieren, wobei besagte DNA-Sequenz ein selektiv gegen Insekten wirkendes Toxin oder ein funktionsfähiges Derivat oder Bruchstück davon kodiert und in exprimierbarer Form vorliegt.

Einen transgenen Mikroorganismus, der das von besagter DNA-Sequenz kodierte Toxin oder ein funktionsfähiges Derivat oder Bruchstück davon exprimiert.

Einen Antikörper gegen ein selektiv auf Insekten wirkendes Toxin oder ein funktionsfähiges Derivat oder Bruchstück davon, erhältlich aus Vertretern der Ordnung *Scorpiones* oder der Gattung *Scolopendra*.

Ein insektizides Mittel enthaltend als Wirksubstanz ein aus Tieren erhältliches, selektiv gegen Insekten wirkendes, vorzugsweise rekombinantes Toxin oder ein funktionsfähiges Derivat oder Bruchstück davon.

Ein Verfahren zur Bekämpfung von phytopathogenen Insekten, dadurch gekennzeichnet, dass man auf das Insekt oder dessen Lebensraum einen transgenen Mikroorganismus enthaltend eine DNA-Sequenz erhältlich aus Tieren, vorteilhafterweise aus Arthropoden, vor allem aus Vertretern der Klassen *Arachnida* - (Spinnentiere) oder *Chilopoda* (Hundertfüssler), insbesondere aus Vertretern der Ordnung *Scorpiones* sowie der Gattung *Scolopendra*, wobei besagte DNA-Sequenz ein selektiv gegen Insekten wirkendes Toxin oder ein funktionsfähiges Derivat oder Bruchstück davon kodiert, wobei besagte DNA-Sequenz in exprimierbarer Form vorliegt und stabil in das Genom integriert ist, oder ein Mittel enthaltend als Wirksubstanz ein aus Tieren erhältliches, selektiv gegen Insekten wirkendes, vorzugsweise rekombinantes Toxin oder ein funktionsfähiges Derivat oder Bruchstück davon, in einer insektizid wirksamen Menge appliziert.

Ein Verfahren zum Schutz von Kulturpflanzen vor Schäden durch phytopathogene Insekten, dadurch gekennzeichnet, dass man die Kulturpflanze mit einer rekombinanten DNA transformiert, wobei die rekombinante DNA eine DNA-Sequenz erhältlich aus Tieren enthält, wobei besagte DNA-Sequenz ein selektiv gegen Insekten wirkendes Toxin oder ein funktionsfähiges Derivat oder Bruchstück davon kodiert, und dass weiterhin das besagte gegen Insekten wirkende Toxin oder ein funktionsfähiges Derivat oder Bruchstück davon in einer insektizid wirksamen Menge in der Pflanze exprimiert wird.

Zum besseren Verständnis wird die allgemeine Beschreibung nachfolgend durch nicht-limitierende Beispiele ergänzt.


BEISPIELE


Beispiel 1: Reinigung, Primärstruktur und Wirkungsweise von LqhP35

Das Rohgift des Skorpions *L. quinquestriatus hebraeus* wird durch elektrisches Melken (Zlotkin und Gordon, 1985) im Feld gesammelter Skorpione und nachfolgende Lyophilisierung gewonnen. Das erregende, auf Insekten wirkende Toxin AaIT, das aus dem Gift des Skorpions *Androctonus australis* stammt, wird gemäss Zlotkin et al. (1971a) gereinigt. Das gegenüber Säugetieren wirksame $\alpha$-Toxin AaH2 wird von Prof. H. Rochat (Fac. Medicine, Biochimie, Marseille, Frankreich) bezogen.

Die *Sarcophaga falculata*-Larven (100 bis 130 mg KG) werden im Labor gemäss Zlotkin et al. (1971b) gezüchtet. *Hemilepistus* spec. (Asseln, terrestrische Crustaceen)(300 bis 400 mg KG) werden im Feld gesammelt und Albino-Labormäuse (Variante "Sabra") werden von der Labortierfarm der Hadassah Medical School, Jerusalem, gekauft.

Die Synaptosomen von Heuschrecken und die davon abstammenden Membranvesikel werden aus Homogenaten der zerschnittenen Zentralnervensysteme von *Locusta migratoria* gemäss Zlotkin und Gordon (1985) erhalten.

Die lethalen und lähmenden Wirkungen der toxischen Substanzen werden durch subkutane Injektionen bestimmt. Schmeissfliegen-larven werden in einem Test verwendet, bei dem sich eine neue Symptomatologie, ausgedrückt als eine verzögerte und anhaltende spastische Lähmung, zeigt, indem ihnen 2 bis 10 $\mu$l/100 mg KG in die Zwischensegmentmembran eines Abdominalsegments injiziert wird. Die Bestimmung der lähmenden Einheit (PU) basiert auf der Unbeweglichkeit, die von einer Kontraktion des Tieres begleitet ist, 5 Minuten nach der Injektion. Asseln, denen 1 bis 5 $\mu$l/300 mg KG zwischen Thorax und Abdomen injiziert wird, werden für die Bestimmung der PU, basierend auf einer kompletten Unbeweglichkeit 5 Minuten nach der Injektion, benutzt. Mäuse werden zur Bestimmung der $LD_{50}$ (Dosis, die für 50 % der Tiere lethal ist), die 24 Stunden nach der Injektion bestimmt wird, benutzt. Die Probennahme und die Bestimmung der 50 % Endpunkte (PU und $LD_{50}$-Dosen) basiert auf Reed und Muench (1938).

Drei verschiedene Säulenchromatographie-Verfahren werden benutzt:

(a) Molekularsiebsäulen mit Sephadex G50 Gel (Pharmacia, Schweden) mit dem flüchtigen Ammoniumacetat-Puffer: 1650 $A_{280}$-Einheiten L. quinquestriatus hebraeus-Gift (entsprechen 2 g Rohgift nach wässriger Extraktion und zweimaliger Sephadex G50 Säulenchromatographie) werden wiederholt auf eine Serie von vier Säulen [4x(100x3,2 cm)] aufgetragen, die mit 45 ml/Stunde 0,1 M Ammoniumacetat-Puffer pH 8,5 äquilibriert und eluiert werden (Zlotkin et al, 1971a). Die verschiedenen Fraktionen werden entsprechend dem Elutionsprofil gesammelt. Die Fraktion IV ($A_{280}$ = 200 Einheiten) führt zu Lähmung und Lethalität bei Schmeissfliegen, Asseln und Mäusen.

(b) Kationenaustauschchromatographie über Carboxymethylcellulose (CM52, Whatman, England) mit Konzentrationsgradienten-Elution mit Ammoniumacetat-Puffer: 23 mg (31,5 $A_{280}$-Einheiten Fraktion IV, a) werden auf eine 10 ml Säule aufgetragen, die mit dem Kationenaustauscher CM-Cellulose CM52 (Whatman, England) gefüllt und mit 10 ml/Stunde 0,01 M Ammoniumacetat-Puffer pH 6,4 äquilibriert ist. Der erste Elutionsschritt wird unter Gleichgewichtsbedingungen durchgeführt und führt zu Fraktionen (a) und (b), die bei Sarcophaga-Larven zur Lähmung führen. Der zweite Elutionsschritt wird mit einem linearen Konzentrationsgradienten von 0,1 bis 0,5 M durchgeführt und führt zu Fraktionen (c), (d) und (e), deren Toxizität gegenüber Fliegenlarven und Mäusen in Tabelle 1 abgelesen werden kann.

(c) HPLC Reverse Phase Chromatographie mit einer TSK-RP-CL8-Säule (LKB, Schweden): Puffer: A 0,1 % TFA, B 0,1 % TFA, Acetonitril: Isopropanol = 1:1. B-Gradient: 0 Zeit 5 %, 15 Minuten 20%, 75 Minuten 50 %. Durchflussrate 1 ml/Minute.

Platten-Gelelektrophorese wird in zwei Formen durchgeführt:

(a) SDS-PAGE in Gegenwart von Harnstoff (Swank und Munkres, 1971): Die Auftrennungen werden in einem kontinuierlichen Minigel (60x80x1,5 mm) durchgeführt, bei einer Polyacrylamid-Konzentration von 12,5 % und in Gegenwart von 8 M Harnstoff.

(b) Analytische isoelektrische Fokussierung in Polyacrylamid in Gegenwart von Ampholinen (LKB, Technical Bulletin 1217-2001ME).

Die Proteinmenge wird gemäss Lowry et al. (1951) mit RSA als Vergleich bestimmt.

Das LqhP35-Toxin wird alkyliert und mit 4-Vinylpyridin reduziert, indem Proben mit 6 M Guanidin-HCl, 1 M Tris-HCl pH 8,6, 10 mM EDTA und 20 mM Dithiothreitol eine Stunde lang bei 37°C inkubiert werden. 4-Vinylpyridin (Sigma, USA) wird bis zu einer Endkonzentration von 50 mM hinzugefügt und die Inkubation wird für eine Stunde bei Raumtemperatur fortgesetzt. Das modifizierte Protein wird durch HPLC über eine Vydac-C-8- oder Hypersil-ODS-Säule in 0,1 % Isopropanol : Acetonitril = 1:1 entsalzt. Peptide werden durch Abbau des reduzierten und alkylierten Proteins mit Asp-N, Lys-C und Trypsin (Boehringer Mannheim, USA) gemäss den vom Hersteller angegebenen Vorschriften erhalten. Peptide weden durch saure Teilhydrolyse erhalten. Die Peptide werden durch HPLC über eine Hypersil-ODS-Säule in 0,1 % TFA getrennt, mit einem Isopropanol : Acetonitril = 1: 1 -Gradienten von 0 bis 60 %. Die Aminosäurensequenzanalyse wird durch einen automatisierten Edman-Abbau mit einem Applied Biosystems 470A Gasphase Sequencer (USA) durchgeführt. Phenylthiohydantoin-Aminosäuren werden mit Hilfe des On-line Applied Biosystems 120APTH Analyzers durchgeführt. Jede Sequenz wird mit wenigstens zwei getrennten Bestimmungen bestätigt [Allen, 1981, Inglis, 1980 (Inglis, A. et al., in Birr, C. (Hrsg.) Methods in Peptide and Protein Sequence Analysis, Proc. Int. Conf. 3rd., Elsevier, Amsterdam, 329, 1980)]. Für Sequenzvergleiche sind die Aminosäurensequenzen zur Erreichung maximaler Homologie mit Hilfe der Profilanalyse der Genetics Computing Group der Universität von Wisconsin abgeglichen (Devereux et al., 1984). Der Anteil der Gesamtpositionen, die identische Reste enthalten, wird berechnet.

Gemäss Zlotkin und Gordon (1985) werden mit $^{125}$I markiertes AaIT hergestellt und Bindungsstudien zur kompetitiven Ersetzbarkeit durchgeführt.

Präparation eines Insektenaxons. Sogenannte voltage clamp and current clamp-Experimente werden mit Riesenaxonen durchgeführt, die aus den abdominalen Nervensträngen der Schabe Periplaneta americana geschnitten wurden (Pichon und Boistel, 1967). Die normale physiologische Kochsalzlösung hat die folgende Zusammensetzung: 200 mM NaCl, 3,1 mM CKl, 5,4 mM $CaCl_2$, 5,0 mM $MgCl_2$. Der pH wird mit einem Phosphat-Carbonat-Puffer (2 mM $NaHCO_3$, 0,1 mM $NaH_2PO_4$) auf 7,2 gehalten. Current clamp-Experimente werden bei 20 bis 22°C, voltage clamp-Experimente bei 12± 0,5°C durchgeführt.

4-AP (die Konzentrationen werden im Text angegeben) wird verwendet, um selektiv den Kaliumstrom zu blockieren (Pelhate und Pichon, 1974), und synthetisches STX ($2 \times 10^{-7}$ M) wird für die selektive, reversible Blockäge der Natriumströme verwendet (Sattelle et al., 1979). Die gereinigten Skorpiontoxine werden in Gegenwart von RSA (Fraktion V, Armour Co., USA) im Verhältnis 1 : 10 (w/v) lyophilisiert.

Präparation einer einzelnen Skelettmuskelfaser aus Säugetieren. Voltage clamp-Experimente und Current clamp-Experimente werden bei Raumtemperatur (18 bis 22° C) an einzelnen Muskelfasern durchgeführt, die aus dem sogenannten slow twitch-Soleus-Muskel der Ratte *Rattus norvegicus* isoliert wurde (Duval und Léoty, 1978). Die normale physiologische Kochsalzlösung hat die folgende Zusammensetzung: 140 mM NaCl, 6mM KCl, 3mM CaCl₂, 5,0 mM·Glucose. Der pH wird mit 6,5 mM Tris HCl auf 7,3 eingestellt. 10 mM TEA und 2mM 3,4-DAP werden dem Bad hinzugefügt, um den Kaliumstrom zu blockieren, und 1 µM TTX wird verwendet,um den Natriumstrom zu blockieren (Duval und Léoty, 1980).

B. Isolierung eines Faktors, der eine verzögerte und anhaltende Kontraktion von Schmeissfliegen-Larven induziert. 3,7 g lyophilisiertes Rohgift des Skorpions *L. q. hebraeus* wird folgendermassen erhalten: (1) wässrige Extraktion und Lyophilisation; (2) Auftrennung des lyophilisierten wässrigen Extraktes auf einer Sephadex G50-Säule mit 0,1 M Essigsäure, um hochmolekulare Mucoproteine zu entfernen; (3) Auftrennung der lyophilisierten toxischen Fraktionen, die durch die obige Sephadex-Essigsäure-Säule erhalten wurden, über eine Sephadex G50-Säule mit einem 0,1 M Ammoniumacetatpuffer pH 8,5 um niedermolekulare Pigmente, die nicht aus Protein bestehen, zu entfernen. Die lyophilisierten toxischen Fraktionen, die im obigen Schritt (3) erhalten wurden, werden durch wiederholte Passagen über eine Serie von vier Sephadex G50-Säulen unter den oben genannten Bedingungen getrennt. Dieses Vorgehen resultiert in der Auftrennung von vier Hauptfraktionen (I bis IV). Fraktion IV (entspricht etwa 12 % des geladenen Proteins) induziert nach der Injektion in Fliegenlarven eine recht ungewöhnliche Mischung von Symptomen einschliesslich Erschlaffung (typisch für die hemmenden auf Insekten wirkenden Toxine) und auch Kontraktivität (typisch für die erregenden auf Insekten wirkenden Toxine), die jedoch nach einer Verzögerung auftreten und eine verlängerte Dauer haben. Fraktion IV is auch moderat lethal für Mäuse ($LD_{50}$ 50µg/20 g KG). Diese Lethalität wird von erregenden Symptomen der Vergiftung begleitet, die typisch für die Gifte von *Buthinae* Skorpionen und für die von ihnen abgeleiteten, auf Säugetiere wirkenden Toxine sind (Rochat et al., 1979).

Die Auftrennung der obigen Fraktion IV über eine Kationenaustauscher (CM52) Säule ergibt eine Reihe von Fraktionen (a bis e). Die Fraktionen a und b induzieren die erschlaffende Lähmund von Schmeissfliegen-Larven, die typisch für hemmende auf Insekten wir kende Toxine ist (Zlotkin, 1986). Die Fraktionen, c, d und e zeigen jedoch eine Toxizität gegen Mäuse (Tabelle 1) und eine klare neue Symptomatologie gegen Schmeissfliegen-Larven, die sich im Vorkommen einer verzögerten und anhaltenden (langdauernden) Kontraktionslähmung ausdrückt. Wie in Tabelle 1 gezeigt, zeigen die Fraktionen c, d und e verschiedene Grade der obigen verzögert-anhaltenden Kontraktionslähmung und Lethalität für Mäuse. Fraktion d, die die höchste Toxizität gegen Schmeissfliegen und die niedrigste Toxizität gegen Mäuse hat, wird weiter gereinigt.

Tabelle 1

| Toxizität der Fraktionen c, d und e [CM52-Chromatographie] gegen Schmeissfliegen-Larven und Mäuse | | | |
|---|---|---|---|
| Test | Fraktion | | |
| | c | d | e |
| Schmeissfliegen-Larven $PU_{50}$[a] (µg/100 mg KG)<br>Maus-Lethalität $LD_{50}$[b] | 0,054<br>12,0 | 0,028<br>120,0 | 0,7<br>25,0 |

[a]Eine 5 Minuten nach der Injektion unbewegliche und kontrahierte Larve wird als positive Antwort angesehen.
[b]Die Lethalität wird nach 24 Stunden bestimmt. Die $LD_{50}$ der Fraktion IV für Mäuse entspricht 40 µg/20 g KG.

Die endgültige Reinigung des neuen, Schmeissfliegen-Larven beeinträchtigenden Faktors wird durch einen zusätzlichen Chromatographie-Schritt über eine Reverse Phase Säule in einem HPLC-System erreicht. Das endgültige Produkt wird als LqhP35-Toxin bezeichnet (Lqh bezeichnet den Skorpion, P die Lähmung und 35 entspricht der Auftrennungs-Zeit auf der HPLC-Säule). Das resultierende Produkt enthält etwa 30 % des Proteingehalts der CM52-Fraktion "d" und etwa 60 % von deren Aktivität gegen Schmeissfliegen-Larven. Seine Reinheit und Eigenschaften werden mit SDS-PAGE (zeigt ein MG von etwa 5 kD) und durch analytische isoelektrische Fokussierung (zeigt einen pI von etwa pH 9.0) überprüft.

C. Bestimmung der Primärstruktur des LqhP35-Toxins. LqhP35 ist ein einfachkettiges, aus 64 Aminosäuren bestehendes Protein mit einem MG von etwa 7 kD (MG = 7255), das typisch für verschiedene Toxine aus Skorpiongift ist (Possani, 1984). Die vorliegende MG-Bestimmung stimmt im wesentlichen mit der obigen SDS-PAGE überein, die nur un gefähre Daten mit einer erwarteten Ungenauigkeit von wenigstens 20 % ergibt (Swank und Munkres, 1971). Der hohe isoelektrische Punkt (pl), der durch analytische isoelektrische Fokussierung erhalten wird. stimmt im wesentlichen mit der Sequenzanalyse überein, die einen Überschuss von positiv geladenen Bestandteilen gegenüber den negativ geladenen, einschliesslich dem Vorkommen von drei Argininen (pK = 12,48) offenbart. Die hydrophoben Aminosäuren, die ein Drittel der Bestandteile ausmachen, sind gleichmässig über die Länge des Moleküls verteilt. Es wird angenommen, dass die acht Cysteine vier Disulphidbrücken bilden. Diese Annahme wird indirekt durch den pl-Wert (pH 9,0 bis 9,2) des LqhP35-Toxins angezeigt. Bei dem obigen pH-Wert würden die Sulfhydrylgruppen, wenn sie frei wären, negative Ladungen beitragen, die auf ihre Ionisierung zurückzuführen sind, und so den pl-Wert erniedrigen. Das Vorkommen eines Cystein-Arginins am C-Terminus wie beim LqhP35-Toxin ist bereits beim gegen Säugetiere wirkenden LqqIV α-Toxin aus dem Gift des verwandten Skorpions *L. q. quinquestriatus* gezeigt worden (Possani, 1984).

D. Biologische Aktivität des LqhP35-Toxins. Symptomatologie. Im Gegensatz zu den erregenden auf Insekten wirkenden Toxinen, die eine sofortige und vorübergehende Kontraktionslähmung von Schmeissfliegen-Larven bewirken, induziert das LqhP35-Toxin eine verzögerte und anhaltende Kontraktionslähmung.

Toxizität. Die lähmende und lethale Kraft des LqhP35-Toxins gegenüber Arthropoden und Mäusen ist in Tabelle 2 dargestellt.

Tabelle 2

| Toxische Aktivität des LqhP35-Toxins | | |
|---|---|---|
| Testtier | Wirkung | $ED_{50}$-Wert |
| Schmeissfliegen-Larve | verzögerte anhaltende Kontraktionslähmung (PU) | 14ng/100mg KG |
| Asseln | Lähmung innerhalb von 5 Minuten (PU) | 20ng/100mg KG |
| Maus | Lethalität nach 24 Stunden ($LD_{50}$) | 100μg/20g KG[a] |

[a]Etwa zwei Grössenordnungen weniger toxisch als die gewöhnlichen, auf Säugetiere wirkenden, aus Skorpiongift abgeleiteten Toxine (Rochat et al., 1979).

Bindungsversuche. 210 μl Reaktionsmischung enthält 1,5 nM $^{125}$I AaIT, 40 μg Protein in Form von synaptosomalen Membranvesikeln aus Heuschrecken (Zlotkin und Gordon, 1985) und zunehmende Konzentrationen der kompetierenden Substanz im Standard-Bindungsmedium [0,15 M Cholinchlorid, 1 mM $MgSO_4$, 2 mM $CaCl_2$, 0,1 % BSA (Zlotkin und Gordon, 1985)]. Die Membranen werden 40 Minuten bei 22°C inkubiert. Die Trennung zwischen dem freien und dem Membran-gebundenen $^{125}$I AaIT wird mit einem raschen Filterverfahren durchgeführt (Zlotkin und Gordon, 1985). Die Bindung des markierten Toxins, gemessen in Gegenwart eines grossen Überschusses an nicht-markiertem Toxin (1 μM) wird als unspezifische Bindung definiert.

Im Gegensatz zu den erregenden (Zlotkin et al., 1985, Gordon et al., 1984) und den hemmenden (LqqIT2, Zlotkin et al., 1985) Toxinen ist das LqhP35-Toxin unfähig, das $^{125}$I AaIT-Toxin in einem synaptosomalen Heuschrecken-Präparat zu verdrängen. Dies kann anzeigen, dass das LqhP35-Toxin bestimmte Bindungsstellen hat, die sich von denen der obigen erregenden und hemmenden, gegen Insekten wirksamen Toxine unterscheiden.

Elektrophysiologische Studien. Das LqhP35-Toxin wird unter current and voltage clamp-Bedingungen an zwei verschiedenen Präparaten einer erregbaren Membran untersucht: am isolierten Riesenaxon von *Periplaneta americana* und an der isolierten Skelettmuskelfaser der Ratte. Die Wirkung des LqhP35-Toxins auf die Aktionspotentiale des Schaben-Axons wird bestimmt, indem Aktionspotentiale in Anwesenheit des Toxins durch einen kurzen (0,5 msec) depolarisierenden Strom von 10 nA hervorgerufen werden. Die Wirkung des LqhP35-Toxins auf den $Na^+$-Strom des Schaben-Axons wird in einem sogenannten voltage clamp-Experiment in Gegenwart von $2 \times 10^{-4}$ M 3,4-DAP bestimmt. Die Wirkung des LqhP35-Toxins auf die Aktionspotentiale der isolierten Skelettmuskelfaser der Ratte wird bestimmt, indem Aktionspotentiale in normaler Ringer-Lösung und nach externer Verabreichung des Toxins verglichen werden. Die Wirkung von LqhP35 auf Ionenströme der isolierten Skelettmuskelfaser der Ratte wird bestimmt, indem das Membranpotential auf bestimmten Werten festgehalten wird (voltage clamp-Experiment)(schrittweise Depolarisierung von einem Ruhepotential von -90 mV bis -40 mV).

In beiden Präparaten induziert das Toxin im wesentlichen denselben Effekt, die Verlängerung des induzierten Aktionspotentials, die auf die offensichtliche Hemmung der Natrium-Inaktivierung zurückzuführen ist. Das Toxin beeinträchtigt oder verändert nicht (a) die Amplitude des Aktionspotentials, (b) die Höhe des Ruhepotentials der Membran und (c) die Kalium-Leitfähigkeit.

Das LqhP35-Toxin zeigt eine offensichtliche Vorliebe gegenüber der erregbaren Membran von Insekten, wenn seine Aktivität mit der des wirkungsvollenvollen, gegen Säugetiere wirksamen Toxins AaH2 verglichen wird, das mit denselben Präparaten untersucht wird. Unter den vorliegenden current clamp-Bedingungen wird die Verlängerung des Aktionspotentials durch eine LqhP35 Konzentration verursacht, die etwa zwei Grössenordnungen niedriger ist als die, die im Insektenaxon-Präparat für das AaH2-Toxin erforderlich ist (Pelhate und Zlotkin, 1981), und die wenigstens eine Grössenordnung höher ist als die für AaH2 in der Skelettmuskelmembran der Ratte. Im letzteren Fall ist die maximale Dauer des Aktionspotentials jedoch offensichtlich niedriger für das LqhP35-Toxin ($10^{-6}$ M, 2234 ± 584 msec, n = 7) als die für das AaH2-Toxin ($10^{-7}$ M, 700 ± 420 msec, n = 8).

Beispiel 2: Gewinnung des Hundertfüssler-Giftes

*S. canidens* werden in der Umgebung des Toten Meeres und Jerusalems gesammelt. Im Feld gesammelte Hundertfüssler werden separat in Laborbehältern gehalten, die ein Feuchtigkeit absorbierendes Substrat und eine Wasserversorgung enthalten. Die Hundertfüssler werden mit lebenden Insekten gefüttert (einmal alle zwei Wochen).

Das Gift wird den Hundertfüsslern entnommen, indem sie folgendermassen gemolken werden: die Basis der Gift"zähne" wird elektrisch stimuliert und das Gift wird in Kapillarröhrchen aus Plastik gesammelt, die fest und den Spitzen der Gift"zähne" befestigt wurden.

Tabelle 3 zeigt die Körperlänge der Hundertfüssler, das Volumen des pro Melkvorganges gewonnen Giftes und die Proteinkonzentration des isolierten Giftes.

Tabelle 3

| Volumen und Proteingehalt des Hundertfüssler-Giftes | | | |
|---|---|---|---|
| Hundertfüssler | Körperlänge | Giftvolumen pro Melkvorgang | Proteingehalt[a] |
| | (cm) | ($\mu$l) | ($\mu$g/$\mu$l) |
| *S. canidens* (Totes Meer) | 6 bis 8 | 0,29,0,2 bis 0,4 (5) | 210 |
| *S. canidens* (Jerusalem) | 11 bis 14 | 4,33,3,7 bis 50 (3) | 190 |

[a]gemäss Lowry et al. (1951)

Beispiel 3: Stabilität des *S. canidens* Giftes

Das Hundertfüsslergift von *S. canidens* aus der Umgebung des Toten Meeres wird wie oben beschrieben isoliert und auf seine Stabilität bei Lagerung bei Zimmertemperatur oder nach Lyophilisation getestet. Derselbe Ansatz von *S. canidens* (Totes Meer) wie in Beispiel 2 wird verwendet. Die Ergebnisse dieses Experiments sind in Tabelle 4 dargestellt.

Tabelle 4

| Stabilität des Hundertfüsslergiftes (*S. canidens* aus der Umgebung des Toten Meeres) | | | |
|---|---|---|---|
| Bedingungen[a] | frisch gemolkenes Gift | fünftägige Lagerung bei Raumtemperatur | Tiefkühlung und Lyophilisierung |
| Aktivität[b] | 2,6 | 2,5 | 31 |

[a]in allen Behandlungen wird das Gift mit Wasser verdünnt
[b]die Aktivität wird als lähmende Einheit gegenüber *Sarcophaga*-Fliegenlarven bestimmt, ausgedrückt in ng Protein (Lowry et al., 1951)/100 mg Körpergewicht

Beispiel 4: Toxizität von Giften verschiedener Hunderfüssler der Gattung *Scolopendra* auf verschiedene Tiere

Tabelle 5: <u>Toxizität von Giften verschiedener Hunderfüssler der Gattung *Scolopendra* auf verschiedene Tiere</u>

| Hundertfüssler | *S. cingulata* | *S. canidens* (Jerusalem) | *S. canidens* (Totes Meer) |
|---|---|---|---|
| Test[a] | (µg) | (µg) | (µg) |
| Lähmung von *Sarcophaga*-Fliegenlarven | 0,13 | 0,14 | 0,005 |
| Lethalität von *Sarcophaga*-Fliegenlarven | 0,075 | 0,10 | 0,04 |
| Lähmung von *Spodoptera*-Larven | 8,23 | 8,82 | 2,99 |
| Lethalität von *Spodoptera*-Larven | 8,23 | 8,82 | 2,99 |
| Lähmung von erwachsenen *Locusta* | 0,65 | 0,64 | 0,031 |
| Lethalität von erwachsenen *Locusta* | 0,16 | 0,15 | 0,043 |
| $LD_{50}$ von Mäusen[b] | 350 | 245 | >1000 |

[a]die Lähmung wird bestimmt als $PU_{50}$, ausgedrückt in µg/100 mg KG, nach 30 sec für *Sarcophaga*-Larven und nach 5 Minuten für *Spodoptera* und *Locusta*. Die Lethalität wird nach 24 Stunden als lethale Dosis für 50 % ($LD_{50}$) bestimmt, ausgedrückt in µg/100 mg KG. Die Erprobung (5 oder 7 Tiere pro Dosis) und die Berechnung der Wirkdosen - 50 % erfolgt gemäss Reed und Muench (1938). Das durchschnittliche KG der verschiedenen Tiere ist 130 bis 150 mg für *Sarcophaga*-Larven, 70 bis 400 mg für *Spodoptera*-Larven, 1,3 bis 1,6 g für männliche, erwachsene *Locusta* und 7 bis 12 g für Albinomäuse.
[b]ausgedrückt in µg/10 g KG.

Gifte werden von drei Arten Hundertfüssler gesammelt: *S. canidens* (Totes Meer), *S. canidens* - (Jerusalem) und *S. cingulata* (gesammelt in Obergaliläa und auf den Golanhöhen). Die Toxizität der lyophilisierten Gifte für drei Insektenarten und für Mäuse ist in Tabelle 5 dargestellt. Das Gift von *S. canidens* aus der Umgebung des Toten Meeres besitzt die höchste Toxizität für Insekten und ist praktisch inaktiv gegenüber Mäusen, das Toxin in diesem Gift ist somit selektiv für Insekten. Die Injektion von 1 mg/10 g Maus induziert noch nicht einmal Symptome einer Vergiftung.

Beispiel 5: Reaktion eines Hundertfüsslers auf das Gift eines Tieres derselben Art

Tabelle 6

| Reaktion eines Hundertfüsslers (*S. canidens* gesammelt in der Umgebung des Toten Meeres) auf das Gift eines Tieres derselben Art | | | | | |
|---|---|---|---|---|---|
| Hundertfüssler | Gewicht | injiziertes Gift | | Wirkung | |
| | (mg) | (µg Protein)[a] | Heuschrecken lähmende Einheiten[b] | sofort | nach 24 Stunden |
| 1 | 420 | 2,2 | 25 | keine | keine |
| 2 | 460 | 7,4 | 75 | keine | keine |
| 3 | 490 | 10,5 | 100 | keine | keine |
| 4 | 580 | 18,7 | 150 | keine | keine |
| 5 | 580 | 18,7 | 150 | vorübergehend[c] Lähmung | |
| 6 | 580 | 18,7 | 150 | vorübergehend[c] Tod Lähmung | |

[a]bestimmt nach Lowry et al. (1951)
[b]die lähmende Potenz der Giftprobe von *Locusta migratoria* ist 21,5 ng/100 mg KG. Die Anzahl der Heuschrecken-lähmenden Einheiten entspricht Heuschrecken mit einem Körergewicht, das identisch mit dem der entsprechenden Hundertfüssler ist.
[c]die Lähmung wird um die Injektionsstelle herum lokalisiert und verschwindet nach 20 Minuten

*S. canidens* aus der Umgebung des Toten Meeres ist resistent gegen Gift von Tieren derselben Art und kann einer Giftdosis widerstehen, die wenigstens 150 Heuschrecken vom selben Gewicht lähmen können (Tabelle 6).

Beispiel 6: Verlust der Toxizität durch Erhitzen

Die Wirkung von Hitze (80° C, 5 Minuten) auf die Stabilität des Giftes wird mit *Sarcophaga*-Fliegenlarven bestimmt. Wie in Tabelle 7 gezeigt, wird die Toxizität des Hundertfüssler-Giftes durch eine Testbehandlung zerstört, wie es gemäss der lähmenden Wirkung auf *Sarcophaga*-Larven bestimmt wird.

Tabelle 7

| Verlust der Toxizität durch Erhitzen[a] | | |
|---|---|---|
| Giftquelle | unbehandelt 2 µl (2 PU$_{50}$) | erhitzt (80° C, 5 min) 10 µl (10 PU$_{50}$) |
| *S. canidens* (Totes Meer) | aktiv | inaktiv |
| *S. canidens* (Jerusalem) | aktiv | inaktiv |

[a]die PU$_{50}$-Werte für *Sarcophaga*-Fliegenlarven sind 5 ng/100 mg KG, wenn das Gift von einem Hundertfüssler der Umgebung des Toten Meeres, und 150 ng/100 mg KG, wenn das Gift von einem Hundertfüssler der Umgebung von Jerusalem stammt.

27

Beispiel 7: Einfluss proteolytischer Enzyme auf die Toxizität von Hundertfüssler-Gift für *Sarcophaga*-Larven

Die Toxizität von Hundertfüssler-Gift wird durch gewöhnliche proteolytische Enzympräparate zerstört (Tabelle 8). Trypsin stellt sich als wirksamer heraus als Pronase. Dieses Ergebnis zeigt, dass das Hundertfüssler-Toxin ein Protein ist.

Tabelle 8

| Einfluss von Trypsin und Pronase E (Sigma USA) in einem Enzym/Substrat-Verhältnis von 5% auf die Toxizität des Giftes von *S. canidens* (Jerusalem) auf *Sarcophaga*-Larven | | |
|---|---|---|
| Behandlung | Inkubationsdauer (Stunden) | |
| | 1 | 5 |
| ohne Protease, 2 PU$_{50}$ injiziert | + | + |
| Trypsin und 10 PU$_{50}$ injiziert | - | - |
| Pronase E und 10 PU$_{50}$ injiziert | + | - |
| Trypsin injiziert | - | - |
| Pronase E injiziert | - | - |

[a]das Medium ist eine Phosphat-gepufferte Kochsalzlösung (pH 7,4 - Sigma USA).Der PU$_{50}$-Wert des Giftes beträgt 150 ng/100 mg KG.

Beispiel 8: Reinigung von Hundertfüssler-Giften

Hundertfüssler-Gifte werden teilweise durch Fraktionierung über eine analytische HPLC-Molekularsiebsäule und eine Reverse Phase-Chromatographie gereinigt.

Die Analyse unter Verwendung der Molekularsiebsäule (Suprose 12 10/30 Pharmacia; 0,05 M Ammoniumacetat-Puffer pH 8,5, Durchflussrate 0,5 ml/min) zeigt, dass nur 20 % der Toxizität für Fliegenlarven aus dem Gift von Hundertfüsslern aus der Umgebung des Toten Meeres erhalten bleiben können. Eine qualitative Unterscheidung wird zwischen Faktoren beobachtet, die eine Erschlaffung induzieren, und solchen, die kontraktiv auf Fliegenlarven wirken. In Hundertfüsslern aus der Umgebung des Toten Meeres entspricht der letztere Faktor Faktoren mit einem abgeschätzten MG von 15 bis 20 kD.

Für die Reverse Phase-HPLC-Analyse werden C-8 (Merck); A (Wasser + 0,1 % TFA); B (Isopropanol + Acetonitril + 0,1 % TFA) verwendet.

Die Elutionsprofile der drei Gifte stimmen nicht mit den "offiziellen" taxonomischen Definitionen der Hundertfüssler-Arten überein. *S. canidens* aus der Umgebung von Jerusalem und *S. cingulata* zeigen identische Elutionsprofile bei Molekularsieb- und Reverse Phase Chromatographie und unterscheiden sich damit beide vom Hundertfüssler *S. canidens* aus der Umgebung des Toten Meeres. Vor diesem Hintergrund ist auch zu sehen, dass alle drei Gruppen auf Grund ihrer Grössen und Farbmuster leicht zu unterscheiden sind.

Beispiel 9: Konstruktion eines vom Ti-Plasmid abgeleiteten Vektors

Der Vektor pCIB10 (Rothstein et al., 1987) ist ein vom Ti-Plasmid abgeleiteter Vektor, der für den Transfer chimärer Gene in Pflanzen via *A. tumefaciens* verwendet werden kann. Der Vektor leitet sich von dem Plasmid pRK252 ab, das einen weiten Wirtsbereich aufweist und das von Dr. W. Barnes, Washington University, St. Louis, Mo. bezogen werden kann. Der Vektor enthält weiterhin ein Gen, welches eine Kanamycinresistenz in *Agrobacterium* vermittelt und aus dem Transposon Tn903 stammt, sowie linke und rechte T-DNA Grenzsequenzen aus dem Ti-Plasmid pTiT37. Zwischen den Grenzsequenzen befinden sich eine Polylinkerregion aus dem Plasmid pUC18 und ein chimäres Gen, welches eine Kanamycinresistenz in Pflanzen hervorruft.

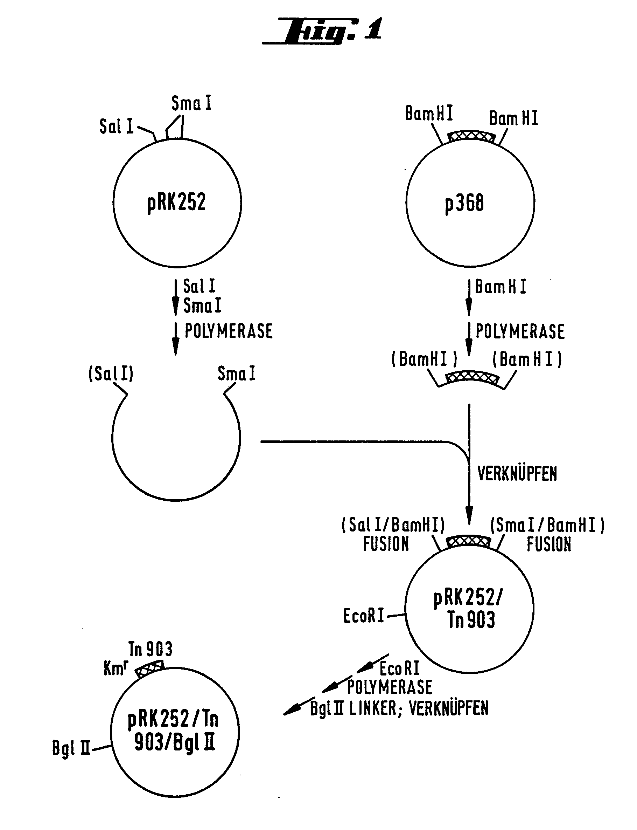

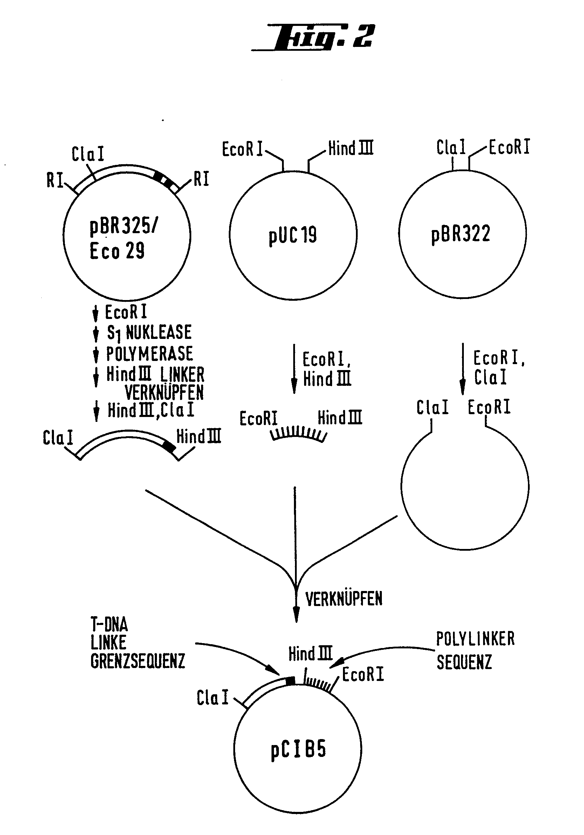

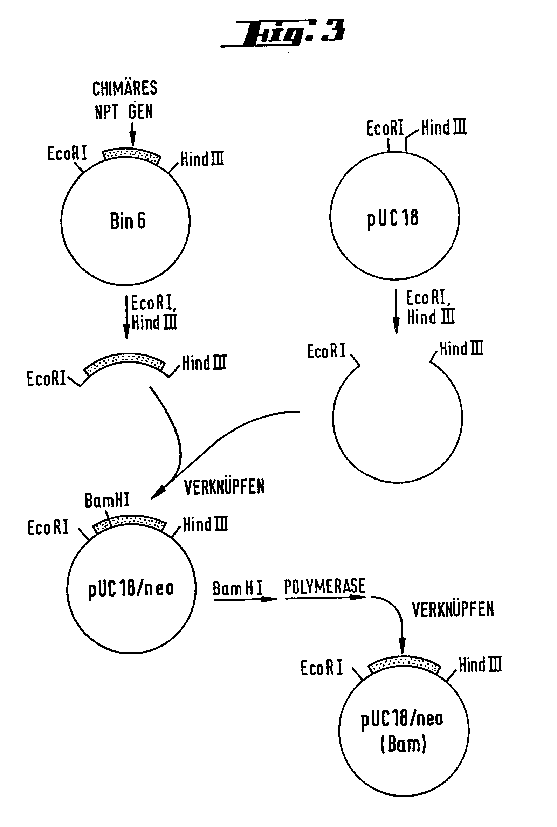

In einem ersten Verfahrensschritt wird das Plasmid pRK252 in der Weise modifiziert, dass das Tetracyclinresistenzgen gegen das Kanamycinresistenzgen aus dem Transposon Tn903 (Oka et al., 1981) ausgetauscht wird. Eine weitere Modifikation betrifft den Austausch der einzigen EcoRI Schnittstelle in pRK252 gegen eine BglII Schnittstelle (Fig. 1 fasst die Modifikation zusammen). Das Plasmid pRK252 wird zunächst mit den Endonukleasen SalI und SmaI geschnitten und anschliessend mit der grossen Untereinheit der DNA Polymerase I zur Herstellung glatter Enden behandelt. Das grosse Vektorfragment wird über eine Agarose-Gelelektrophorese gereinigt. Als nächstes wird das Plasmid p368, das Tn903 in einem etwa 1050 Bp grossen BamHI-Fragment enthält, mit der Endonuklease BamHI geschnitten und mit dem grossen Fragment der DNA Polymerase behandelt. Das etwa 1050 Bp umfassende Fragment wird nach Durchführung einer Agarose-Gelelektrophorese isoliert. Dieses Fragment enthält das Gen aus dem Transposon Tn903, das eine Resistenz gegenüber dem Antibiotikum Kanamycin vermittelt (Oka et al., 1981). Plasmid p368 ist bei der ATCC unter der Hinterlegungsnummer 67700 hinterlegt. Zur Erzeugung glatter Enden werden beide Fragmente mit der grossen Untereinheit der DNA-Polymerase behandelt. Anschliessend werden beide Fragmente vermischt und über Nacht bei einer Temperatur von 50° C mit T4 DNA-Ligase inkubiert. Nach der Transformation des E. coli-Stamms HB101 und einer Selektion Kanamycin-resistenter Kolonien erhält man das Plasmid pRK252/Tn903.

Das so erhaltene Plasmid pRK252/Tn903 wird an seiner einzigen EcoRI Schnittstelle geschnitten und anschliessend zur Herstellung glatter Enden mit der grossen Untereinheit der E. coli DNA-Polymerase behandelt. Dieses Fragment wird mit synthetischen sogenannten Linkern, die eine BglII Restriktionsschnittstelle enthalten, vermischt und über Nacht mit T4 DNA-Ligase inkubiert. Die aus dieser Behandlung resultierende DNA wird mit einem Überschuss an BglII Restriktionsendonuklease geschnitten und das grössere Vektorfragment mit Hilfe einer Agarose-Gelelektrophorese gereinigt. Das resultierende Fragment wird erneut mit T4 DNA Ligase inkubiert, um das Fragment über seine neu hinzugefügten kohäsiven BglII Enden zu rezirkularisieren. Nach Transformation des E. coli-Stamms HB 101 erhält man das Plasmid pRK252/Tn903/BglII (Fig. 1).

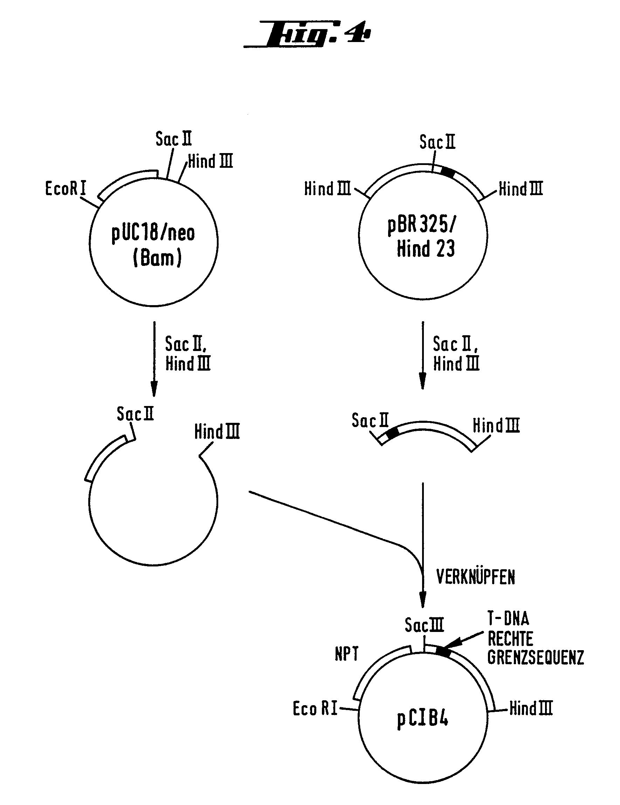

Im nächsten Verfahrensschritt wird ein Derivat des Plasmids pBR322 konstruiert, das neben den T-DNA Grenzsequenzen aus dem Ti-Plasmid und der Polylinkerregion aus dem Plasmid pUC19 ein selektierbares Gen für Kanamycinresistenz in Pflanzen enthält (Fig. 2). Das Plasmid pBR325/Eco29 enthält das 1.5 kBp umfassende EcoRI Fragment aus dem Nopalin Ti-Plasmid pTiT37. Dieses Fragment enthält die linke T-DNA Grenzsequenz (Yadav et al., 1982). Für den Austausch der EcoRI Enden dieses Fragments durch HindIII Enden wird das Plasmid pBR325/Eco29 mit EcoRI geschnitten und anschliessend mit Nuklease S1 inkubiert. Es folgt eine Inkubation mit dem grossen Fragment der DNA-Polymerase zur Herstellung glatter Enden. Dieser Reaktionsansatz wird mit synthetischen HindIII-Linkern vermischt und mit T4 DNA-Ligase inkubiert. Die resultierende DNA wird mit den Endonukleasen ClaI und einem Überschuss an HindIII geschnitten; das resultierende 1.1 kBp umfassende Fragment, das die linke T-DNA Grenzsequenz enthält, wird mit Hilfe einer Gelelektrophorese gereinigt. Als nächstes wird die Polylinkerregion des Plasmids pUC19 isoliert, indem man die Plasmid DNA mit den Endonukleasen EcoRI und HindIII schneidet und das kleinere Fragment (annähernd 53 Bp) über eine Agarose-Gelelektrophorese isoliert. Anschliessend wird das Plasmid pBR322 mit den Endonukleasen EcoRI und ClaI geschnitten, mit den beiden anderen, zuvor isolierten Fragmenten vermischt, mit T4 DNA-Ligase inkubiert und damit der E. coli Stamm HB101 transformiert. Das resultierende Plasmid pCIB5 enthält die Polylinkerregion und die linke T-DNA Grenzsequenz integriert in einen Abkömmling des Plasmids pBR322 (Fig. 2).

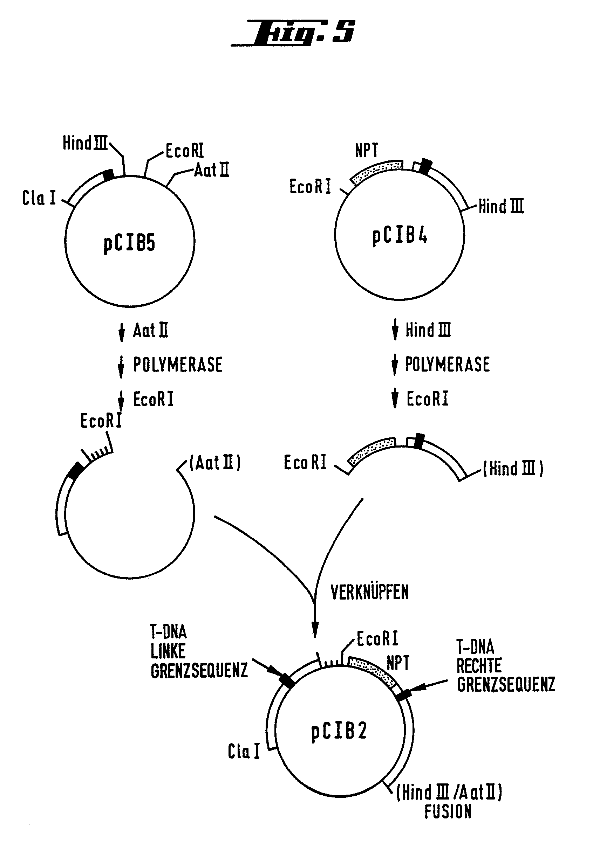

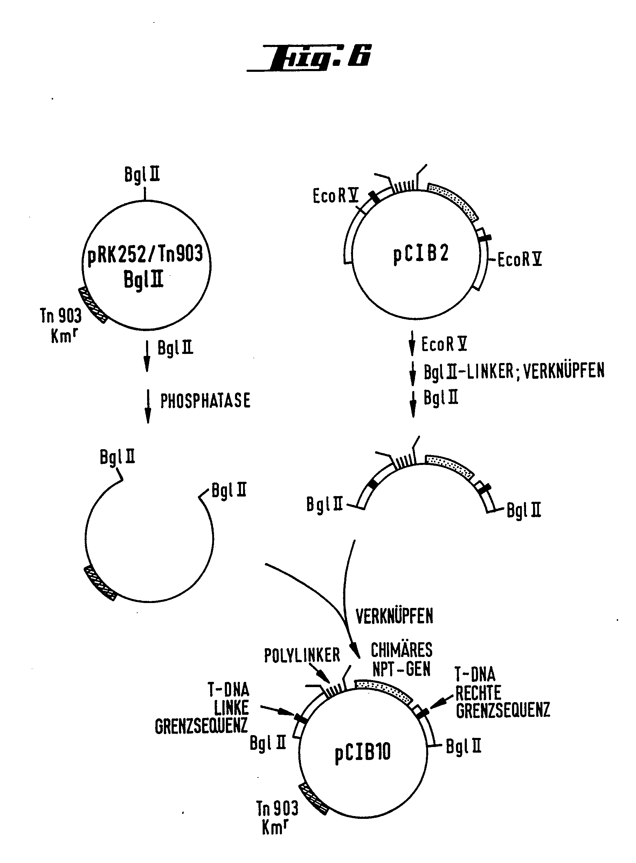

Als nächstes wird ein Plasmid konstruiert, das ein Gen enthält, welches die Expression von Kanamycinresistenz in Pflanzen vermittelt (Fig. 3 und 4). Das Plasmid Bin 6 (Bevan, 1984) ist erhältlich bei Dr. M. Bevan, Plant Breeding Institute, Cambridge, UK. Das Plasmid Bin 6 wird mit EcoRI und HindIII geschnitten. Das etwa 1.5 kBp umfassende Fragment, welches das chimäre NPT-Gen enthält, wird isoliert und anschliessend über eine Agarosegelelektrophorese gereinigt. Das Fragment wird mit pUC18 DNA vermischt, die zuvor mit den Endonukleasen EcoRI und HindIII geschnitten wurde. Nach Inkubation mit T4 DNA-Ligase wird mit der resultierenden DNA der E. coli Stamm HB101 transformiert. Das entstehende Plasmid wird als pUC18/neo bezeichnet. Dieses Plasmid enthält eine unerwünschte BamHI-Schnittstelle zwischen dem NPT-Gen und der Terminator-Sequenz des Nopalinsynthase-Gens (Bevan, 1984). Um diese Erkennungssequenz zu entfernen, wird das Plasmid pUC18/neo mit der Endonuklease BamHI geschnitten, gefolgt von einer Behandlung mit der grossen Untereinheit der DNA-Polymerase zur Erzeugung glatter Enden. Um das Fragment zu rezirkularisieren, wird es anschliessend mit T4 DNA-Ligase inkubiert. Mit diesem Fragment wird E. coli Stamm HB101 transformiert. Das resultierende Plasmid pUC18/neo(Bam) besitzt keine BamHI-Erkennungssequenz mehr.

Weiterhin wird die rechte T-DNA Grenzsequenz unmittelbar neben das chimäre NPT Gen inseriert (Fig.

4). Das Plasmid pBR325/Hind23 enthält das 3.4 kBp HindIII-Fragment des Plasmids pTiT37. Dieses Fragment enthält die rechte T-DNA Grenzsequenz (Bevan et al., 1983). Das Plasmid pBR325/Hind23 wird mit den Endonukleasen SacII und HindIII geschnitten und ein 1.0 kBp umfassendes Fragment, welches die rechte Grenzsequenz enthält, im Anschluss an eine Agarosegelelektrophorese in gereinigter Form isoliert. Das Plasmid pUC18/neo(Bam) wird mit den Endonukleasen SacII und HindIII geschnitten und das 4.0 kBp umfassende Vektorfragment mit Hilfe einer Agarosegelelektrophorese isoliert. Die beiden Fragmente werden miteinander vermischt, mit T4 DNA Ligase inkubiert und damit der E. coli Stamm HB 101 transformiert. Das resultierende Plasmid pCIB4 (Fig. 4) enthält die rechte T-DNA Grenzsequenz sowie den in Pflanzen selektierbaren Marker für Kanamycinresistenz in einem Abkömmling des Plasmids pUC18.

In einem letzten Verfahrensschritt wird ein Plasmid konstruiert, das sowohl die linke als auch die rechte T-DNA Grenzsequenz Pflanzen selektierbare Kanamycinresistenzgen und den Polylinker des Plasmids pUC18 enthält (Fig. 5). Zunächst wird das Plasmid pCIB4 mit der Endonuklease HindIII geschnitten, gefolgt von einer Behandlung mit der grossen Untereinheit der DNA Polymerase zur Herstellung glatter Enden sowie einer Fragmentierung mit der Endonuklease EcoRI. Das 2.6 kBp umfassende Fragment, welches das chimäre Kanamycinresistenzgen und die rechte T-DNA Grenzsequenz enthält, wird mit Hilfe einer Agarosegelelektro phorese isoliert. Anschliessend wird das Plasmid pCIB5 mit der Endonuklease AatII geschnitten, zur Erzeugung glatter Enden mit T4 DNA-Polymerase behandelt und dann mit der Endonuklease EcoRI geschnitten. Das grössere Vektorfragment wird mit Hilfe einer Agarosegelelektrophorese gereinigt, mit dem pCIB4-Fragment vermischt und mit T4 DNA-Ligase inkubiert. Mit diesem Fragment wird der E. coli Stamm HB101 transformiert. Das resultierende Plasmid pCIB2 (Fig. 5) ist ein Derivat des Plasmids pBR322, das die gewünschten Sequenzen zwischen den beiden T-DNA Grenzsequenzen enthält.

Die folgenden Schritte komplettieren die Konstruktion des Vektors pCIB10 (Fig. 6). Das Plasmid pCIB2 wird mit der Endonuklease EcoRV geschnitten und, wie zuvor beschrieben, mit synthetischen Linkern, die eine BglII Erkennungsstelle enthalten, versehen. Nach Schneiden mit einem Überschuss an BglII wird das annähernd 2.6 kBp umfassende Fragment mit Hilfe einer Agarosegelelektrophorese isoliert. Das zuvor bereits beschriebene Plasmid pRK252/Tn903/BglII (Fig. 1) wird mit der Endonuklease BglII geschnitten und anschliessend mit Phosphatase behandelt, um eine Rezirkularisierung zu verhindern. Diese beiden DNA Fragmente werden miteinander vermischt und mit T4 DNA-Ligase inkubiert. Anschliessend wird E. coli Stamm HB 101 transformiert. Das resultierende Plasmid ist der vervollständigte Vektor pCIB10.

Das Plasmid pCIB10 umfasst T-DNA-Grenzsequenzen, die ein in Pflanzen exprimierbares NPT-Gen und gebräuchliche Restriktionsschnittstellen zur Insertion innerhalb der T-DNA-Grenzsequenzen einschliessen.

Beispiel 10: Synthese von Genen, die die selektiv auf Insekten wirkenden Toxine kodieren

A. Reinigung von für Insekten selektiven Toxine. Die Reinigung verschiedener für Insekten selektiven Toxine ist bereits in mehreren Arbeiten von Zlotkin ( Zlotkin et al., 1971a und 1985, Lester et al., 1982) beschrieben worden. Ein alternatives Verfahren, mit dem hohe Ausbeuten bei limitierten Mengen Gift möglich sind, ist die HPLC. Diese Technik wird beispielhaft anhand der Reinigung von LqhIT2 dargestellt.

Zur Reinigung von LqhIT2 wird gefriergetrocknetes Gift von L. quinquestriatus hebraeus (Sigma) dreimal mit 0,5 ml Wasser/20 mg Gift extrahiert. Die wässrigen Extrakte werden vereinigt und einer Ionen-Austausch-Chromatographie über eine Sulfoethylaspartamid-HPLC-Säule (Nest Group) unterworfen. Der Extrakt wird auf die vorher mit 5 mM KPO$_4$, pH 3 in 25 % Acetonitril äquilibrierte Säule aufgetragen und die Säule mit einem KCl- Gradienten von 0 bis 0,5 M im gleichen Puffer 60 Minuten lang eluiert. Einzelne Fraktionen werden entsalzt und weiter über eine Reverse Phase Chromatographie über eine Vydac C-8-Säule, äquilibriert in 0,1 % TFA und eluiert mit einem 75 Minuten-Gradienten von 0 bis 70 % B (B = Acetonitril : Isopropanol 1 : 1 in 0,1 % TFA) aufgetrennt. Die Toxizität einzelner Fraktionen gegen Insekten wird durch Injektion in Sarcophaga- oder Heliothis-Larven getestet, wie von Zlotkin et al. (1985) beschrieben.

B. Aminosäurensequenzanalyse von auf Insekten wirkenden Toxinen. Das auf Insekten wirkende Toxin wird reduziert, indem Proben in 6 M Guanidin HCl, 1 M Tris HCl, pH 8,6, 10 mM EDTA und 20 mM Dithiothreitol eine Stunde bei 37° C inkubiert werden. 4-Vinylpyridin (Sigma) wird bis zu einer Endkonzentration von 50 mM hinzugefügt und die Inkubation eine Stunde bei Raumtemperatur fortgesetzt. Das modifizierte Protein wird wie oben beschrieben auf einer Vydac C-8-Säule entsalzt. Durch enzymatischen Abbau mit Trypsin, Lys-C oder Glu-C oder durch partielle saure Hydrolyse gemäss Standardverfahren (Allen, 1981) werden Peptide produziert. Vor der Sequenzierung werden die Peptide durch Reverse Phase HPLC getrennt. Die Aminosäurensequenzen des intakten Toxins und der einzelnen Peptide werden durch einen automatischen Edman-Abbau bestimmt, indem ein Model 470A Protein Sequencer (Applied Biosystems, Foster City, CA) benutzt wird, ausgestattet mit on-line Reverse Phase HPLC zur Analyse der Phenylthiohydantoin-Derivate der Aminosäuren und einem Model 900 Daten-Analyse-System.

Die Sequenzen anderer auf Insekten wirkender Toxine, die unter Benutzung der gleichen Verfahren bestimmt wurden, sind in Fig. 7 wiedergegeben.

C. Synthese des Gens, das ein auf Insekten wirkendes Toxin kodiert. Da die auf Insekten wirkenden Toxine kleine Proteine (< 80 Aminosäuren) sind, kann ein Gen, das ein Toxin kodiert, durch DNA-Synthese konstruiert werden. Nachfolgend wird die Synthese eines Gens beschrieben, das AaIT kodiert, das auf Insekten wirkende Toxin von *Androctonus australis*.

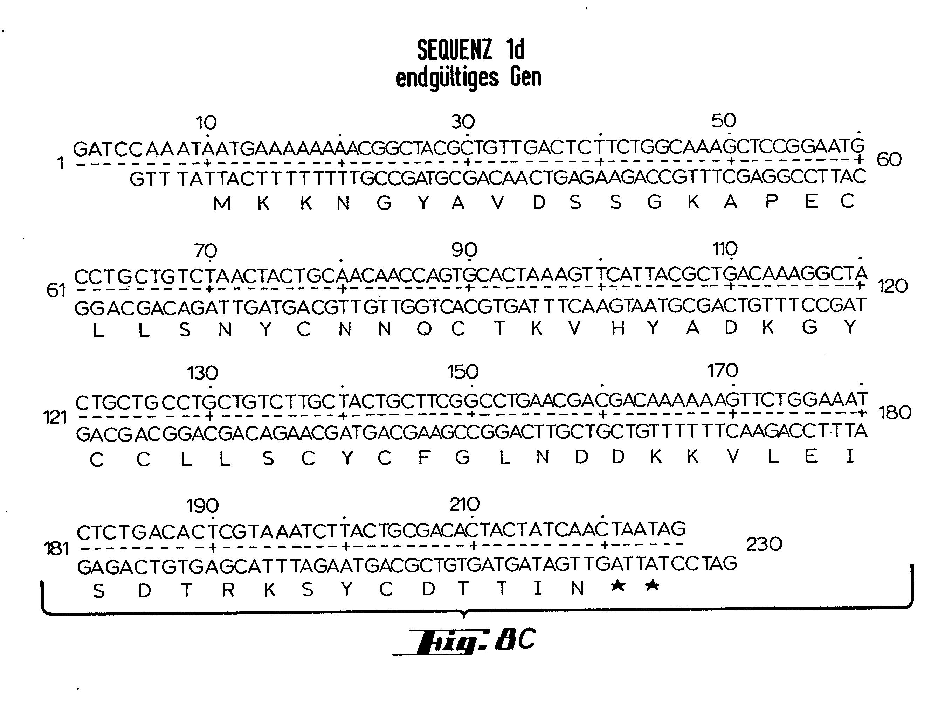

Die veröffentlichte Sequenz (Darbon et al., 1982) wird rückübersetzt, indem der genetische Code mit der Codon-Häufigkeit, die aus allen Mais-Proteinen in der GenBank-Datenbank mit Hilfe der Computerprogramme der Genetics Computer Group der Universität von Wisconsin berechnet wurde, benutzt wird. In einigen Fällen können alternative Codons ausgewählt werden, um die Synthese zu erleichtern und/oder für passende Restriktionsschnittstellen zu sorgen. Stop- und Startsignale der Translation werden zusammen mit BamHI-Linkern zur Erleichterung in folgenden Handhabungen an beide Enden angefügt. Durch diesen Prozess werden die Sequenzen Sequenzen 1a und 1b (Fig. 8) erhalten.

Oligonukleotide, die den Regionen 1 bis 20 (Sequenzen c) entsprechen, werden mit Hilfe eines Model 380A DNA-Synthesizers (Applied Biosystems, Foster City, CA) unter Anwendung der β-Cyanoethyl-Chemie synthetisiert.

Das Gen wird mit den folgenden Schritten zusammengesetzt:

(1) Vorbereitung der folgenden Reaktionsmischungen, die 40 pMol der jeweiligen Fragmente enthalten.

    A. Fragmente 2, 12, 13
    B. Fragmente 3, 4, 14, 15
    C. Fragmente 5, 6, 7, 16, 17, 18
    D. Fragmente 8, 9, 19, 20
    E. Fragmente 10, 11, 21

(2) Ein 5'-Phosphat wird an die 5'-Enden der Fragmente in jeder Mischung angefügt, indem T4 Polynukleotid-Kinase gemäss dem von Maniatis et al. (1982) beschriebenen Verfahren verwendet wird.

(3) Nach dem Entfernen von überschüssigem Reagens durch Phenol/Chloroform-Extraktion, Chloroform-Extraktion und Ethanol-Fällung wird der Niederschlag jeder Mischung, der die phosphorylierten Fragmente enthält, in T4-Ligase-Puffer gelöst. 40 pMol Fragment 1 wird zu Mischung A und 40 pMol Fragment 22 zu Mischung E hinzugefügt. Die Mischungen werden auf 85°C erhitzt, anschliessend langsam auf 15°C abgekühlt und bei dieser Temperatur für wenigstens 4 Stunden inkubiert, damit die Fragmente hybridisieren können.

(4) ATP wird bis zu einer Konzentration von 1 mM zusammen mit T4 Ligase hinzugefügt und die Inkubation wird 4 Stunden lang fortgesetzt. Die Reagentien werden durch Extraktion und Fällung wie in Schritt (1) entfernt. Um die Wirksamkeit der Reaktion zu überprüfen, wird ein Aliquot der Produkte in einem 10 bis 15 % Acrylamidgel analysiert. Falls nötig, kann das ent sprechende Fragment durch präparative Gelelektrophorese gereinigt und aus dem Gel gewonnen werden. Verunreinigungen werden wiederum durch Fällung entfernt.

Die folgenden Fragmentgrössen sind nach dem ersten Verknüpfungsschritt zu erwarten:

    Mischung A: 49 Bp
    Mischung B: 45 Bp
    Mischung C: 65 Bp
    Mischung D: 45 Bp
    Mischung E: 46 Bp

(5) Die Produkte der ersten Verknüpfungen von A und B werden in Reaktion F vermischt. Die Produkte der Verknüpfungen von D und E werden für Reaktion G vermischt. Die Schritte (3) und (4) werden mit den Mischungen F und G wiederholt. Aus Reaktion F resultiert ein 89 Bp Fragment und aus Reaktion G ein 86 Bp Fragment.

(6) Die gereinigten Fragmente von F, G und C werden miteinander vermischt und die Schritte (3) und (4) werden wiederholt, um das endgültige Gen von 230 Bp und mit BamHI-Enden zu erhalten. Die endgültige Sequenz ist als Sequenz 1d (Fig. 8) abgebildet. Das gereinigte Fragment wird zur Verknüpfung mit den BamHI-Schnittstellen geeigneter Vektoren verwendet.

(7) Um die DNA zu vervielfältigen, wird das gereinigte Fragment mit der BamHI-Schnittstelle von pUC18 verknüpft und in einem geeigneten *E. coli* Wirt kloniert. Die DNA-Sequenz des Einschubs wird unter Verwendung von standardisierten Sequenzierungsverfahren kontrolliert.

Beispiel 11: Ein Transformationsvektor für Pflanzen, der chimäre CaMV 35S Promotor/Toxin-Gene enthält

Vektoren, die einen in Pflanzen exprimierbaren Promotor und Schnittstellen zur Einfügung heterologer kodierender Sequenzen enthalten, stammen von pCIB10 ab (Rothstein et al., 1987). Das Plasmid pCIB770 enthält den CaMV 35S Promotor als in Pflanzen exprimierbaren Promotor. Die ein Toxin mit insektizider Aktivität kodierende Sequenz wird stromabwärts (downstream) des Promotors mit einer durch BamHI geschaffenen Schnittstelle verknüpft.

Das mit diesem Vektor transformierte Pflanzengewebe wird unter Verwendung der Antibiotika Kanamycin oder G418 wie weiter unten beschrieben und wie dem Fachmann bekannt selektioniert.

Beispiel 12: Transformationsvektoren für Pflanzen, die chimäre, in Pflanzen exprimierbare Toxin-Gene und einen Hygromycin-Resistenz-Marker zur Selektion von Pflanzen enthalten

Das Plasmid pCIB743 (Rothstein et al., 1987) enthält innerhalb der T-DNA-Grenzen ein in Pflanzen exprimierbares Hygromycin-Resistenz-Gen. Ein zweites in Pflanzen exprimierbares chimäres Gen wird unter Benutzung der einmal vorkommenden Restriktionsschnittstellen eingefügt und in Pflanzen eingeführt.

Das mit diesem Vektor transformierte Pflanzengewebe wird unter Verwendung des Antibiotikums Hygromycin oder analoger Antibiotika wie weiter unten beschrieben und wie dem Fachmann bekannt selektioniert.

Beispiel 13: Transformation von Tabak-Blattscheiben

A. Die Transformation von pflanzlichem Material mit *Agrobacterium*. Die verschiedenen Genotypen von *A. tumefaciens* werden in AB Minimalmedium (Watson et al., 1975) plus Mannit oder Glutamatsalze 48 Stunden lang bei 28° C kultiviert. Die Bakterien werden sedimentiert, in MSBN-Medium zweifacher Verdünnung resuspendiert und 3 Stunden lang bei 25° C inkubiert. Das MSBN-Medium besteht aus den kompletten sogenannten Haupt- und Nebensalzen (major and minor) Salzen gemäss Murashige und Skoog (1962) (KC Biologicals) mit den folgenden Zusätzen (Endkonzentrationen): 6-Benzyladenin (1 mg/liter); Nicotinsäure (1 mg/liter); Pyridoxin (1 mg/liter); Thiamin-HCl (10 mg/liter) und Saccharose (30 g/liter). Der pH ist auf 5,8 eingestellt. Blattscheiben mit einem Durchmesser von 5 bis 7 mm werden aseptisch aus in vitro kultiviertem *Nicotiana tabacum* cv. Xanthi ausgestanzt und 10 Minuten lang in eine Bakteriensuspension in einem abgewandelten Verfahren von Horsch et al, (1985) getaucht. Die Blattscheiben werden anschliessend auf Filterpapier auf MSBN-Medium gelegt. Nach 48 Stunden werden die Blattscheiben zur Selektion transformierter Zellen in flüsssiges MSBN-Medium, das 500 mg/liter Carbenicillin enthält, getaucht und auf festes MSBN-Medium (0,8 % Agar), das 100 mg/liter Kanamycin und 500 mg/liter Carbenicillin enthält, gelegt.

B. Reifung der Pflanzen und Selbstbefruchtung. Sprosse, die aus Kalli auf MSBN-Selektionsmedium entstehen, werden auf 0MS-Medium transferiert, das MS Haupt- und Nebensalze und Fe-EDTA (Gibco #500-1117; 4,3 g/liter), B5-Vitamine (Gamborg et al., 1968), 100 mg/liter myo-Inosit und 30 g/liter Saccharose, pH 5,8 enthält, ergänzt mit 100 mg/liter Kanamycin und 250 mg/liter Carbenicillin. Kanamycin und Carbenicillin werden als Filter-sterilisierte Lösungen hinzugefügt, nachdem der Rest des Mediums autoklaviert wurde. Man lässt die Pflänzchen wenigstens drei Wochen lang wachsen. Die Pflänzchen werden geteilt, um mehrfache Kopien von Schnitzeln zu erhalten, die man sich wenigstens drei Wochen lang entwickeln und bewurzeln lässt. Bewurzelte Pflänzchen werden dann in eine Erde-Vermiculite-Mischung umgetopft und ins Gewächshaus gebracht. Frisch eingetopfte Pflänzchen werden zur Abhärtung feucht und schattig unter einem umgedrehten Plastikbecher gehalten. Zur Blütezeit wird die Selbstbefruchtung induziert. Saatgut wird nach der Reife geerntet.

Beispiel 14: Produktion transgenen Tabakkallus und transgener Tabakpflanzen

Durch Paarung wird der T-DNA-enthaltende Vektor oder der Vektor, der die in Pflanzen exprimierbaren chimären Gene enthält, von *E. coli* SM17 (Simon et al., 1983) in *A. tumefaciens* CIB542 transferiert. Alternativ dazu wird *Agrobacterium* CIB542 mit dem Vektor gemäss dem Verfahren von Holsters et al. (1978) transformiert. *Agrobacterium* CIB542 ist Stamm EHA101 (Hood et al., 1986), in dem der Kanamycin-Marker des Plasmids durch den Spectinomycin/Streptomycin-Teil von Tn7 ersetzt wurde. *Agrobacterium-*

Stämme, die das vom T-DNA abgeleitete Plasmid tragen, und pCIB542 werden verwendet, um Tabak durch das oben beschriebene Blattscheiben-Verfahren zu transformieren. Kanamycin-resistente transformierte Pflanzen werden bis zur Reife kultiviert. Alternativ dazu wird Kallus, der sich aus den Blattscheiben auf Kanamycin-enthaltendem MSBN-Selektionsmedium entwickelt, auf einem Kallus-Wachstums-Medium gehalten, das MS Haupt-und Nebensalze und Fe-EDTA (Gibco #500-1117; 4,3 g/liter), MS-Vitamine, 100 mg/liter myo-Inosit, 20 g/liter Saccharose, 2 mg/liter Naphthalinessigsäure und 0,3 mg/liter Kinetin enthält.

Der Kallus kann benutzt werden, um transgene Pflanzen zu regenerieren, indem Kallusstücke auf MSBN-Medium transferiert werden und den oben beschriebenen Verfahren gefolgt wird.

Beispiel 15: Transformation und Regenerierung von *Zea mays*

*Zea mays* wird in den in Tabelle 9 genannten Medien transformiert und regeneriert.

Tabelle 9: Zusammensetzung der verwendeten Medien

Die Makro- und Mikroelemente sowie Fe-EDTA der Medien sind wie in der Literatur angegeben: KM-Medium gemäss Kao und Michayluk (1975), N6-Medium gemäss Chu et al. (1975).

| Medium | KM-8p | N6 |
|---|---|---|
| Organische Stoffe und Vitamine, die im Kulturmedium benutzt werden [mg/Liter]: | | |
| Biotin | 0.01 | |
| Pyridoxin HCl | 1.00 | 0.5 |
| Thiamin HCl | 10.00 | 0.1 |
| Nicotinamid | 1.00 | |
| Nicotinsäure | 0.10 | 0.5 |
| Folsäure | 0.40 | |
| D-Ca-Pantothenat | 1.00 | |
| p-Aminobenzoesäure | 0.02 | |
| Cholin-Chlorid | 1.00 | |
| Riboflavin | 0.20 | |
| Vitamin B-12 | 0.20 | |
| Glycin | 0.10 | 2.0 |
| Zucker und Zuckeralkohole [g/Liter]: | | |
| Saccharose | 0.25 | 30.0 |
| Glucose | 68.40 | |
| Mannit | 0.25 | |

| | | |
|---|---|---|
| Sorbit | 0.25 | |
| Cellobiose | 0.25 | |
| Fructose | 0.25 | |
| Mannose | 0.25 | |
| Ribose | 0.25 | |
| Xylose | 0.25 | |
| Myo-Inosit | 0.10 | |
| | | |
| End-pH | 5.8 | 5.6 |
| Sterilisierung | Filter | Autoklav |

Die Makroelemente werden gewöhnlicherweise als 10fach konzentrierte Stammlösung angesetzt, die Mikroelemente als 1000fach konzentrierte Stammlösung.

Citronen-, Fumar- und Apfelsäure (jeweilige Endkonzentration 40 mg/liter) sowie Natriumpyruvat (Endkonzentration 20 mg/liter) werden als eine 100fach konzentrierte Stammlösung hergestellt, mit NH₄OH auf pH 6.5 eingestellt und zum Medium gegeben.

Adenin (Endkonzentration 0,1 mg/liter) und Guanin, Thymidin, Uracil, Hypoxanthin und Cytosin (jeweilige Endkonzentration 0,03 mg/liter) werden als 1000fach konzentrierte Stammlösung hergestellt, mit NH₄OH auf pH 6.5 eingestellt und zum Medium gegeben.

Die folgenden Aminosäuren werden als eine 10fach konzentrierte Stammlösung (pH 6.5 mit NH₄OH) zum Medium gegeben und erreichen die folgenden Endkonzentrationen: Glutamin (5,6 mg/liter), Alanin, Glutaminsäure (jede 0,6 mg/liter), Cystein (0,2 mg/liter), Asparagin, Asparaginsäure, Cystin, Histidin, Isoleucin, Leucin, Lysin, Methionin, Phenylalanin, Prolin, Serin, Threonin, Tryptophan, Tyrosin und Valin (jede 0,1 mg/liter).

Die Vitamin-Stammlösung wird gewöhnlicherweise 100fach konzentriert zubereitet.


Beispiel 16: Herstellung von Antikörpern gegen selektiv auf Insekten wirkende Toxine

A. Herstellung des Immunogens. Standardisierte Computer-Analysen (Hopp und Woods, 1983) werden zur Voraussage antigener Bereiche aus den Aminosäuresequenzen von selektiv auf Insekten wirkenden Toxinen vorhergesagt. Synthetische Peptide, die diesen Bereichen entsprechen, werden hergestellt. Die Peptide werden über ein zwischengeschaltetes Cystein an einen Ovalbumin-Carrier gekoppelt, indem das Reagens N-Succinimidyl-3-(2-pyridyldithio)propionat (Pierce Chemical Co.) verwendet wird (Carlsson et al., 1978). Der Grad der Verknüpfung wird durch Aminosäureanalyse des Konjugates abgeschätzt.

B. Produktion von Antiseren. Kaninchen werden mit 0,5 bis 1,0 mg Antigen, emulgiert in komplettem Freundschen Adjuvans, immunisiert und monatlich mit Antigen in nichtkomplettem Freundschen Adjuvans geboostet. Der Titer der Seren wird durch gebräuchliche ELISA-Tests bestimmt, indem das Peptid, verknüpft mit einem heterologen Carrier (üblicherweise RSA) verwendet wird. Positive Seren werden gegen das geeignete auf Insekten wirkende Toxin titriert.

C. Ergebnisse. Typischerweise können mit Verdünnungen von 1:10 000 1 bis 10 ng der homologen Peptide nachgewiesen werden. Verdünnungen von 1:300 lassen den Nachweis von 3 bis 10 ng intakten Toxinproteins zu (Tabelle 10).

34

Tabelle 10

| Nachweisgrenzen von Antiseren gegen Toxin-Peptide im ELISA-Test | | | |
|---|---|---|---|
| Toxin | Immunisierendes Peptid | Nachweis des Peptids | Nachweis des Toxins |
| AaIT | N-terminal 1 bis 16 | 3 ng bei 1:10 000 | 3 ng bei 1:300 |
| AaIT | C-terminal 52 bis 70 | 3 ng bei 1:10 000 | 3 ng bei 1:300 |
| LqhIT2 | N-terminal 1 bis 13 | 1 ng bei 1:1 000 | |
| LqhIT2 | C-terminal 46 bis 61 | 1 ng bei 1:10 000 | |
| BjIT2 | N-terminal 1 bis 13 | 0,3 ng bei 1:3 000 | |
| BjIT2 | C-terminal 46 bis 60 | 10 ng bei 1:3 000 | 1 ng bei 1:300 |

Beispiel 17: Mais, der durch die Expression von AaIT resistent gegen *Diabrotica* ist

A. Konstruktion des Vektors. Das wie in Beispiel 9 hergestellte synthetische AaIT-Gen wird wie oben beschrieben mit der BamHI-Schnittstelle von pCIB710 (Rothstein et al., 1987) verknüpft. Das Gen eines gewünschten selektiven Markers (beispielsweise das NPT Gen, das Kanamycin-Resistenz verleiht) wird mit einer der vielfach vorhandenen Klonierungsstellen unter Verwendung standardisierter Techniken verknüpft.

B. Transformation und Regenerierung von Mais. Maisgewebe wird mit dem pCIB710 Vektor, der den AaIT-Geneinschub trägt, transformiert und Pflanzen werden wie in den obigen Beispielen beschrieben regeneriert. Zur Kontrolle werden Pflanzen, die nur mit dem pCIB710 Vektor transformiert sind, auf dieselbe Weise hergestellt. Die Ausgangspflanzen werden selbstbefruchtet und man erhält Samen (T1 Samen).

C. Test von Pflanzen auf AaIT-Expression. Pflanzen, die aus den T1 Samen gewachsen sind, werden auf das Vorhandensein und die Expression des AaIT-Gens hin untersucht, indem verschiedene Tests angewendet werden.

(1) DNA wird isoliert und mit BamHI geschnitten; die Abbauprodukte werden auf einem 1,5 % Agarose-Gel elektrophoretisch aufgetrennt. Die DNA-Fragmente werden auf Nitrocellulose übertragen und mit dem AaIT-Gen, das mit $^{32}$P durch nick- Translation markiert ist, hybridisiert (Maniatis et al., 1982). Die Anwesenheit des AaIT-Gens wird durch eine Bande ausfindig gemacht, die etwa 230 Bp entspricht und mit der Probe hybridisiert.

(2) RNA wird durch das Northern Blot Verfahren (Maniatis et al., 1982) als eine Bande ausfindig gemacht, die etwa 230 Basen entspricht und mit dem $^{32}$P-AaIT-Gen hybridisiert, das oben beschrieben ist.

(3) AaIT-Protein wird mit immunologischen Standardtechniken mit einem polyklonalen Kaninchen Antikörper ausfindig gemacht, der gegen synthetische Peptide, die den 16 N-terminalen und den 19 C-terminalen Aminosäuren von AaIT entsprechen, produziert wurde (siehe Beispiel 16).

(4) AaIT-Aktivität wird dadurch ausfindig gemacht, dass Material aus den Pflanzenextrakten mit immunologischen Methoden gereinigt wird, indem der polyklonale Anti-AaIT-Antikörper aus Kaninchen und Protein A Sepharose verwendet und das isolierte Material auf Toxizität gegen Insekten getestet wird, indem das Material in *Sarcophaga*-Larven injiziert wird, mit Verfahren, die in Beispiel 10 beschrieben sind.

D. Resistenz von transformierten Maispflanzen gegen Schaden durch *Diabrotica*. Aus T1 Samen erhaltene Keimlinge werden in grobem Vermikulit in 100 mm Petrischalen (5 pro Schale, 5 Schalen) gepflanzt. Wenn die zweiten Blätter am Keimling erscheinen, wird jede Schale mit 20 *Diabrotica*-Larven im zweiten Häutungsstadium infiziert. Nach 7 Tagen werden die Zahl, das Gewicht der Überlebenden und das Gewicht der gewaschenen Wurzeln der Maispflanze bestimmt. Die Resistenz der transformierten Pflanzen wird durch eine statistisch signifikante (Student's Test p <0,05) Erniedrigung der Gewichtszunahme der Larven, Erniedrigung der Überlebensrate der Larven oder in der Erniedrigung im Verlust des Wurzelgewichts, verglichen mit Insekten-freien Pflanzen bestimmt, indem Pflanzen, die das AaIT-Gen exprimieren, mit Kontrollpflanzen, die nur mit dem Vektor ohne Geneinschub transformiert sind, oder mit nicht-transformierten Pflanzen verglichen werden.

Beispiel 18: Kartoffeln und Tomaten, die durch die -Expression von AaIT resistent gegen *Leptinotarsa decemlineata* (Kartoffelkäfer) sind

A. Konstruktion des Vektors. Das AaIT-Gen wird synthetisiert und mit dem Vektor pCIB710 verknüpft wie es in Beispiel 17, Teil A beschrieben ist. Das AaIT-Gen wird zusammen mit dem 35S CaMV-Promotor aus dem pCIB710-Vektor herausgeschnitten und in den Vektor pCIB10 eingebracht, indem pCIB710 mit XbaI und EcoRI geschnitten wird, das dabei entstehende 1460 Bp Fragment isoliert und mit dem zuvor mit XbaI und EcoRI geschnittenen pCIB10 verknüpft wird, um den pCIB10-AaIT-Vektor zu erhalten.

B. Transformation und Regenerierung von Pflanzen. Der pCIB10-AaIT-Vektor wird in *A. tumefaciens*, dass ein Virulenz-Plasmid wie beispielsweise LBA 4404 oder pCIB542 trägt, eingeführt. pCIB542 ist ein *A. tumefaciens* Plasmid, das ein bearbeitetes, von pTiBo542 abgeleitetes *vir*-Plasmid (Hood et al., 1986) enthält. Bei pCIB542 ist das bakterielle Kanamycin-Resistenz-Gen ersetzt durch ein bakterielles Streptomycin/Spectinomycin-Resistenz-Gen. Der Stamm, der sowohl pCIB710-AaIT als auch pCIB542 enthält, wird verwendet, um Tomatenpflanzen gemäss Fischhoff et al. (1987) zu transformieren. Kartoffelpflanzen, die pCIB10-AaIT enthalten, werden gemäss Stockhaus et al. (1987) erhalten.

C. Test von Transformanten auf AaIT-Expression. Transformanten werden auf die Expression von AaIT getestet wie in Beispiel 17, Teil C beschrieben.

D. Resistenz von transformierten Pflanzen gegen Schaden durch den Kartoffelkäfer. Zehn vier Wochen alte Pflanzen werden je mit fünf Kartoffelkäferlarven im zweiten Häutungsstadium infiziert. Nach 4 Tagen werden die Insektenmortalität, die Gewichtszunahme der Insekten und das Ausmass des der Pflanze zugefügten Schadens bestimmt. Die Resistenz der transformierten Pflanzen wird durch eine statistisch signifikante (Student's Test $p < 0{,}05$) Erniedrigung der Gewichtszunahme der Larven, Erniedrigung der Überlebensrate der Larven oder in der Erniedrigung des Pflanzenschadens bestimmt, indem Pflanzen, die das AaIT-Gen exprimieren, mit Kontrollpflanzen, die nur mit dem Vektor ohne Geneinschub transformiert sind, oder mit nicht-transformierten Pflanzen verglichen werden.

Beispiel 19: *Dactylis glomerata* (Knäuelgras), das durch die Expression von AaIT resistent gegen Käfer ist

A. Konstruktion des Vektors. Das AaIT-Gen wird synthetisiert und mit dem Vektor pCIB710 zusammen mit dem Kanamycin-Resistenz-Gen verknüpft wie es in Beispiel 17, Teil A beschrieben ist.

B. Transformation und Regenerierung von Pflanzen. Transformierte Pflanzen werden erhalten wie oben beschrieben.

C. Test von Transformanten auf AaIT-Expression. Transformanten werden auf die Expression von AaIT getestet wie in Beispiel 17, Teil C beschrieben.

D. Resistenz von transformierten Pflanzen gegen Schaden durch *Diabrotica undecimpunctata*. Aus T1 Samen erhaltene Keimlinge werden in grobem Vermikulit in 100 mm Petrischalen (10 pro Schale, 5 Schalen) gepflanzt. Wenn die zweiten Blätter am Keimling erscheinen, wird jede Schale mit 20 *D. undecimpunctata*-Larven im zweiten Häutungsstadium infiziert. Nach 7 Tagen werden die Zahl, das Gewicht der Überlebenden und das Gewicht der gewaschenen Wurzeln der Maispflanze bestimmt. Die Resistenz der transformierten Pflanzen wird durch eine statistisch signifikante (Student's Test $p < 0{,}05$) Erniedrigung der Gewichtszunahme der Larven, Erniedrigung der Überlebensrate der Larven oder in der Erniedrigung im Verlust des Wurzelgewichts, verglichen mit Insekten freien Pflanzen bestimmt, indem Pflanzen, die das AaIT-Gen exprimieren, mit Kontrollpflanzen, die nur mit dem Vektor ohne Geneinschub transformiert sind, oder mit nicht-transformierten Pflanzen verglichen werden.

Beispiel 20: Baumwolle, die durch die Expression von AaIT resistent gegen den Baumwollkapselkäfer (*Anthonomus grandis*) ist

A. Konstruktion des Vektors. Das AaIT-Gen in pCIB10 wird hergestellt wie es in Beispiel 18, Teil A beschrieben ist.

B. Transformation und Regenerierung von Pflanzen. Transformierte Pflanzen werden erhalten wie oben beschrieben.

C. Test von Transformanten auf AaIT-Expression. Transformanten werden auf die Expression von AaIT getestet wie in Beispiel 17, Teil C beschrieben.

D. Resistenz transformierter Pflanzen gegen Schaden durch *Anthonomus grandis*. Zehn transformierte Pflanzen werden kultiviert bis sich Kapseln bilden. Jede Pflanze wird mit drei erwachsenen weiblichen *Anthonomus grandis* infiziert. Der Schaden an den Pflanzen wird nach einer Woche bestimmt und überlebende Erwachsene werden entfernt. Schaden an den Larven, Larvenzahl und Gewicht jeder Pflanze werden mit wöchentlichen Abständen vier Wochen lang gemessen. Die Resistenz der transformierten Pflanzen wird durch eine statistisch signifikante (Student's Test p < 0,05) Erniedrigung der Schadensrate, Abnahme der Larvenzahl oder Erniedrigung der Gewichtszunahme der Larven bestimmt, indem Pflanzen, die das Aalt-Gen exprimieren, mit Kontrollpflanzen, die nur mit dem Vektor ohne Geneinschub transformiert sind, oder mit nicht-transformierten Pflanzen verglichen werden.

## Beispiel 21: Mais, der durch die Expression von LqhIT2 resistent gegen Schmetterlinge ist

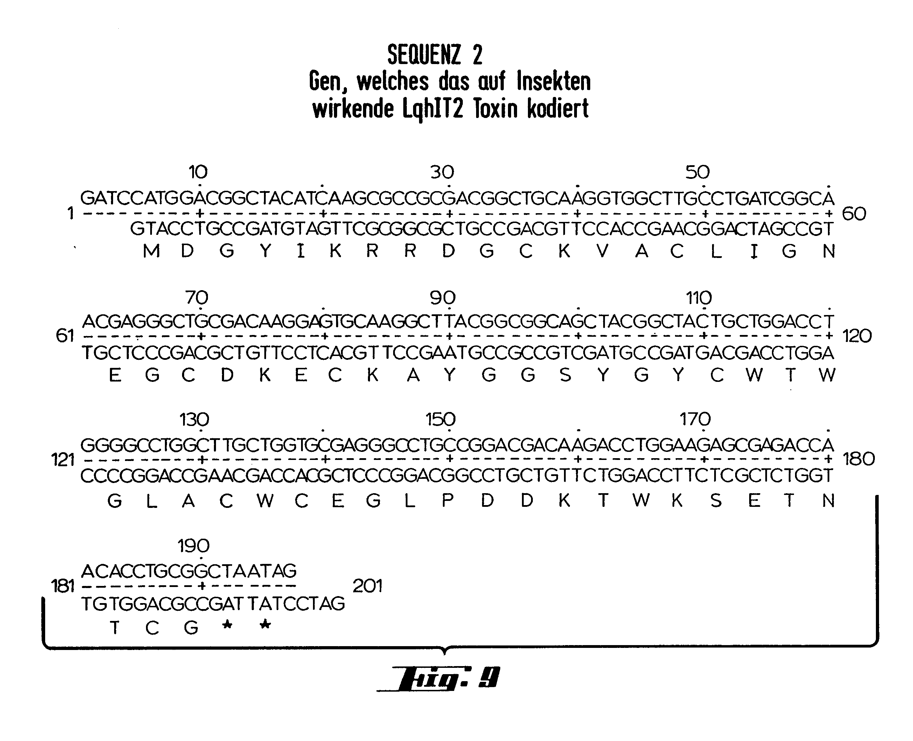

A. Konstruktion des pCIB710-LqhIT2-Vektors. Ein synthetisches Gen für LqhIT2 wird hergestellt, indem vorgegangen wird, wie es allgemein für das AaIT-Gen in Beispiel 9 beschrieben ist. Das resultierende Gen hat die Sequenz, die als Sequenz 2 (Fig. 9) abgebildet ist. Das resultierende 200 Bp Fragment wird isoliert und mit der BamHI-Schnittstelle des pCIB710-Vektors verknüpft, wie es in Beispiel 17, Teil A beschrieben ist. Das Gen für den gewünschten selektiven Marker (beispielsweise das NPT-Gen, das Kanamycin-Resistenz verleiht) wird mit einem der zahlreichen Klonierungsstellen verbunden, indem Standardtechniken verwendet werden. Der resultierende Vektor wird pCIB710-LqhIT2 genannt.

B. Transformation und Regenerierung von Mais. Die Transformation und Regenerierung von Maispflanzen wird durchgeführt, wie es in Beispiel 17, Teil B beschrieben ist.

C. Test von Pflanzen auf die Expression von LqhIT2. Pflanzen, die aus dem T1-Saatgut gezogen wurden, werden auf das Vorhandensein des LqhIT2-Gens hin analysiert, indem verschiedene Tests angewendet werden.

(1) DNA wird isoliert und mit BamHI geschnitten; die Abbauprodukte werden auf einem 1,5 % Agarose-Gel elektrophoretisch aufgetrennt. Die DNA-Fragmente werden auf Nitrocellulose übertragen und mit dem LqhIT2-Gen, das mit $^{32}P$ durch nick-Translation markiert ist, hybridisiert (Maniatis et al., 1982). Die Anwesenheit des LqhIT2-Gens wird durch eine Bande ausfindig gemacht, die etwa 200 Bp entspricht und mit der Probe hybridisiert.

(2) RNA wird durch das Northern Blot Verfahren (Maniatis et al., 1982) als eine Bande ausfindig gemacht, die etwa 200 Basen entspricht, und mit dem $^{32}P$-LqhIT2-Gen hybridisiert, das oben beschrieben ist.

(3) LqhIT2-Protein wird mit immunologischen Standardtechniken mit polyklonalen Kaninchen Antikörpern ausfindig gemacht, die gegen synthetische N- und C-terminale Peptide von LqhIT2 wie in Beispiel 16 beschrieben produziert wurden.

(4) LqhIT2-Aktivität wird dadurch ausfindig gemacht, dass Material aus den Pflanzenextrakten mit immunologischen Methoden gereinigt wird, indem der polyklonale Anti-LqhIT2-Antikörper aus Kaninchen und Protein A Sepharose verwendet und das isolierte Material auf Toxizität gegen Insekten getestet wird, indem das Material in *Heliothis*-Larven injiziert wird wie in Beispiel 10 beschrieben.

D. Resistenz transformierter Pflanzen gegen Schäden durch Schmetterlingslarven. T1-Samen werden gekeimt und Blattstücke von Keimlingen im Vierblattstadium werden an frischgeschlüpfte *Ostrinia nubilalis* oder *Heliothis zea*-Larven verfüttert. Frischgeschlüpfte Larven werden einzeln in Fütterungstöpfe mit einem 1 cm² Blattstück gesetzt. Fünfzig Insekten werden pro Gruppe getestet. Nach 5 Tagen werden das Gewicht der Insekten, die Zahl der Überlebenden und die Menge der gefressenen Blätter bestimmt. Die Resistenz der transformierten Pflanzen wird durch eine statistisch signifikante (Student's Test p <0,05) Erniedrigung der Gewichtszunahme der Larven, Erniedrigung der Überlebensrate der Larven oder in der Erniedrigung der gefressenen Blattmenge bestimmt, indem Pflanzen, die das LqhIT2-Gen exprimieren, mit Kontrollpflanzen, die nur mit dem Vektor ohne Geneinschub transformiert sind, oder mit nicht-transformierten Pflanzen verglichen werden.

## Beispiel 22: Baumwolle, die durch die Expression von LqhIT2 resistent gegen Schmetterlinge ist

A. Konstruktion des Vektors. Das LqhIT2-Gen wird zusammen mit dem 35S CaMV-Promotor aus pCIB710 entfernt und unter Verwendung geeigneter Restriktionsenzyme mit pCIB10 verknüpft. Dieser Vektor wird pCIB10-LqhIT2 genannt.

B. Transformation und Regenerierung von Pflanzen. Transformierte Pflanzen werden wie oben beschrieben erhalten.

C. Test von Transformanten auf die Expression von LqhIT2. Transformanten werden wie in Beispiel 21, Teil C beschrieben auf die Expression von LqhIT2 getestet.

D. Resistenz transformierter Pflanzen gegen Schäden durch Schmetterlingslarven. Blattstücke von vier Wochen alten transformierten Pflanzen werden an frischgeschlüpfte *Heliothis virescens, Heliothis zea* oder *Pectinophora gossypiella* verfüttert. Frischgeschlüpfte Larven werden einzeln in Fütterungstöpfe mit einem 1 cm² Blattstück gesetzt. Fünfzig Insekten werden pro Gruppe getestet. Nach 5 Tagen werden das Gewicht der Insekten, die Zahl der Überlebenden und die Menge der gefressenen Blätter bestimmt. Die Resistenz der transformierten Pflanzen wird durch eine statistisch signifikante (Student's Test p < 0,05) Erniedrigung der Gewichtszunahme der Larven, Erniedrigung der Überlebenstrate der Larven oder in der Erniedrigung der gefressenen Blattmenge bestimmt, indem Pflanzen, die das LqhIT2-Gen exprimieren, mit Kontrollpflanzen, die nur mit dem Vektor ohne Geneinschub transformiert sind, oder mit nicht-transformierten Pflanzen verglichen werden.

Beispiel 23: Tomaten, die durch die Expression von LqhIT2 resistent gegen Schmetterlinge sind

A. Konstruktion des Vektors. Der pCIB10-LqhIT2-Vektor wird wie in Beispiel 22, Teil A beschrieben hergestellt.

B. Transformation und Regenerierung von Pflanzen. Transformierte Pflanzen werden wie in Beispiel 17, Teil B beschrieben hergestellt, indem der pCIB10-LqhIT2-Vektor an Stelle des pCIB10-AaIT-Vektors benutzt wird.

C. Test von Transformanten auf die Expression von LqhIT2. Transformanten werden wie in Beispiel 21, Teil C beschrieben auf die Expression von LqhIT2 getestet.

D. Resistenz transformierter Pflanzen gegen Schäden durch Schmetterlinge. Blattstücke von vier Wochen alten transformierten Pflanzen werden an frischgeschlüpfte *Heliothis zea* oder *Manduca sexta* verfüttert. Frischgeschlüpfte Larven werden einzeln in Fütterungstöpfe mit einem 1 cm² Blattstück gesetzt. Fünfzig Insekten werden pro Gruppe getestet. Nach 5 Tagen werden das Gewicht der Insekten, die Zahl der Überlebenden und die Menge der gefressenen Blätter bestimmt. Die Resistenz der transformierten Pflanzen wird durch eine statistisch signifikante (Student's Test p < 0,05) Erniedrigung der Gewichtszunahme der Larven, Erniedrigung der Überlebensrate der Larven oder in der Erniedrigung der gefressenen Blattmenge bestimmt, indem Pflanzen, die das LqhIT2-Gen exprimieren, mit Kontrollpflanzen, die nur mit dem Vektor ohne Geneinschub transformiert sind, oder mit nicht-transformierten Pflanzen verglichen werden.

Beispiel 24: Tabak, der durch die Expression von LqhIT2 resistent gegen Schmetterlingslarven ist

A. Konstruktion des Vektors. Der pCIB10-LqhIT2-Vektor wird wie in Beispiel 22, Teil A beschrieben hergestellt.

B. Transformation und Regenerierung von Pflanzen. Transformierte Pflanzen werden wie oben beschrieben hergestellt, indem der pCIB10-LqhIT2-Vektor benutzt wird.

C. Test von Transformanten auf die Expression von LqhIT2. Transformanten werden wie in Beispiel 21, Teil C beschrieben auf die Expression von LqhIT2 getestet.

D. Resistenz transformierter Pflanzen gegen Schäden durch Schmetterlingslarven. Blattstücke von vier Wochen alten transformierten Pflanzen werden an frischgeschlüpfte *Heliothis virescens* oder *Manduca sexta* erfüttert. Frischgeschlüpfte Larven werden einzeln in Fütterungstöpfe mit einem 1 cm² Blattstück gesetzt. Fünfzig Insekten werden pro Gruppe getestet. Nach 5 Tagen werden das Gewicht der Insekten, die Zahl der Überlebenden und die Menge der gefressenen Blätter bestimmt. Die Resistenz der transformierten Pflanzen wird durch eine statistisch signifikante (Student's Test p < 0,05) Erniedrigung der Gewichtszunahme der Larven, Erniedrigung der Überlebensrate der Larven oder in der Erniedrigung der gefressenen Blattmenge bestimmt, indem Pflanzen, die das LqhIT2-Gen exprimieren, mit Kontrollpflanzen, die nur mit dem Vektor ohne Geneinschub transformiert sind, oder mit nicht-transformierten Pflanzen verglichen werden.

Beispiel 25: Knäuelgras, das durch die Expression von LqhIT2 resistent gegen Schmetterlingslarven ist

A. Konstruktion des Vektors. Das LqhIT2-Gen wird wie in Beispiel 21, Teil A beschrieben synthetisiert und mit dem Vektor pCIB710 verknüpft.

B. Transformation und Regenerierung von Pflanzen. Transformierte Pflanzen werden wie oben beschrieben erhalten.

C. Test von Transformanten auf die Expression von LqhIT2. Transformanten werden wie in Beispiel 21, Teil C beschrieben auf die Expression von LqhIT2 getestet.

D. Resistenz von transformierten Pflanzen gegen Schaden durch Schmetterlinge. Aus T1 Samen erhaltene Keimlinge werden in feine Erde in 100 mm Petrischalen (10 pro Schale, 5 Schalen) gepflanzt. Wenn die zweiten Blätter am Keimling erscheinen, wird jede Schale mit 20 *Crambus caliginosellus* im zweiten Häutungsstadium infiziert. Nach 7 Tagen werden die Zahl, das Gewicht der Überlebenden und das Gewicht der gewaschenen Graswurzeln bestimmt. Die Resistenz der transformierten Pflanzen wird durch eine statistisch signifikante (Student's Test $p < 0,05$) Erniedrigung der Gewichtszunahme der Larven, Erniedrigung der Überlebensrate der Larven oder in der Erniedrigung im Verlust des Wurzelgewichts, verglichen mit Insekten-freien bestimmt, indem Pflanzen, die das LqhIT2-Gen exprimieren, mit Kontrollpflanzen, die nur mit dem Vektor ohne Geneinschub transformiert sind, oder mit nicht-transformierten Pflanzen verglichen werden.

Allen, G., Sequencing of Proteins and Peptides, North Holland, Amsterdam, 43, 1981

Benoist, C., Chambon, P., Nature **290**, 304, 1981

Berry-Lowe, S.L., McKnight, T.D., Shah, D.M., Meagher, R.B., J. Mol. Appl. Genet. **1**, 483, 1982

Bevan, M., Barnes, W.M., Chilton, M.-D., Nucl. Acids Res. **11**, 369, 1983

Bevan, M., Nucl. Acids Res. **12**, 8711, 1984

Binding, H., in: Fowke, L.C. et al. (Hrsg.), Plant Protoplasts, CRC Press, Boca Raton, 21, 1985

Bollon, A.P., Stauver, M., J. Clin. Hematol. Oncol. **10**, 39, 1980

Botstein, D., Shortle, D., Rose, M., in: Ahmad, F., Schultz, J., Smith, E.E., Whelan, W.J. (Hrsg.), Miami Winter Symposia **19**, From Gene to Protein: Translation into Biotechnology, Academic Press, New York, 265, 1982

Broach, J.R., in: Strathern, J.N., Jones, E.W., Broach, J.R. (Hrsg.), The Molecular Biology of the Yeast Saccharomyces, Cold Spring Harbor Laboratory, Cold Spring Harbor, 445, 1981

Broach, J.R., Cell **28**, 203, 1982

Campbell, A.M., in: Burdon, R.H., van Knippenberg, P.H. (Hrsg.) Laboratory Techniques in Biochemistry and Molecular Biology **13**, Elsevier, Amsterdam, 1984

Carlsson, J., Drevin, H., Axén, R., Biochem. J. **173**, 723, 1978

Cashmore, A.R., in: Basic Life Sciences **26**, Genetic Engineering of Plants: An Agricultural Perspective, Plenum, New York, 29, 1983

Catterall, W.A., Ann. Rev. Pharmacol. Toxicol. **20**, 15, 1980

Catterall, W.A., Science **223**, 653, 1984

Cenatiempo, Y., Biochimie **68**, 505, 1986

Chater, K.F., Lomovskaya, N.D., Voeykova, T.A., Sladkova, I.A., Mkrtumian, N.M., Muravnik, G.L., in: Szabó, G., Biró, S., Goodfellow, M. (Hrsg.), Sixth Int. Symp. on Actinomyces Biology, Akademiai Kaido, Budapest, 45, 1986

Chu, C., Wang, C., Sun, C., Hsü, C., Yin, K. Chu, C., Bi, F., Scientia Sinica **18**, 659, 1975

Coruzzi, G., Broglie, R., Cashmore, A., Chua, N.-H., J. Biol. Chem. **258**, 1399, 1983

Couraud, F., Jover, E., Dubois, J.M., Rochat, H., Toxicon **20**, 9, 1982

Couraud, F., Jover, E., in: Tu, A.T. (Hrsg.), Handbook of natural toxins **2**, 659, Dekker, New York, 1984

Dale, P.J., in: Potrykus, I., Harms, C.T., Hinnen, A., Hütter, R., King, P.J., Shillito, R.D. (Hrsg.), Protoplasts 1983, Lecture Proceedings 6th Intl. Protoplast Symposium Basel, 1983, Birkhäuser, Basel, 31, 1983

Darbon, H., Zlotkin, E., Kopeyan, C., van Rietschoten, J., Rochat, H., Int. J. Peptide Protein Res. **20**, 320, 1982

Davey, M.R., in: Potrykus, I., Harms, C.T., Hinnen, A., Hütter, R., King, P.J., Shillito, R.D. (Hrsg.), Protoplasts 1983, Lecture Proc. 6th Intl. Protoplast Symp. Basel, Birkhäuser, Basel, 19, 1983

Devereux, J., Haeberli, P., Smithies, O., Nucleic Acids Res. **12**, 387, 1984

Dunsmuir, P., Smith, S.M., Bedbrook, J., J. Mol. Appl. Genet, **2**, 285, 1983

Duval, A., Léoty, C., J. Physiol. **278**, 403, 1978

Duval, A., Léoty, C., J. Physiol. **307**, 43, 1980

Eisen, H.N., in: Davis, B.D., Microbiology, Harper & Row, Philadelphia, 287, 1980

Evans, D.A., Bravo, J.E., in: Handbook of Plant Cell Culture **1**, Techniques for propagation and breeding, MacMillan Publ. Co., New York, 124, 1983

Fischhoff, D.A., Bowdish, K.S., Perlak, F.J., Marrone, P.G., McCormick, S.M., Niedermeyer, J.G., Dean, D.A., Kusano-Kretzmer, K., Mayer, E.J., Rochester, D.E., Rogers, S.G., Fraley, R.T., Biotechnology **5**, 807, 1987

Fraley, R.T., Rogers, S.G., Horsch, R.B., Sanders, P.R., Flick, J.S., Adams, S.P., Bittner, M.L., Brand, L.A., Fink, C.L., Fry, J.S., Galluppi, G.R., Goldberg, S.B., Hoffmann, N.L., Woo, S.C., Proc. Natl. Acad. Sci. USA **80**, 4803, 1983

Fritz, L.C., Tzeng, M.-C., Mauro, A., Nature **283**, 486, 1980

Fromm, M., Taylor, L.P., Walbot, V., Proc. Natl. Acad. Sci. USA **82**, 5824, 1985

Fromm, M.E., Taylor, L.P., Walbot, V., Nature **319**, 791, 1986

Gamborg, O.L., Miller, R.A., Ojima, K., Exptl. Cell Res. **50**, 151, 1968

Gilman, M.Z., Glenn, J.S., Singer, V.L., Chamberlin, M.J., Gene **32**, 11, 1984

Glick, B.R., Whitney, G.K., J. Ind. Microbiol. **1**, 277, 1987

Gold, L., Pribnow, D., Schneider, T., Shinedling, S., Swebilius Singer, B., Stormo, G., Ann. Rev. Microbiol. **35**, 365, 1981

Gordon, D., Jover, E., Couraud, F., Zlotkin, E., Biochim. Biophys. Acta **778**, 349, 1984

Gorus, F., Schram, E., Clin. Chem. **25**, 512, 1979

Gottesman, S., in: Roman, H.L., Campbell, A., Sandler, L.M. (Hrsg.), Annual Review of Genetics **18**, Annual Reviews, Palo Alto, 415, 1984

Graves, A.C.F., Goldman, S.L., Plant Mol. Biol. **7**, 43, 1986

Grimsley, N., Hohn, T., Davies, J.W., Hohn, B., Nature **325**, 177, 1987

Grimsley, N.H., Ramos, C., Hein, T., Hohn, B., Biotechnology **6**, 185, 1988

Gryczan, T.J., in: Dubnau, D.A. (Hrsg.), The Molecular Biology of the Bacilli **I**, Academic Press, New York, 307, 1982

Hamer, D.H., Walling, M., J. Mol. Appl. Genet, **1**, 273, 1982

Hames, B.D., Higgins, S.J. (Hrsg.), Nucleic Acid Hybridisation, IRL Press, Oxford, 1985

Hämmerling, G.J., Hämmerling, U., Kearney, J.F. (Hrsg.), Research Monographs in Immunology **3**, Monoclonal Antibodies and T-Cell Hybridomas, Elsevier, New York, 1981

Holsters, M., de Waele, D., Depicker, A., Messens, E., van Montagu, M., Schell, J., Mol. Gen. Genet. **163**, 181, 1978

Hood, E.E., Helmer, G.L., Fraley, R.T., Chilton, M.-D., J. Bacteriol. **168**, 1291, 1986

Hooykaas-Van Slogteren, G.M.S., Hooykaas, P.J.J., Schilperoort, R.A., Nature **311**, 763, 1984

Hopp, T.P., Woods, K.R., Molec. Immunol. **20**, 483, 1983

Horsch, R.B., Fraley, R.T., Rogers, S.G., Sanders, P.R., Lloyd, A., Hoffmann, N., Science **223**, 496, 1984

Horsch, R.B., Fry, J.E., Hoffmann, N.L., Eichholtz, D., Rogers, S.G., Fraley, R.T., Science **227**, 1229, 1985

Hultmark, D., Engström, Å., Bennich, H., Kapur, R., Boman, H.G., Eur. J. Biochem **127**, 207, 1982

Hultmark, D., Engström, Å., Andersson, K., Steiner, H., Bennick, H., Boman, H.G., EMBO J. **2**, 571, 1983

Inglis, A., in: Birf, C. (Hrsg.), Solid Phase Methods in Protein Sequence Analysis, Proc. 3rd Intl. Conf., Heidelberg 1979, Elsevier, Amsterdam, 1980

Isaki, K., Jpn. J. Bacteriol. **33**, 729, 1978

Jangi, B.S., in: Tu, A.T. (Hrsg.), Handbook of natural toxins **2**, 333, Dekker, New York, 1984

Jasny, B.R., Science **238**, 1653, 1987

Joh, J.F.Jr., Twitty, J.A., Rev. Infect. Dis. **8**, 693, 1986

Johnston, S.A., Hopper, J.E., Proc. Natl. Acad. Sci. USA **79**, 6971, 1982

Kaiser, E.T., Kézdy, F.J., Ann. Rev. Biophys. Biophys. Chem. **16**, 561, 1987

Kao, K.N., Michayluk, M.R., Planta **126**, 105, 1975

Katsu, T., Ninomiya, C., Kuroko, M., Kobayashi, H., Hirota, T., Fujita, Y., Biochim. Biophys. Acta **939**, 57, 1988

Kendall, K.J., Cohen, S.N., J. Bacteriol. **169**, 4177, 1987

Kennett, R.H., McKearn, T.J., Bechtol, K.B. (Hrsg.), Monoclonal Antibodies, Hybridomas: A New Dimension in Biological Analyses, Plenum Press, New York, 1980

Klein, J., Immunology, The Science of Self-Nonself Discrimination, J. Wiley, New York, 1982

Klein, T.M., Wolf, E.D., Wu, R., Sanford, J.C., Nature **327**, 70, 1987

Klein, T.M., Gradziel, T., Fromm, M.E., Sanford, J.C., Biotechnology **6**, 559, 1988

Knott, T.J., Rall, S.C.Jr., Innerarity, T.L., Jacobson, S.F., Urdea, M.S., Levy-Wilson, B., Powell, L.M., Pease, R.J., Eddy, R., Nakai, H., Byers, M., Priestley, L.M., Robertson, E., Rall, L.B., Betsholtz, C., Shows, T.B., Mahley, R.W., Scott, J., Science **230**, 37, 1985

Knowles, B.H., Ellar, D.J., Biochim. Biophys. Acta **924**, 509, 1987

Köhler, G., Milstein, C., Nature **256**, 495, 1975

Köhler, G., Milstein, C., Eur. J. Immunol. **6**, 511, 1976

Köhler, G., Howe, S.C., Milstein, C., Eur. J. Immunol. **6**, 292, 1976

Kopeyan, C., Martinez, G., Rochat, H., FEBS Letters **89**, 54, 1978

Kopeyan, C., Martinez, G., Rochat, H., Toxicon **20**, 71, 1982

Lathe, R., J. Mol. Biol. **183**, 1, 1985

Lazarovici, P., Zlotkin, E., Comp. Biochem. Physiol. **71C**, 177, 1982

Lazarovici, P., Yanai, P., Pelhate, M., Zlotkin, E., J. Biol. Chem. **257**, 8397, 1982

Lazarovici, P., Menashe, M., Zlotkin, E., Arch. Biochem. Biophys. **229**, 270, 1984

Lehninger, A.L., Biochemistry, Worth Publ., New York, 1975

Lester, D., Lazarovici, P., Pelhate, M., Zlotkin, E., Biochim. Biophys. Acta **701**, 370, 1982

Lis, H., Sharon, N., in: Richardson, C.C. Boyer, P.D., Dawid, I.B., Meister, A. (Hrsg.), Annual Review of Biochemistry **55**, 35, 1986

Liu, C.S., Wu, T.-C., Lo, T.-B., J. Peptide Protein Res. **21**, 209, 1983

Lörz, H., Baker, B., Schell, J., Mol. Gen. Genet. **199**, 178, 1985

Lowry, O.H. Rosebrough, N.J., Farr, A.L., Randall, R.J., J. Biol. Chem. **193**, 265, 1951

Maniatis, T., in: Goldstein, Lester, Prescott (Hrsg.), Cell Biology: a Comprehensive Treatise 3, Gene Expression, Academic Press, New York, 563, 1980

Maniatis, T., Fritsch, E.F., Sambrook, J., Molecular cloning, a laboratory manual, Cold Spring Harbor Laboratory, Cold Spring Harbor, 1982

Marton, L., in: Cell Culture and Somatic Cell Genetics of Plants 1, 514, 1984

May, T.E., Piek, T., J. Insect Physiol. **25**, 685, 1979

McKnight, S.L., Cell **31**, 355, 1982

Miller, L.K., Lingg, A.J., Bulla, L.A., Science **219**, 715, 1983

Miller, D.W., Safer, P., Miller, L.K., in: Setlow, J.K., Hollaender, A., Genetic Engineering 8, Plenum, New York, 277, 1986

Murashsige, T., Skoog, F., Physiol. Plant. **15**, 473, 1962

Nakagawa, S.H., Lau, H.S.H., Kézdy, F.J., Kaiser, E.T., J. Am. Chem. Soc. **107**, 7087, 1985

Negrutiu, I., Shillito, R., Potrykus, I., Biasini, G., Sala, F., Plant Mol. Biol. **8**, 363, 1987

Oike, Y., Kimata, K., Sinomura, T., Suzuki, S., Takahashi, N., Tanabe, K., J. Biol. Chem. **257**, 9751, 1982

Oiki, S., Danho, W., Montal, M., Proc. Natl. Acad. Sci. USA **85**, 2393, 1988

Oka, A., Sugisaki, H., Takanami, M., J. Mol. Biol. **147**, 217, 1981

Okayama, H., Berg, P., Mol. Cell. Biol. **3**, 280, 1983

Ornberg, R.L., Smyth, T., Benton, A.W., Toxicon **14**, 329, 1976

Paszkowski, J., Shillito, R.D., Saul, M., Mandak, V., Hohn, T., Hohn, B., Potrykus, I., EMBO J. **3**, 2717, 1984

Pelhate, M., Pichon, Y., J. Physiol. **242**, 90P, 1974

Pelhate, M., Zlotkin, E., J. Physiol, (London) **319**, 30P, 1981

de la Peña, A., Lörz, H., Schell, J., Nature **325**, 274, 1987

Pichon, Y., Boistel, J., J. Exp. Biol. **47**, 343, 1967

Piek, T., Veenendaal, R.L., Mantel, P., Comp. Biochem. Physiol. **72C**, 303, 1982

Possani, L.D., in: Tu, A.T. (Hrsg.), Handbook of natural toxins 2, 513, Dekker, New York, 1984

Potrykus, I., Saul, M.W., Petruska, J., Paszkowski, J., Shillito, R.D., Mol. Gen. Genet. **199**, 183, 1985

Rathmeyer, W., Zeitsch. vergl. Physiol. **45**, 413, 1962

Reed, L.J., Muench, H., Am. J. Hyg. **27**, 493, 1938

Reich, T.J., Iyer, V.N., Haffner, M., Holbrook, L.A., Miki, B.L., Can. J. Bot. **64**, 1259, 1986 a

Reich, T.J., Iyer, V.N., Miki, B.L., Biotechnology 4, 1001, 1986 b

Rochat H., Rochat, C., Sampieri, F., Miranda, F., Eur. J. Biochem. **28**, 381, 1972

Rochat, H., Bernard, P., Couraud, F., Adv. Cytopharmacol. **3**, 325, 1979

Rothstein, S.J., Lahners, K.N., Lotstein, R.J., Carozzi, N.B., Jayne, S.M., Rice, D.A., Gene **53**, 153, 1987

Rubin, G.M., Science **240**, 1453, 1988

Sacchi, V.F., Parenti, P., Hanozet, G.M., Giordana, B., Lüthy, P., Wolfersberger, M.G., FEBS Lett. **204**, 213, 1986

Sattelle, D.B., Pelhate, M., Hue, B., J. Exp. Biol. **83**, 41, 1979

Shillito, R.D., Saul, M.W., Paszkowski, J., Müller, M., Potrykus, I., Biotechnology 3, 1099, 1985

Silver, P.A., Keegan, L.P., Ptashne, M., Proc. Natl. Acad. Sci. USA 81, 5951, 1984

Simon, R., Priefer, U., Pühler, A., Biotechnology **1**, 784, 1983

Soini, E., Hemmilä, I., Clin. Chem. **25**, 353, 1979

Stockhaus, J., Eckes, P., Blau, A., Schell, J., Willmitzer, L., Nucl. Acids Res. **15**, 3479, 1987

Swank, R.T., Munkres, K.D., Anal. Biochem. **39**, 462, 1971

Tobkes, N., Wallace, B.A., Bayley, H., Biochemistry **24**, 1915, 1985

Ulmanen, I., Lundström, K., Lehtovaara, P., Sarvas, M., Ruohonen, M., Palva, I., J. Bacteriol. **162**, 176, 1985

Wahl, R.L., Parker, C.W., Philpott, G.W., J. Nucl. Med. **24**, 316, 1983

Walther, C., Zlotkin, E., Rathmeyer, W., J. Insect Physiol. **22**, 1187, 1976

Wands, J.R., Zurawski, V.R.Jr., Gastroenterology **80**, 225, 1981

Ward, J.M., Janssen, G.R., Kieser, T., Bibb. M.J., Buttner, M.J., Bibb, M.J., Mol. Gen. Genet. **203**, 468, 1986

Watson, B., Currier, T.C., Gordon, M.P. Chilton, M.-D., Nester, E.W., J. Bacteriol. **123**, 255, 1975

Watson, J.D., Hopkins, N.H., Roberts, J.W., Argetsinger Steitz, J., Weiner, A.M., Molecular biology of the gene, Benjamin/Cummings, Menlo Park, 1977

Wisdom, G.B., Clin. Chem. **22**, 1243, 1976

Yadav, N.S., Vanderleyden, J., Bennett, D.R., Barnes, W.M., Chilton, M.D., Proc. Natl. Acad. Sci. USA **79**, 6322, 1982

Zasloff, M., Proc. Natl. Acad. Sci. USA **84**, 5449, 1987

Zlotkin, E., Rochat, H., Kopeyan; C., Miranda, F., Lissitzky, S., Biochimie **53**, 1973, 1971 a

Zlotkin, E., Fraenkel, G., Miranda, F., Lissitzky, S., Toxicon **9**, 1, 1971 b

Zlotkin, E., Miranda, F., Kupeyan, C., Lissitzky, S., Toxicon **9**, 9, 1971 c

Zlotkin, E., in: Kerkut, G.A., Gilbert, L.I. (Hrsg.), Comprehensive Insect Physiology, Biochemistry and Pharmacology **10**, Pergamon Press, Oxford, 499, 1985

Zlotkin, E., Kadouri, D., Gordon, D., Pelhate, M., Martin, M.F., Rochat, H., Arch. Biochem. Biophys. **240**, 877, 1985

Zlotkin, E., Gordon, D., in: Breer, H., Miller, T.A. (Hrsg.), Neurochemical Techniques in Insect Research, 243, Springer, Berlin, 1985

Zlotkin, E., in: Neuropharmacology and Pesticide Action, 352, 1986

**Ansprüche**

1. Ein selektiv gegen Insekten wirkendes Toxin, dadurch gekennzeichnet, dass es die nachfolgende Aminosäuresequenz hat: VRDAYIAKNY NCVYECFRDA
YCNELCTKNG ASSGYCQWAG KYGNACWCYA LPDNVPIRVP GKCR.

2. Rekombinante DNA enthaltend eine DNA-Sequenz erhältlich aus Tieren, wobei besagte DNA-Sequenz ein selektiv gegen Insekten wirkendes Toxin oder ein funktionsfähiges Derivat oder Bruchstück davon kodiert.

3. Rekombinante DNA nach Anspruch 2, dadurch gekennzeichnet, dass besagte DNA in einer in Pflanzen exprimierbaren Form vorliegt.

4. Rekombinante DNA nach einem der Ansprüche 2 oder 3, dadurch gekennzeichnet, dass besagte DNA ein aus Arthropoden erhältliches, selektiv gegen Insekten wirkendes Toxin oder ein funktionsfähiges Derivat oder Bruchstück davon kodiert.

5. Rekombinante DNA nach einem der Ansprüche 2 oder 3, dadurch gekennzeichnet, dass besagte DNA ein aus Vertretern der Klaasen *Arachnida* (Spinnentiere) oder *Chilopoda* (Hundertfüssler) erhältliches, selektiv gegen Insekten wirkendes Toxin oder ein funktionsfähiges Derivat oder Bruchstück davon kodiert.

6. Rekombinante DNA nach einem der Ansprüche 2 oder 3, dadurch gekennzeichnet, dass besagte DNA ein aus Vertretern der Ordnung *Scorpiones* erhältliches, selektiv gegen Insekten wirkendes Toxin oder ein funktionsfähiges Derivat oder Bruchstück davon kodiert.

7. Rekombinante DNA nach einem der Ansprüche 2 oder 3, dadurch gekennzeichnet, dass besagte DNA ein aus Vertretern der Gattung *Scolopendra* erhältliches, selektiv gegen Insekten wirkendes Toxin oder ein funktionsfähiges Derivat oder Bruckstück davon kodiert. ·

8. Rekombinante DNA nach einem der Ansprüche 2 oder 3, dadurch gekennzeichnet, dass besagte· DNA ein Toxin mit der Aminosäuresequenz
KKNGYAVDSS GKAPECLLSN YCNNQCTKVH YADKGYCCLL SCYCFGLNDD KKVLEISDTR KSYCDTTIIN,
DGYIRKRDGC KLSCLFGNEG CNKECKSYGG SYGYCWTWGL ACWCEGLPDE KTWKSETNTC G,
DGYIRKKDGC KVSC(V/I)IIGNEG CRKECVAHGG SFGYCWTWGL ACWCENLPDA VTWKSSTNTC G,
DGYIKRRDGC KVACLIGNEG CDKECKAYGG SYGYCWTWGL ACWCEGLPDD KTWKSETNTC G,
ALPLSGEYEP CVRPRKCKPG LVCNKQQICV DPK oder
VRDAYIAKNY NCVYECFRDA YCNELCTKNG ASSGYCQWAG KYGNACWCYA LPDNVPIRVP GKCR oder
ein funktionsfähiges Derivat oder Bruchstück davon kodiert.

9. Vektor enthaltend eine DNA gemäss einem der Ansprüche 2 bis 8.

10. Wirtsorganismus enthaltend einen Vektor gemäss Anspruch 9.

11. Transgene Pflanzenzelle enthaltend eine DNA-Sequenz erhältlich aus Tieren, wobei besagte DNA-Sequenz ein selektiv gegen Insekten wirkendes Toxin oder ein funktionsfähiges Derivat oder Bruckstück davon kodiert.

12. Transgene Pflanzenzelle gemäss Anspruch 11, dadurch gekennzeichnet, dass besagte DNA-Sequenz erhältlich ist aus Arthropoden.

13. Transgene Pflanzenzelle gemäss Anspruch 11, dadurch gekennzeichnet, dass besagte DNA-Sequenz erhältlich ist aus Vertretern der Klassen *Arachnida* (Spinnentiere) oder *Chilopoda* -

(Hundertfüssler).

14. Transgene Pflanzenzelle gemäss Anspruch 11, dadurch gekennzeichnet, dass besagte DNA-Sequenz erhältlich ist aus Vertretern der Ordnung *Scorpiones.*

15. Transgene Pflanzenzelle gemäss Anspruch 11, dadurch gekennzeichnet, dass besagte DNA-Sequenz erhältlich ist aus Vertretern der Gattung *Scolopendra.*

16. Transgene Pflanzenzelle gemäss einem der Ansprüche 11 bis 15, dadurch gekennzeichnet, dass besagte DNA-Sequenz stabil in das pflanzliche Genom integriert ist.

17. Transgene Pflanzenzelle gemäss Anspruch 16, dadurch gekennzeichnet, dass besagte DNA-Sequenz in exprimierbarer Form vorliegt.

18. Transgene Pflanzenzelle gemäss Anspruch 17, dadurch gekennzeichnet, dass sie das von besagter DNA-Sequenz kodierte Toxin oder ein funktionsfähiges Derivat oder Bruckstück davon exprimiert.

19. Transgene Pflanze sowie deren sexuelles und asexuelles Vermehrungsgut enthaltend eine DNA-Sequenz erhältlich aus Tieren, wobei besagte DNA-Sequenz ein selektiv gegen Insekten wirkendes Toxin oder ein funktionsfähiges Derivat oder Bruckstück davon kodiert.

20. Transgene Pflanze sowie deren sexuelles und asexuelles Vermehrungsgut gemäss Anspruch 19, dadurch gekennzeichnet, dass besagte DNA-Sequenz erhältlich ist aus Arthropoden.

21. Transgene Pflanze sowie deren sexuelles und asexuelles Vermehrungsgut gemäss Anspruch 19, dadurch gekennzeichnet, dass besagte DNA-Sequenz erhältlich ist aus Vertretern der Klaasen *Arachnida* - (Spinnentiere) oder *Chilopoda* (Hundertfüssler).

22. Transgene Pflanze sowie deren sexuelles und asexuelles Vermehrungsgut gemäss Anspruch 19, dadurch gekennzeichnet, dass besagte DNA-Sequenz erhältlich ist aus Vertretern der Ordnung *Scorpiones.*

23. Transgene Pflanze sowie deren sexuelles und asexuelles Vermehrungsgut gemäss Anspruch 19, dadurch gekennzeichnet, dass besagte DNA-Sequenz erhältlich ist aus Vertretern der Gattung *Scolopendra.*

24. Transgene Pflanze sowie deren sexuelles und asexuelles Vermehrungsgut gemäss einem der Ansprüche 19 bis 23, dadurch gekennzeichnet, dass besagte DNA-Sequenz stabil in das pflanzliche Genom integriert ist.

25. Transgene Pflanze sowie deren sexuelles und asexuelles Vermehrungsgut gemäss Anspruch 24, dadurch gekennzeichnet, dass besagte DNA-Sequenz in exprimierbarer Form vorliegt.

26. Transgene Pflanze sowie deren sexuelles und asexuelles Vermehrungsgut gemäss Anspruch 25, dadurch gekennzeichnet, dass sie das von besagter DNA-Sequenz kodierte Toxin oder ein funktionsfähiges Derivat oder Bruchstück davon exprimiert.

27. Transgener Mikroorganismus enthaltend eine DNA-Sequenz erhältlich aus Tieren, wobei besagte DNA-Sequenz ein selektiv gegen Insekten wirkendes Toxin oder ein funktionsfähiges Derivat oder Bruchstück davon kodiert.

28. Transgener Mikroorganismus gemäss Anspruch 27, dadurch gekennzeichnet, dass besagte DNA-Sequenz erhältlich ist aus Arthropoden.

29. Transgener Mikroorganismus gemäss Anspruch 27, dadurch gekennzeichnet, dass besagte DNA-Sequenz erhältlich ist aus Vertretern der Klassen *Arachnida* (Spinnentiere) oder *Chilopoda* - (Hundertfüssler).

30. Transgener Mikroorganismus gemäss Anspruch 27, dadurch gekennzeichnet, dass besagte DNA-Sequenz erhältlich ist aus Vertretern der Ordnung *Scorpiones.*

31. Transgener Mikroorganismus gemäss Anspruch 27, dadurch gekennzeichnet, dass besagte DNA-Sequenz erhältlich ist aus Vertretern der Gattung *Scolopendra.*

32. Transgener Mikroorganismus gemäss einem der Ansprüche 27 bis 31, dadurch gekennzeichnet, dass besagte DNA-Sequenz stabil in das Genom integriert ist.

33. Transgener Mikroorganismus gemäss Anspruch 32, dadurch gekennzeichnet, dass besagte DNA-Sequenz in exprimierbarer Form vorliegt.

34. Transgener Mikroorganismus gemäss Anspruch 33, dadurch gekennzeichnet, dass er das von besagter DNA-Sequenz kodierte Toxin oder ein funktionsfähiges Derivat oder Bruckstück davon exprimiert.

35. Antikörper gegen selektiv auf Insekten wirkende Toxine oder funktionsfähige Derivate oder Bruchstücke davon erhältlich aus Vertretern der Ordnung *Scorpiones* oder der Gattung *Scolopendra.*

36. Insektizides Mittel enthaltend als Wirksubstanz ein aus Tieren erhältliches, selektiv gegen Insekten wirkendes Toxin oder ein funktionsfähiges Derivat oder Bruchstück davon.

37. Insektizides Mittel nach Anspruch 36, dadurch gekennzeichnet, dass es sich bei dem Toxin um ein rekombinantes Toxin handelt.

38. Verfahren zur Bekämpfung von phytopathogenen Insekten, dadurch gekennzeichnet, dass man auf das Insekt oder dessen Lebensraum einen Mikroorganismus gemäss einem der Ansprüche 27 bis 34 oder ein Mittel gemäss einem der Ansprüche 36 oder 37 in einer insektizid wirksamen Menge appliziert.

39. Verfahren zum Schutz von Kulturpflanzen vor Schäden durch phytopathogene Insekten, dadurch gekennzeichnet, dass man die Kulturpflanze mit einer rekombinanten DNA nach Anspruch 2 transformiert, und dass weiterhin das besagte gegen Insekten wirkende Toxin oder ein funktionsfähiges Derivat oder Bruchstück davon in einer insektizid wirksamen Menge in der Pflanze exprimiert wird.

*Fig. 1*

# Fig. 2

Fig. 3

Fig. 4

# Fig. 5

## Fig. 6

# Sequenzen von Skorpionentoxinen

## Toxine der *Buthinae* Skorpione

| | | | | | | |
|---|---|---|---|---|---|---|
| LqhIT2 | DGYIKRRDGC | KVACLIGNEG | CDKECKAYGG | SYGYCWTWGL | ACWCEGLPDD | KTWKSETNTC G |
| LqqIT2 | DGYIRKRDGC | KLSCLFGNEG | CNKECKSYGG | SYGYCWTWGL | ACWCEGLPDE | KTWKSETNTC G |
| BjIT2 | DGYIRKKDGC | KVSCIIGNEG | CRKECVAHGG | SFGYCWTWGL | ACWCENLPDA | VTWKSSTNTC G |
| LqhP35 | VRDAYIAKNY | NCVYECFRDA | YCNELCTKNG | ASSGYCQWAG | KYGNACWCYA | LPDNVPIRVP GKCR |

## Toxine der *Chactidae* Skorpione

| | | | | |
|---|---|---|---|---|
| SmpIT2 | ALPLSGEYEP | CVRPRKCKPG | LVCNKQQICV | DPK |
| SmpCT2 | VSCTGSRDCY | APCKRQTGCT | SAKCINKSCK | CYGC |
| SmpCT3 | VSCTGSKDCY | APCRKQTGCP | NAKCINKSCK | CYGC |
| SmpMT | VSCTGSKECY | APCKKQTGCP | NAKCMNRKCK | CYGC |

*Fig. 1*

EP 0 374 753 A2

EP 0 374 753 A2

# SYNTHESE DES AAIT-GENS

## SEQUENZ 1a
### kodierender Strang

```
5'    1   GATCCAAATA  ATGAAAAAAA  ACGGCTACGC  TGTTGACTCT  TCTGGCAAAG
          I_____1_____I I_____2_____I I_____

     51   CTCCGGAATG  CCTGCTGTCT  AACTACTGCA  ACAACCAGTG  CACTAAAGTT
          _____3_____I I_____4_____I I_____5_____

    101   CATTACGCTG  ACAAAGGCTA  CTGCTGCCTG  CTGTCTTGCT  ACTGCTTCGG
          ___I I_____6_____I I_____7_____I I_____

    151   CCTGAACGAC  GACAAAAAAG  TTCTGGAAAT  CTCTGACACT  CGTAAATCTT
          ___8_____I I_____9_____I I_____10_____

    201   ACTGCGACAC  TACTATCAAC  TAATAG      3'
          ____I I_____11_____I
```

## SEQUENZ 1b
### komplementärer Strang

```
5'    1   GATCCTATTAGTTG  ATAGTAGTGT  CGCAGTAAGA  TTTACGAGTG   TCAGAGATTT
          I_____22_____I I_____21_____I I_____20__

     55   CCAGAACTTT  TTTGTCGTCG  TTCAGGCCGA  AGCAGTAGCA  AGACAGCAGG
          _____I I_____19_____I I_____18_____I I___

    105   CAGCAGTAGC  CTTTGTCAGC  GTAATGAACT  TTAGTGCACT  GGTTGTTGCA
          _____17_____I I_____16_____I I_____15_____

    155   GTAGTTAGAC  AGCAGGCATT  CCGGAGCTTT  GCCAGAAGAG  TCAACAGCGT
          _____I I_____14_____I I_____13_____I I___

    201   AGCCGTTTTT  TTTCATTATT  TG          3'
          _____12_____I
```

*Fig. 8A*

## SEQUENZ 1c
## Synthetisierte Fragmente

| | |
|---|---|
| 1 | GATCCAAATAATGAAAAAAACGG |
| 2 | CTACGCTGTTGACTCTTCTG |
| 3 | GCAAAGCTCCGGAATGCCTG |
| 4 | CTGTCTAACTACTGCAACAA |
| 5 | CCAGTGCACTAAAGTTCATT |
| 6 | ACGCTGACAAAGGCTACTGC |
| 7 | TGCCTGCTGTCTTGCTACTG |
| 8 | CTTCGGCCTGAACGACGACA |
| 9 | AAAAAGTTCTGGAAATCTCT |
| 10 | GACACTCGTAAATCTTACTG |
| 11 | CGACACTACTATCAACTAATAG |
| 12 | CGTAGCCGTTTTTTTTCATTATTTG |
| 13 | TTTGCCAGAAGAGTCAACAG |
| 14 | GACAGCAGGCATTCCGGAGC |
| 15 | ACTGGTTGTTGCAGTAGTTA |
| 16 | AGCGTAATGAACTTTAGTGC |
| 17 | AGGCAGCAGTAGCCTTTGTC |
| 18 | CGAAGCAGTAGCAAGACAGC |
| 19 | TTTTTTGTCGTCGTTCAGGC |
| 20 | GTGTCAGAGATTTCCAGAAC |
| 21 | TGTCGCAGTAAGATTTACGA |
| 22 | GATCCTATTAGTTGATAGTAG |

Fig. 8B

## SEQUENZ 1d
### endgültiges Gen

```
              10                    30                    50
   GATCCAAATAATGAAAAAAAACGGCTACGCTGTTGACTCTTCTGGCAAAGCTCCGGAATG
1  ----+----+----+----+----+----+----+----+----+----+----+----+ 60
   GTTTATTACTTTTTTTTGCCGATGCGACAACTGAGAAGACCGTTTCGAGGCCTTAC
    M   K   K   N   G   Y   A   V   D   S   S   G   K   A   P   E   C

              70                    90                   110
   CCTGCTGTCTAACTACTGCAACAACCAGTGCACTAAAGTTCATTACGCTGACAAAGGCTA
61 ----+----+----+----+----+----+----+----+----+----+----+----+ 120
   GGACGACAGATTGATGACGTTGTTGGTCACGTGATTTCAAGTAATGCGACTGTTTCCGAT
    L   L   S   N   Y   C   N   N   Q   C   T   K   V   H   Y   A   D   K   G   Y

              130                   150                   170
   CTGCTGCCTGCTGTCTTGCTACTGCTTCGGCCTGAACGACGACAAAAAAGTTCTGGAAAT
121 ----+----+----+----+----+----+----+----+----+----+----+----+ 180
   GACGACGGACGACAGAACGATGACGAAGCCGGACTTGCTGCTGTTTTTTCAAGACCTTTA
    C   C   L   L   S   C   Y   C   F   G   L   N   D   D   K   K   V   L   E   I

              190                   210
   CTCTGACACTCGTAAATCTTACTGCGACACTACTATCAACTAATAG
181 ----+----+----+----+----+----+----+------ 230
   GAGACTGTGAGCATTTAGAATGACGCTGTGATGATAGTTGATTATCCTAG
    S   D   T   R   K   S   Y   C   D   T   T   I   N   *   *
```

*Fig. 8C*

EP 0 374 753 A2

## SEQUENZ 2
### Gen, welches das auf Insekten
### wirkende LqhIT2 Toxin kodiert

```
                  10                      30                      50
      GATCCATGGACGGCTACATCAAGCGCCGCGACGGCTGCAAGGTGGCTTGCCTGATCGGCA
  1   ---- -----+---------+---------+---------+---------+---------+  60
      GTACCTGCCGATGTAGTTCGCGGCGCTGCCGACGTTCCACCGAACGGACTAGCCGT
       M  D  G  Y  I  K  R  R  D  G  C  K  V  A  C  L  I  G  N

                  70                      90                      110
      ACGAGGGCTGCGACAAGGAGTGCAAGGCTTACGGCGGCAGCTACGGCTACTGCTGGACCT
  61  --------+---------+---------+---------+---------+--------+  120
      TGCTCCCGACGCTGTTCCTCACGTTCCGAATGCCGCCGTCGATGCCGATGACGACCTGGA
       E  G  C  D  K  E  C  K  A  Y  G  G  S  Y  G  Y  C  W  T  W

                  130                     150                     170
      GGGGCCTGGCTTGCTGGTGCGAGGGCCTGCCGGACGACAAGACCTGGAAGAGCGAGACCA
  121 --------+---------+---------+---------+---------+---------+  180
      CCCCGGACCGAACGACCACGCTCCCGGACGGCCTGCTGTTCTGGACCTTCTCGCTCTGGT
       G  L  A  C  W  C  E  G  L  P  D  D  K  T  W  K  S  E  T  N

                  190
      ACACCTGCGGCTAATAG
  181 --------+-------        201
      TGTGGACGCCGATTATCCTAG
       T  C  G  *  *
```

## *Fig. 9*